# EUROPEAN PATENT APPLICATION

(11) **EP 2 669 378 A1**
(43) Date of publication of application: **04.12.2013**
(21) Application number: 12170232.8
(22) Date of filing: 31.05.2012
(51) Int. Cl.: C12N 15/53, C12N 9/00, A61K 48/00, C12N 5/10

(54) **Cytochrome P450 suicide gene system**

(71) Applicant: Hanenberg, Helmut, Indianapolis, IN 46278 (US)
(72) Inventor: Hanenberg, Helmut, Indianapolis, Indiana 46278 (US); Wiek, Constanze, 40591 Düsseldorf (DE); kramm, Christof, 06114 Halle (DE); Schmidt, Eva, 40223 Düsseldorf (DE)
(74) Representative: Elbel, Michaela

(57) **Abstract**

The invention relates to a modified human CYP4B1 protein and a nucleic acid encoding for the modified human CYP4B1 protein. The invention further relates to an expression vector comprising a nucleic acid encoding for a modified human CYP4B1 protein, as well as to a cell comprising a modified CYP4B1 protein. In addition, the invention relates to a modified human CYP4B1 protein for use in treating transplantation induced graft-versus-host-disease (GvHD), and to 4-ipomeanol (4-IPO) for use in killing a cell.

## Description

### Field of the Invention

The invention relates to a modified human CYP4B1 protein and a nucleic acid encoding for the modified human CYP4B1 protein. The invention further relates to an expression vector comprising a nucleic acid encoding for a modified human CYP4B1 protein, as well as to a cell comprising a modified CYP4B1 protein. In addition, the invention relates to a modified human CYP4B1 protein for use in treating transplantation induced graft-versus-host-disease (GvHD), and to 4-ipomeanol (4-IPO) for use in killing a cell.

### Background of the Invention

Transplantation of allogeneic hematopoietic stem cells from bone marrow, blood, or cord blood can occur in a wide variety of hematological and genetic diseases that affect the body and immune system. However, the only treatment that can potentially cure patients with relapsed acute or chronic leukemia after stem cell transplantation is the infusion of mature T-lymphocytes from the stem cell donor. The effectiveness of these donor lymphocyte infusions is limited by the fact that the fresh immunocompetent T-cells of the donor do not only mediate the curative anti-tumor graft-versus-leukemia (GvL) reaction, but often induce a potentially life-threatening severe graft-versus-host disease (GvHD) that predominantly affects liver, intestine and skin of the recipient (Barrett, A. J. and Malkovska V., 1996; Collins, R. H. et al., 1997). GvL and GvHD are closely linked, as both the probability to cure the leukemia and the risk of severe GvHD strongly increase with the amount of donor cells that were infused. Therefore, if too many donor lymphocytes are infused, the patient could die as consequence of the treatment. If too few donor lymphocytes are applied, the patient could die from the leukemia relapse. Thus, the control and stabilization of the delicate balance between GvL effect and GvHD is an important issue for donor lymphocyte infusions.

An import strategy to effectively influence the GvHD is to equip the donor lymphocytes prior to infusion with a suicide gene that will consecutively allow controlling the donor T-cells *in vivo* in the patient. Suicide genes introduced into the donor T-cells encode for proteins, which convert non-toxic substances called pro-drugs into cytotoxic metabolites. Importantly, endogenous cells of the patient do not carry the suicide gene and thus do not express the suicide protein, such that the active cytotoxic agent will not be produced or taken up by other cells than the donor T-lymphocytes. In case of a life threatening GvHD, the application of the pro-drug will thus specifically eliminate the suicide gene-positive donor lymphocytes that cause the immunologic reaction and thereby stop the GvHD.

The prototype suicide gene for donor lymphocyte infusions is the thymidine kinase gene of herpes simplex virus type (HSV-tk) in combination with its pro-drug, the antiviral substance ganciclovir (Bonini et al., 1997; Bordignon et al., 1995; Tiberghin et al. 2001). However, the usefulness of this system is limited by the strong immunogenicity of the HSV-tk protein that often leads to a rapid elimination of the genetically modified T-cells. Additionally, ganciclovir is paramount for the treatment of viral infections or reactivations after transplantation such that the modified T-cells would be killed during regular antiviral treatments.

Therefore, an alternative suicide gene system is needed to specifically eliminate donor cells after transplantation in case the recipient develops a GvHD.

### Summary of the Invention

In a first aspect, the invention is directed to a modified human CYP4B1 protein, wherein at least one part of the human CYP4B1 protein is replaced by a corresponding part of a CYP4B1 protein of a different species.

In a second aspect, the invention is directed to a modified human CYP4B1 protein with a serine to proline exchange in the meander region, wherein the 5'-region, the middle region, the 3'-region of the human CYP4B1 protein and/or a part of any of the regions is replaced by a corresponding part of a CYP4B1 protein of a different species.

In a further aspect, the invention is directed to a modified human CYP4B1 protein with a serine to proline exchange at position 427, wherein amino acids 124 to 227 consist of SEQ ID NO: 19.

In a further aspect, the invention is directed to a nucleic acid encoding for a modified human CYP4B1 protein, wherein at least one part of the human CYP4B1 protein is replaced by a corresponding part of a CYP4B1 protein of a different species.

In a further aspect, the invention is directed to an expression vector comprising a DNA or RNA encoding a modified human CYP4B1 protein, wherein at least one part of the human CYP4B1 protein is replaced by the corresponding part of a CYP4B1 protein of a different species.

In a further aspect, the invention is directed to a cell comprising a modified human CYP4B1 protein, wherein at least one part of the human CYP4B1 protein is replace by the corresponding part of a CYP4B1 protein of a different species.

In a further aspect, the invention is directed to a modified human CYP4B1 protein for use in treating transplantation induced graft-versus-host-disease (GvHD), wherein at least part of the human CYP4B1 protein is replaced by the corresponding part of a CYP4B1 protein of a different species.

In a further aspect, the invention is directed to 4-ipomeanol (4-IPO) for use in killing a cell, wherein the cell comprises a modified human CYP4B1 protein with at least one part of the human CYP4B1 protein replaced by the corresponding part of a CYP4B1 protein of a different species.

### Brief Description of the Drawings

Figure 1 shows an amino acid alignment of the human and the rabbit CYP4B1 protein. The ERR triade (in bold characters) is the glutamine-arginine-arginine motif, which is conserved in cytochrome P450 sequences. The C-ligand indicates the heme binding cysteine residue. The meander region (underlined) contains the conserved proline-X-arginine motif, which differs between human and rabbit CYP4B1 at amino acid position 427.
Figure 2 shows 4-IPO toxicity experiments in HepG2 cells. The survival curve of the transduced HepG2 cells after one and after three days incubation in the presence of 4-IPO is shown for each construct (mean plus/minus SEM) including codon-optimized constructs (coop). The transduction efficiency in HepG2 cells was above 90 %. In addition, a schematic map of the expression vector used is depicted.
Figure 3A shows the functional analysis of CYP4B1/EGFP (3'-EGFP) and EGFP/CYP4B1 (5'-EGFP) fusion proteins with 4-IPO for comparing the protein stability of human and rabbit CYP4B1 proteins. IRES-EGFP plasmids were used as reference constructs. The percentage survival of EGFP+ HepG2 cells after incubation for 24 and 72 hours with 4-IPO is shown as mean +/- SEM.
Figure 3B shows the schematic map of the lentiviral constructs used.
Figures 3C and D show CYP4B1 stability as analysed by western blotting and by flow cytometry. The studies on protein expression and stability were performed with the rabbit and human CYP4B1/EGFP fusion construction in the lentiviral vector with the additional IRES-neomycin cassette. Half-life analysis was performed by adding 50 µg/ml cycloheximide to the transduced and G418-selected HepG2 cells. Samples were collected at the indicated time points.
Figure 4 shows an amino acid alignment of the human and the rabbit CYP4B1 protein indicating the restriction sites of BstXI and Blpl, as well as sections 1 - 6 for both the human and the rabbit CYP4B1 protein. The meander region is underlined.
Figure 5 shows an amino acid alignment of the human and the rabbit CYP4B1 protein indicating the restriction sites of BstXI and Blpl, as well as all individual amino acid alterations between human and rabbit CYP4B1 protein in sections 1 - 3.
Figure 6A shows the activity of CYP4B1 proteins after different domain exchanges between human and rabbit proteins as measured by survival of transduced cells upon application of 4-IPO.
Figure 6B shows the activity of modified CYP4B1 proteins after exchanging different sections of the middle domain between human and rabbit proteins as revealed by cell survival upon administration of 4-IPO.
Figure 6C shows the activity of modified CYP4B1 proteins after exchanging single amino acid in sections 1 to 3.
Figure 6D shows the activity of modified CYP4B1 proteins after exchanging different combinations of sections 1 to 3 and of the three individual mutations of section 1.
Figure 7A and B show the toxicity of CYP4B1/EGFP fusion proteins in HepG2 (Figure 7 A) and in primary T-cells (Figure 7 B) after 4-IPO treatment.
Figure 7C shows the schematic map of the expression vectors used for transducing HepG2 cells and primary T-cells, respectively.
Figure 8 shows the schematic outline of a foamyviral vector and the corresponding plasmids.
Figure 9 shows the promoter-dependent T-cell transduction efficiency and transgene expression levels with foamyviral vectors.
Figure 10 shows the effect of WPR elements on foamyviral transduction efficiency and transgene expression.
Figure 11 shows an alignment of sections 1 (Figure 11 A), 2 (Figure 11 B) and 3 (Figure 11 C) of the human CYP4B1 protein, the rabbit CYP4B1 protein and several other members of the human CYP4 subfamily. The alignment reveals that twelve of the amino acids differing between sections 1 to 3 of the human CYP4B1 protein and the rabbit CYP4B1 protein are found in other members of the human CYP4 subfamily.

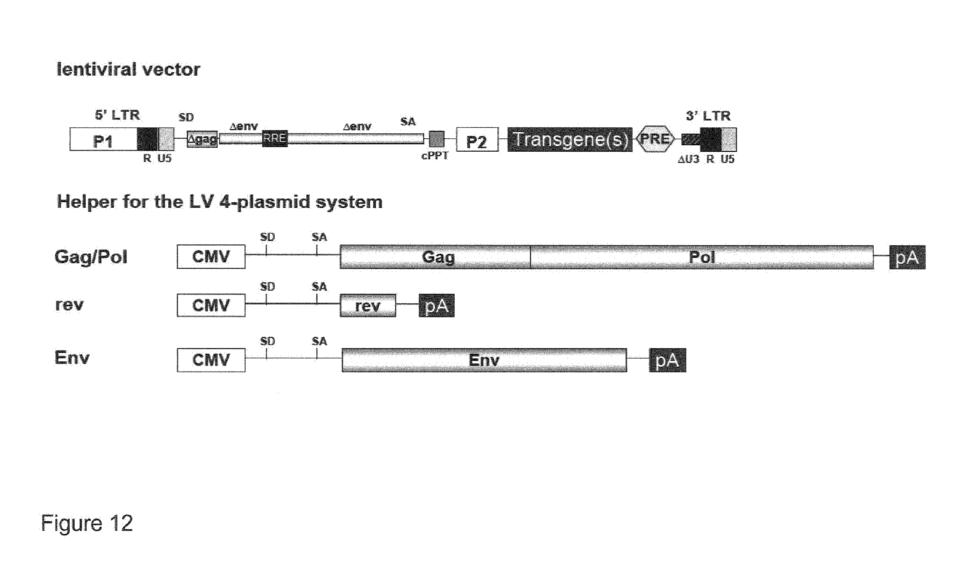

Figure 12 shows the schematic outline of a lentiviral vector and the corresponding plasmids.
Figure 13 shows the promoter-dependent T-cell transduction efficiency and transgene expression levels with lentiviral vectors.
Figure 14 shows the effect of WPR elements on lentiviral transduction efficiency and transgene expression.

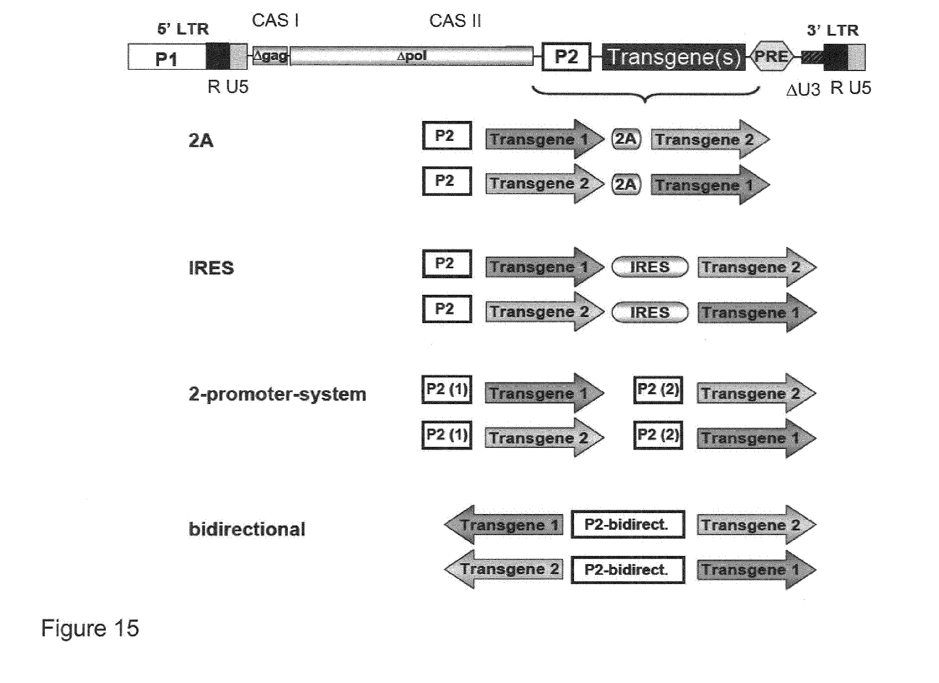

Figure 15 shows the schematic outline of a foamyviral vector and the Linker options in order to express two transgenes

### Detailed Description of the Intention

In a first aspect, the invention is directed to a modified human CYP4B1 protein, wherein at least one part of the human CYP4B1 protein is replaced by a corresponding part of a CYP4B1 protein of a different species.

CYP4B1 is a member of the CYP4 family, which belongs to the superfamily of Cytochrome-P450 enzymes. In mammals six different subfamilies of CYP4 were described, CYP4A, 4B, 4F, 4V, 4X and 4Z. In general, the members of the cytochrome P450 superfamily catalyze the oxidation of organic substances through a monooxygenase reaction using heme as a cofactor. The enzymatic activity of CYP4B1 was found to be responsible for the cytotoxic effect of 4-ipomeanol (4-IPO), which was observed in cattle and other animals, such as dogs, rats, rabbits and mice. In these species, CYP4B1 metabolizes 4-IPO into highly toxic alkylating metabolites, which cause DNA-protein cross-links and DNA strand breaks and thus trigger apoptosis mediated cell death (Boyd and Reznik-Schuller, 1984). Interestingly, however, humans are immune/nonresponsive to 4-IPO and do not show any signs of toxicity upon ingestion of 4-IPO. This is due to the fact that the native human CYP4B1 protein lacks enzymatic activity, presumably because it is unable to bind heme, and thus can not process 4-IPO.

In humans, two different variants of CYP4B1 protein are known, which differ in the fact that one variant comprises a serine insertion at amino acid position 207 due to alternative spicing. The protein sequences of both variants are given in SEQ ID NO: 1 and SEQ ID NO: 20 and the cDNA sequences encoding for the two variants of CYP4B1 are given in SEQ ID NO: 2 and SEQ ID NO: 21, respectively. Despite the insertion of an additional serine, both variants show similar properties, in particular they are both incapable of processing 4-IPO into cytotoxic metabolites.

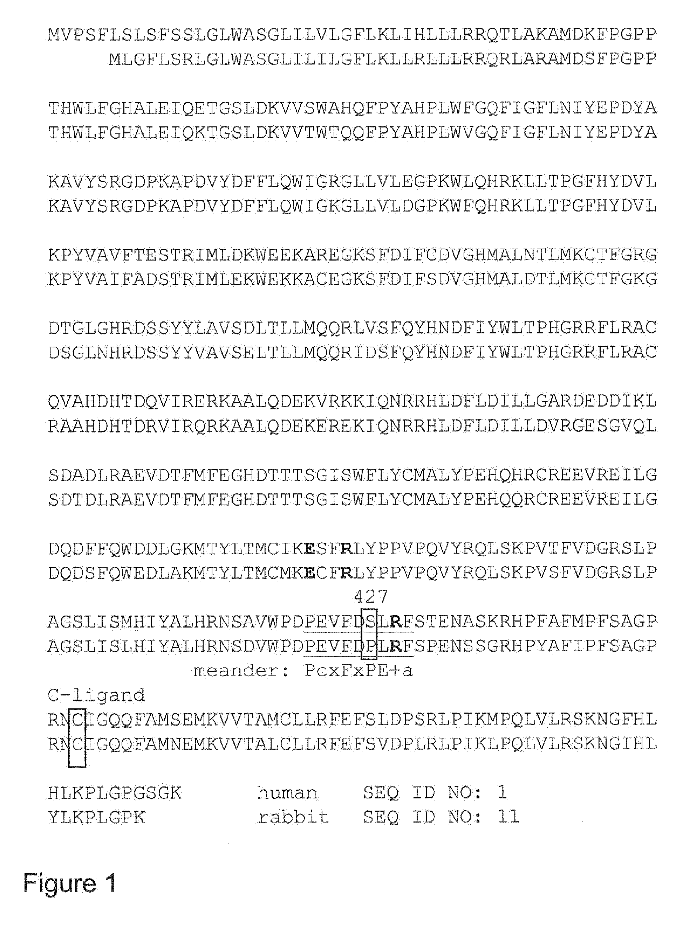

It was now surprisingly found that the enzymatic activity of the human CYP4B1 protein can be re-established by exchanging at least a part of the human CYP4B1 protein by a corresponding part of a CYP4B1 protein of a different species. The term "corresponding" as used herein means that a part of a gene or protein shows structural or functional homology to the gene or protein of a different species. As an example, corresponding regions of a DNA or protein show high sequence similarities as e.g. revealed by an alignment. As a further example, corresponding regions may comprise similar functional domains as e.g. binding sites, enzymatic restriction sites, active structures, such as the meander region of CYP4B1 (**Figure 1**).

When exchanging parts of the native human CYP4B1 protein to the corresponding parts of an enzymatically active CYP4B1 protein of a different species, the resulting modified human CYP4B1 protein shows enzymatic activity and is able to process 4-IPO into highly toxic alkylating metabolites. Accordingly, a cell carrying the modified CYP4B1 protein undergoes apoptotic cell death as a consequence of accumulating 4-IPO metabolites when treated with 4-IPO. Thus, a novel suicide gene system is provided based on a modified human CYP4B1 protein. This suicide gene system is suited for various applications in which a cell should be able to be deliberately eliminated if necessary.

One possible application is in transplantation, when allogeneic cells or organs are introduced into a patient's body. These cells differ from the recipient's own cells and are thereby likely to be recognized as an antigen by the recipient's immune system. This can cause strong immune reactions against the transplanted cells, so-called "rejection reactions". These reactions can seriously endanger the patient's health or even life, such that it becomes necessary to remove the transplanted cells or organ from the body. When an entire organ is transplanted this might be done by surgery. However, when transplanting small pieces of tissue or even individual cells, as e.g. for stem cell transplantation, a removal of the donor cells by surgery might not be possible. In this case, the transplanted cells can be eliminated by administration of 4-IPO if they were equipped with the modified human CYP4B1 suicide gene system prior to transplantation. Since only the modified human CYP4B1 protein is able to process 4-IPO, the recipient's cells are not affected by the administration of 4-IPO.

Another possible application is in transplantation, when autologous or allogeneic cells, that were introduced into a patient's body for therapeutic purposes, undergo malignant transformation and thus give rise to a tumor. In this situation - especially if the tumor cannot be removed surgically or if the tumor cells have already spread to different structures in the body, an important therapeutic safety measure is to eliminate these transformed cells. In this scenario, the tumor cells can be eliminated by administering 4-IPO, if the transplanted cells had been equipped with the modified human CYP4B1 suicide gene prior to transplantation. This toxicity will be restricted to the transplanted cells including the tumor cells, as the normal cells of the recipient that only express the wild-type inactive human CYP4B1 will not be affected.

A further application of the modified human CYP4B1 suicide gene system is in donor lymphocyte infusion in the course of treating relapsed acute or chronic leukemia. The risks of immune reactions are particularly high when large amounts of cells are introduced into the human body as it is the case during donor lymphocyte infusion following hematopoietic stem cell transplantation. The fresh immunocompetent donor T-cells are transplanted to mediate anti-leukemia graft-versus-leukemia (GvL) reactions. However, as they can also recognize healthy tissue cells of the patient as a potential target, they can induce serious graft-versus-host disease (GvHD). In this case it is vital to remove the donor T-cells from the patient's body, since there is a high risk to die from GvHD. By introducing the modified human CYP4B1 suicide gene system into the donor T-cells prior to transplantation, the cells can be deliberately eliminated once GvHD symptoms arise through the application of 4-IPO.

Since only part of the CYP4B1 protein is replaced by the amino acid sequence of a different species, the modified human protein shows reduced risk to trigger responses of the recipient's immune system. In addition, in contrast to the HSV-tk/ganciclovir system, the CYP4B1/4-IPO suicide gene system shows no bystander effect, i.e. the recipient's cells are not affected by the administration of 4-IPO, because they are not able to process the compound. This makes the system particularly suitable for donor lymphocyte infusion applications, because the donor T-cells infiltrate the recipient's organs. The modified human CYP4B1 suicide gene system is further advantageous, since it acts independently of cell proliferation. This is particularly important for donor lymphocyte infusion applications, because in chronic GvHD the autoimmune T-lymphocytes have a relatively low proliferative activity and cannot be eliminated by cell proliferation dependent suicide gene systems as e.g. HSV-tk/gancidovir.

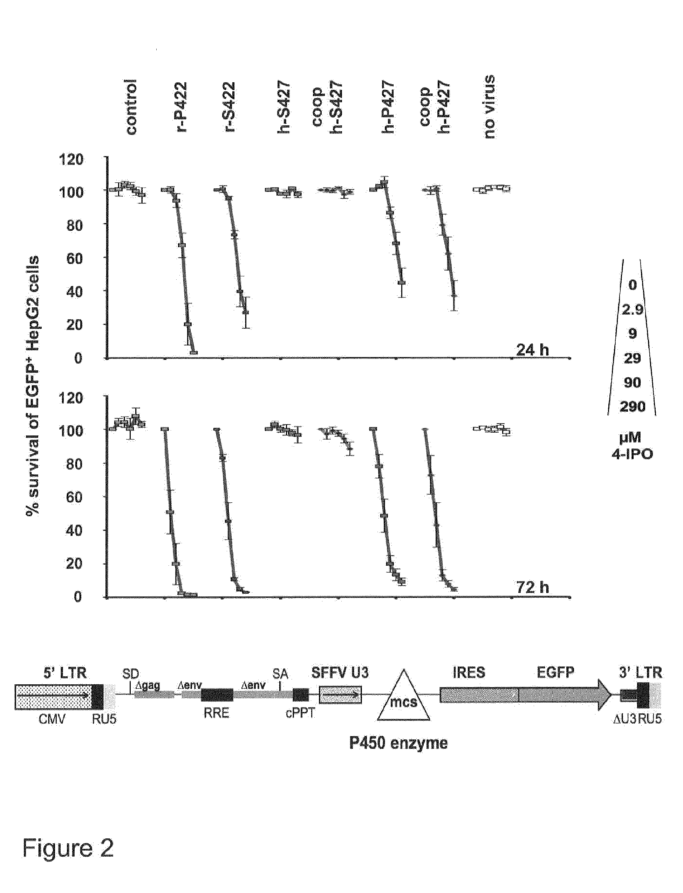

In a preferred embodiment, the part of the CYP4B1 protein, which is replaced, comprises at least one amino acid, preferably about 10 to about 300 amino acids, more preferred about 10 to about 150 amino acids, most preferred about 10 to 100 amino acids. The human CYP4B1 protein differs from CYP4B1 proteins from other species *inter alia* in that it has a serine within the meander region at a position where the CYP4B1 proteins of other species carry a proline. Interestingly, the proline in the meander region is highly conserved in all CYP4B1 proteins from different species, such as fish, primates and rodents as well as in other CYP proteins of the Cytochrome P450 superfamily. The conserved proline residue is part of a proline-X-arginine/histidine motif within the meander region, which is destroyed in the human CYP4B1 due to a serine at the respective position, i.e. at amino acid position 427. A representative alignment between the human CYP4B1 protein and the rabbit CYP4B1 protein is shown in Figure 1. It is believed that the human CYP4B1 protein has a different structural arrangement due to the lack of the proline residue and that this renders the human CYP4B1 protein inactive. Thus, in a particularly preferred embodiment, the part of the human CYP4B1 protein, which is replaced, comprises a serine to proline exchange in the meander region, preferably at amino acid position 427 of the human CYP4B1 protein. The indication for the amino acid position refers to the human CYP4B1 variant given in SEQ ID NO: 1, which is the human CYP4B1 variant without the additional insertion. However, a modified human CYP4B1 protein may be provided on the basis of the human CYP4B1 variant according to SEQ ID NO: 20. In the context of the inventions, it has been found that by exchanging the serine within the meander region of the human CYP4B1 protein with a proline, the resulting modified human CYP4B1 protein is rendered active for processing 4-IPO (**Figure 2**). This was revealed by transducing cells with the native human CYP4B1 protein or with the modified human CYP4B1 protein and applying 4-IPO to the cells. The cells harboring the native CYP4B1 protein are unable to metabolize 4-IPO and therefore do not accumulate cytotoxic products. Accordingly, the survival rates of these cells are comparable to the survival rate of non-transduced control cells. In contrast, the survival rates of cells transduced with the modified human CYP4B1 protein show a reduction in cell survival rates of about 50 % (**Figure 2**). This clearly demonstrates that the modified human CYP4B1 protein is active and therefore metabolizes 4-IPO into cytotoxic products.

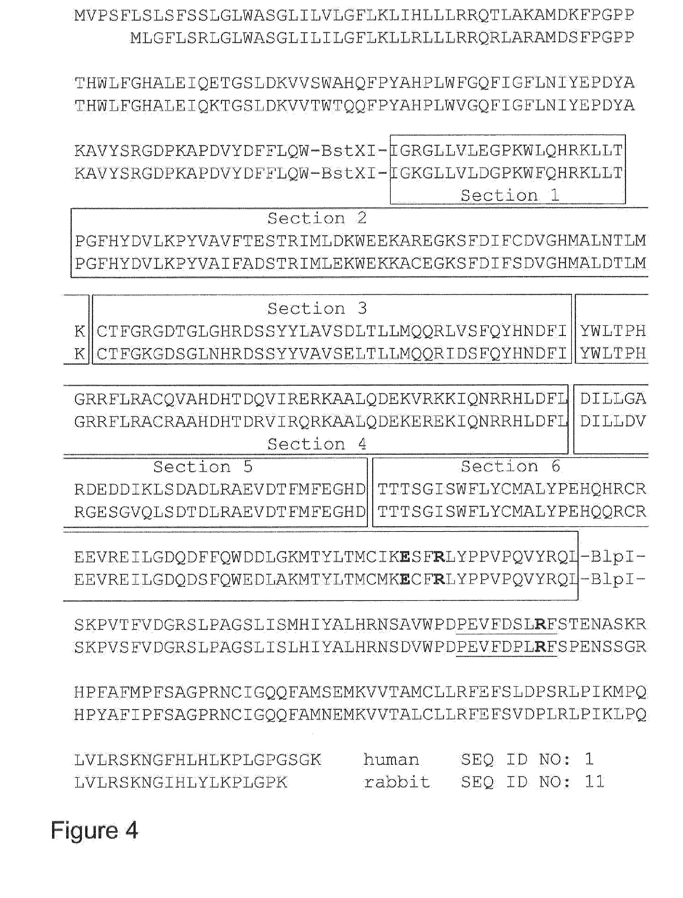

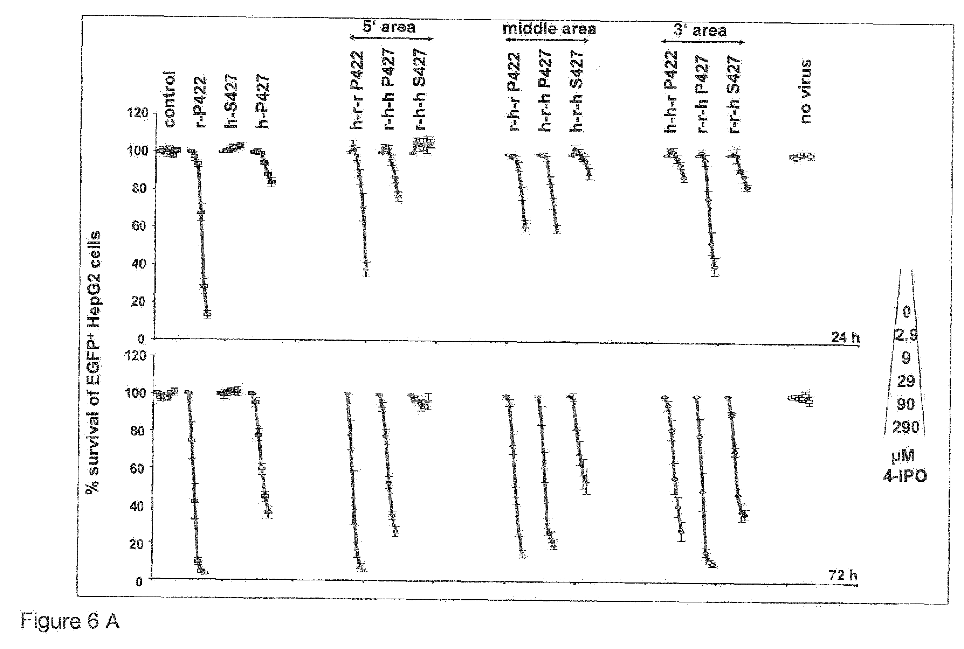

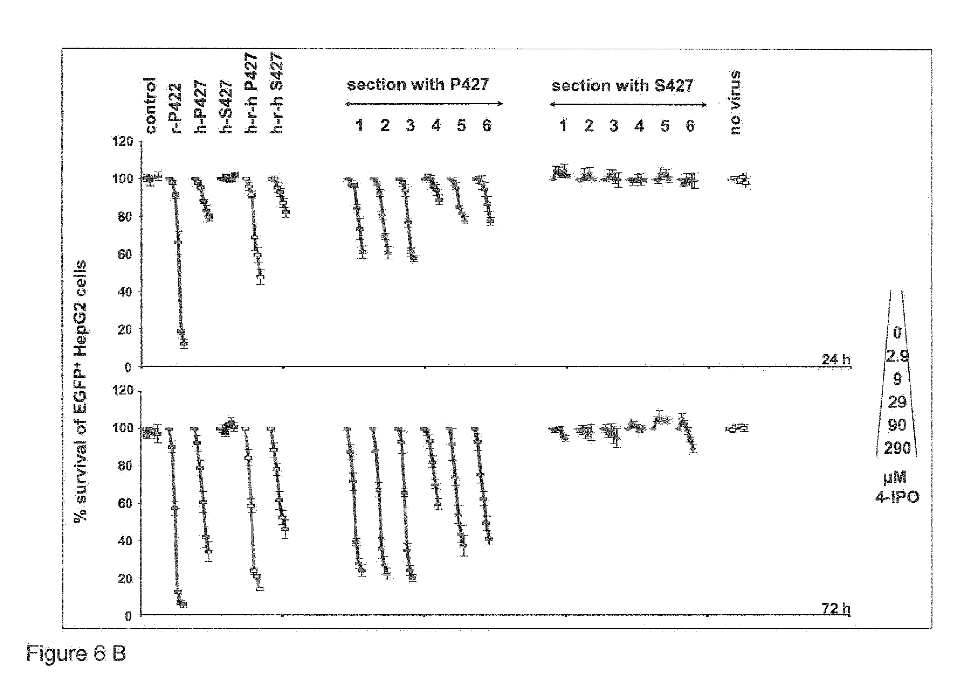

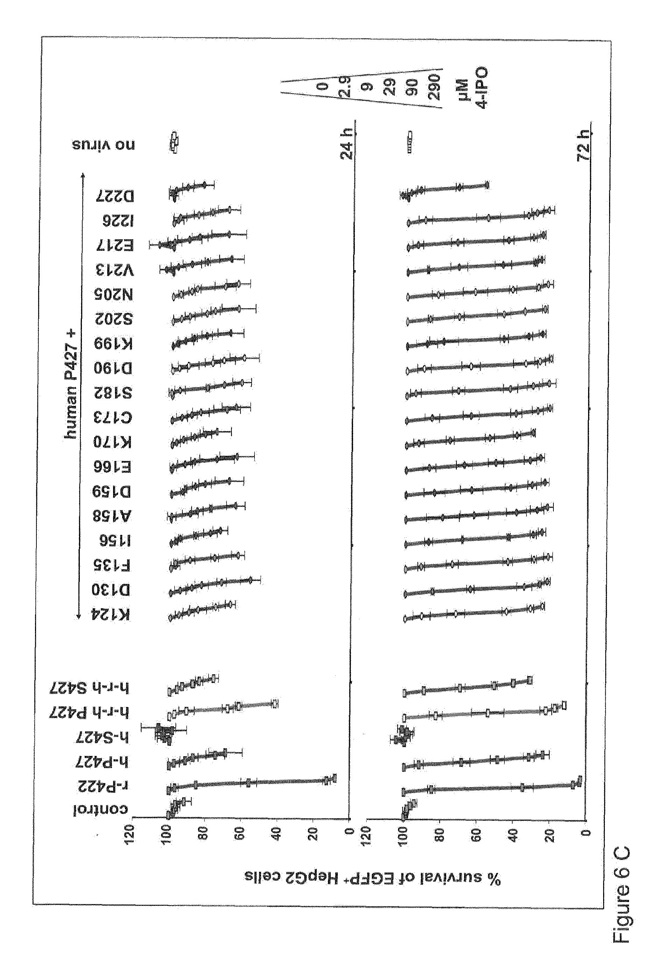

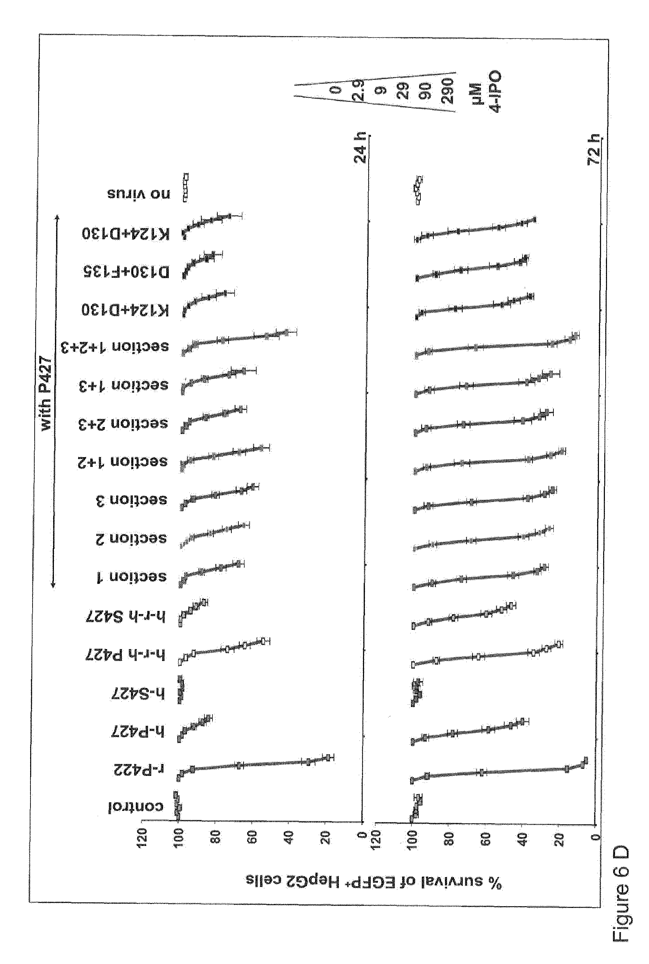

In a preferred embodiment, the part of the CYP4B1 protein, which is replaced, comprises the 5'-region, the middle region, the 3'-region of the CYP4B1 protein and/or a part of any of the regions. Further analyzing the differences between the non-active human CYP4B1 and the highly active rabbit CYP4B1, while carrying out the invention, the CYP4B1 protein was divided into three different regions, i.e. the 5'-region, the middle region and the 3'-region. The respective regions were defined using the restriction enzyme consensus sites for BstXI and BIpl, which are found in the cDNA of the human CYP4B1 as well as the rabbit CYP4B1 (**Figure 4**). Each of the three regions of the human CYP4B1 protein was exchanged for the corresponding region of the rabbit CYP4B1 protein, resulting in the proteins r-h-h-S427, h-r-h-S427 and h-h-r-P422 (**Figure 6** **A**). These experiments revealed that by replacing the middle region or the 5'-region of the human CYP4B1 protein by the corresponding part of the rabbit CYP4B1 protein, the non-active human CYP4B1 protein can be transformed into an active modified human CYP4B1 protein. The resulting modified human CYP4B1 proteins were able to process 4-IPO and, accordingly, cells transduced with any of these modified human CYP4B1 proteins showed a decreased cell survival upon administration of 4-IPO compared to cells having a native human CYP4B1 protein (**Figure 6** **A**). Based on the CYP4B1 protein variant according to SEQ ID NO: 1 the 5'-region comprises amino acids 1 to 121 (SEQ ID NO: 4), the middle region comprises amino acids 122 to 387 (SEQ ID NO: 5) and the 3'-region comprises amino acids 388 to 511 (SEQ ID NO: 6). Accordingly, in a preferred embodiment of the invention, the part of the human CYP4B1 protein comprises amino acids 1 to 121, amino acids 122 to 387, or amino acids 388 to 511.

In a preferred embodiment, the different species is a non-human mammal, preferably selected from the group consisting of rabbit, dog, rat, mouse, chimpanzee and gorilla. In contrast to the human CYP4B1 protein, the CYP4B1 proteins of any other species so far analyzed were able to process 4-IPO, although with differing efficiencies. Thus, parts of the human CYP4B1 may be replaced by corresponding parts of CYP4B1 protein of any other species to render the modified human CYP4B1 active. However, CYP4B1 proteins of those species are preferred, that show particular high activity and / or high protein similarity with the human CYP4B1 protein, because the resulting hybrid protein would be highly similar to the native human CYP4B1 and thus less likely to induce immune responses.

In a preferred embodiment, the 5'-region of the human CYP4B1 protein is replaced by the corresponding 5'-region of the rabbit CYP4B1 protein, the middle region of the human CYP4B1 protein is replaced by the corresponding middle region of the rabbit CYP4B1 protein and/or the 3'-region of the human CYP4B1 protein is replaced by the 3'-region of the rabbit CYP4B1 protein. When analyzing CYP4B1 proteins from different species, the rabbit CYP4B1 showed the highest activity in processing 4-IPO, thus the rabbit CYP4B1 is particularly preferred for exchanging individual regions, sections or amino acids of the human CYP4B1. Based on the protein sequence of the rabbit CYP4B1 protein as given in SEQ ID NO: 11, the 5'-region comprises amino acids 1 to 116 (SEQ ID NO: 13), the middle region comprises amino acids 117 to 382 (SEQ ID NO: 14) and the 3'-region comprises amino acids 383 to 503 (SEQ ID NO: 15).

In a preferred embodiment, the modified human CYP4B1 protein has at least 90 %, preferably at least 95 % identity in its amino acid sequence with the native human CYP4B1 protein. Compared to the rabbit CYP4B1, the native human CYP4B1 has only > 1% activity, although the amino acid sequence of both enzymes shows a striking high similarity of about 84 % (Figure 1). Nevertheless, this difference is sufficient for the human immune system to recognize the rabbit CYP4B1 protein as a foreign antigen. Thus, using an entire CYP4B1 protein from another species is not possible, because the transduced cells would be potentially eliminated by the recipient's immune system. To reduce the risk of rejection reactions against the transduced cells, the modified human CYP4B1 protein should be as similar to the native human CYP4B1 protein as possible. For example, exchanging serine 427 to proline and replacing the middle region of the human CYP4B1 by the middle region of the rabbit CYP4B1 results in a modified human CYP4B1 having 92 % identity with the native human CYP4B1 protein. Exchanging serine 427 to proline and replacing amino acids 124 - 227 of the human CYP4B1 by amino acids 119-222 of the rabbit CYP4B1 results in a modified human CYP4B1 even having 96,3 % identity with the native human CYP481 protein.

In a preferred embodiment, the part of the human CYP4B1 protein, which is replaced, comprises a serine to proline exchange in the meander region and the 3'-region, the middle region, the 5'-region of the CYP4B1 protein and/or a part of any of these regions. The activation of the human CYP4B1 protein by replacing parts of the proteins with the corresponding parts of the rabbit CYP4B1 protein is increased if one of the regions (5'-, middle or 3'-region) is replaced and the modified human CYP4B1 protein additionally comprises a proline in the meander region (r-h-h-P427 (SEQ ID NO.: 65), h-r-h-P427 (SEQ ID NO.: 67), h-h-r-P422 (SEQ ID NO.: 69); **Figure 6** **A**). Transducing cells with these modified human CYP4B1 proteins reduces cell survival rates to less than 20 % compared to controls having a native human CYP4B1 protein (**Figure 6** **A**).

In a preferred embodiment, at least one part of the human CYP4B1 protein, which is replaced, consists of amino acids 124 to 135, amino acids 156 to 190 and/or amino acids 129 to 227, preferably consists of amino acids 124 to 227. As described above, replacing the middle region of human CYP4B1 protein by the corresponding region of rabbit CYP4B1 protein, results in a particular active modified human CYP4B1 protein. Therefore this region was further analyzed by dividing it into six sections (**Figure 4**). Sections 1 to 3 cover amino acids 124 to 135 (SEQ ID NO: 7), amino acids 156 to 190 (SEQ ID NO: 8) and amino acids 199 to 227 (SEQ ID NO: 9), respectively. Replacing any of these sections increased the 4-IPO processing activity of the resulting modified human CYP4B1, in particular if the modified human CYP481 also had a serine to proline exchange in the meander region. Thus, in a particularly preferred embodiment, the modified human CYP4B1 protein has a serine to proline exchange in the meander region and amino acids 124-135, amino acids 156-190 and/or amino acids 199-227, are replaced by a corresponding part of a CYP4B1 protein of a different species. If replaced by the corresponding sections of the rabbit CYP4B1 protein, the respective sections are amino acids 119 to 130 (SEQ ID NO: 16) for section 1, amino acids 151 to 185 (SEQ ID NO: 17) for section 2 and amino acids 194 to 222 (SEQ ID NO: 18) for section 3. Accordingly, in a further preferred embodiment, amino acids 124-135 of the human CYP4B1 protein are replaced by SEQ ID NO: 16, amino acids 156-190 of the human CYP4B1 protein are replaced by SEQ ID NO: 17 and/or amino acids 199-227 of the human CYP4B1 protein are replaced by SEQ ID NO: 18.

Surprisingly, it has been further found that exchanging sections 1, 2 and 3 (i.e. amino acids 124 to 227 of the human CYP4B1 protein, SEQ ID NO: 10) by the corresponding part of the rabbit CYP4B1 protein (i.e. amino acids 119 to 222, SEQ ID NO: 19) in combination with a serine to proline exchange in the meander region, cell survival rates were reduced to about 10 % within 72 hours upon applying 4-IPO (**Figure 6** **D**). Thus, this modified human CYP4B1 protein shows the most efficient 4-IPO processing, with an enzymatic activity that is even better than that of a modified human CYP4B1 protein having the entire middle region exchanged (**Figure 6** **A**). In fact, a modified human CYP4B1 protein with sections 1 to 3 exchanged for the corresponding parts of the rabbit CYP4B1 protein and having a serine to proline exchange in the meander region shows almost the same 4-IPO processing activity as the native rabbit CYP4B1 protein.

Accordingly, in a second aspect, the invention is directed to a modified human CYP4B1 protein with a serine to proline exchange in the meander region, wherein amino acids 124 to 227 consist of SEQ ID NO: 19. Preferably, the serine to proline exchange is at position 427.

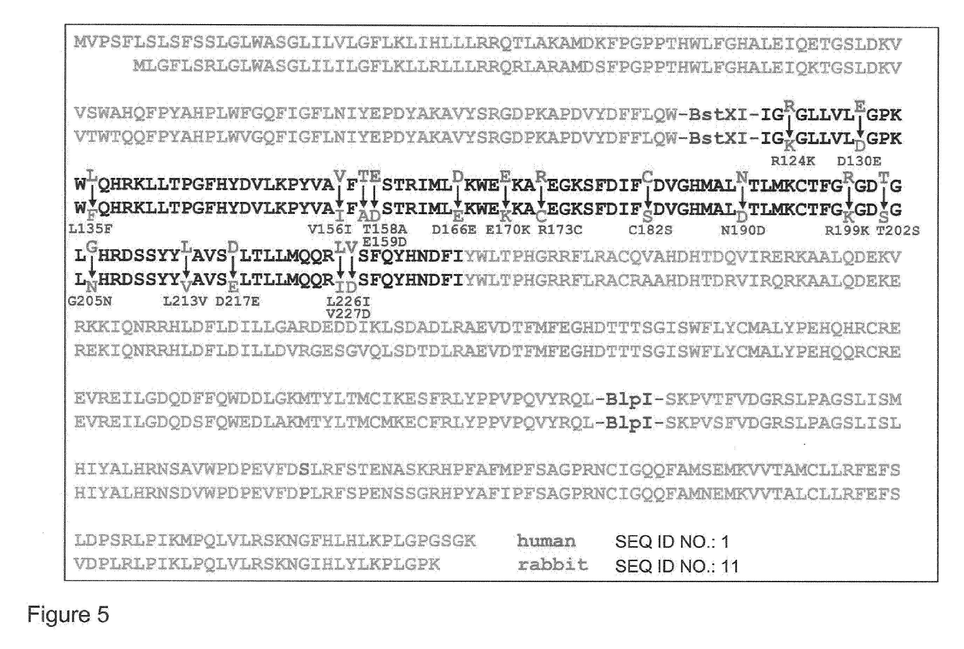

In addition, the modified human CYP4B1 protein having a serine to proline exchange at position 427 and SEQ ID NO: 19 at amino acid position 124 to 227, differs from the native human CYP4B1 protein in only 19 amino acids. These alterations are at amino acid position 124 (arginine → lysine), 130 (glutamic acid → aspartic acid), 135 (leucine → phenylalanine), 156 (valine → isoleucine), 158 (threonine → alanine), 159 (glutamic acid → aspartic acid), 166 (aspartic acid → glutamic acid), 170 (glutamic acid → lysine), 173 (arginine → cysteine), 182 (cysteine → serine), 190 (asparagine → aspartic acid), 199 (arginine → lysine), 202 (threonine → serine), 205 (glycine → asparagine), 213 (lysine → valine), 217 (aspartic acid → glutamic acid), 226 (lysine → isoleucine), 227 (valine → aspartic acid) and 427 (serine → proline) (**Figure 5**). Due to the few alterations, this modified human CYP4B1 protein is unlikely to induce immune responses and is therefore particularly advantageous.

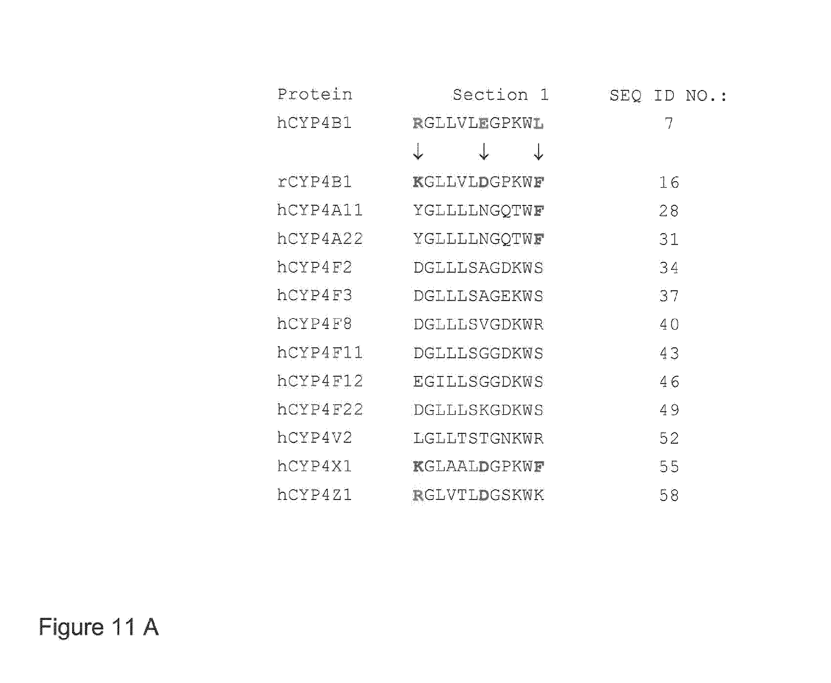

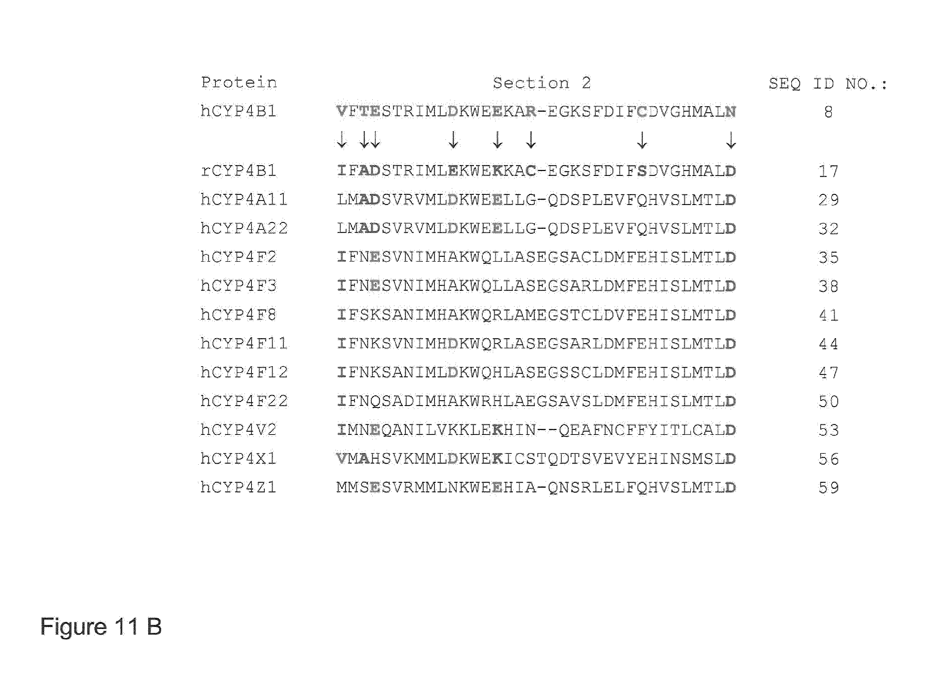

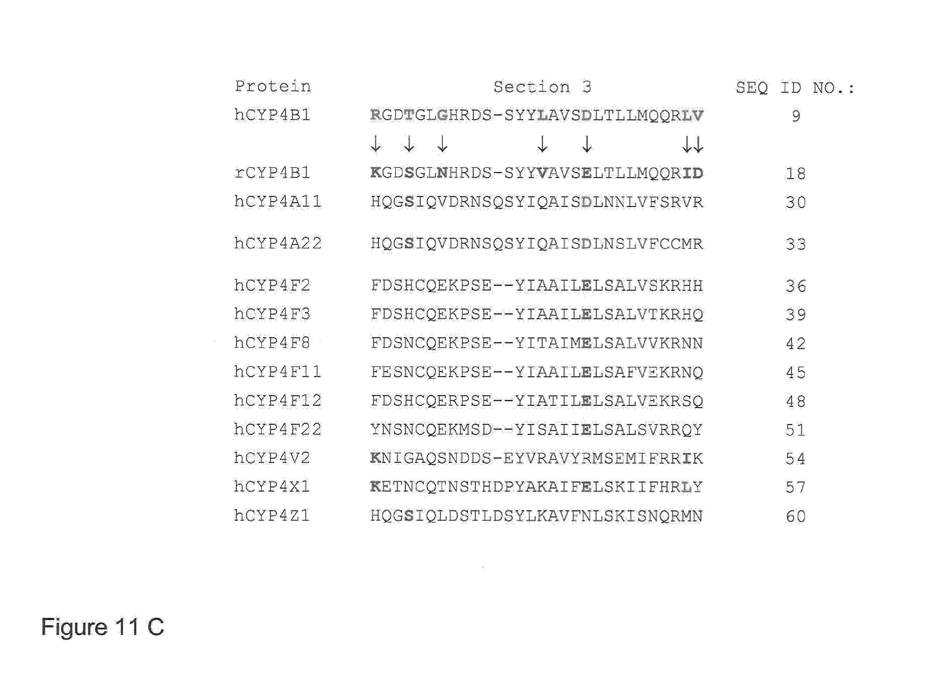

Interestingly, twelve of the eighteen amino acids, which differ in sections 1 to 3 between the native human CYP4B1 and the native rabbit CYP4B1, are also found in other human CYP4 proteins (**Figure 11**). Thus, a modified human CYP4B1 protein with a serine to proline exchange at position 427 and the following amino acids alterations is particularly preferred, since it is most similar to human CYP4 family proteins and thus is particularly unlikely to induce immunogenic responses: amino acid position 124 (arginine → lysine), 130 (glutamic acid → aspartic acid), 135 (leucine → phenylalanine), 156 (valine → isoleucine), 158 (threonine → alanine), 159 (glutamic acid → aspartic acid), 170 (glutamic acid → lysine), 190 (asparagine → aspartic acid), 199 (arginine → lysine), 202 (threonine → serine), 217 (aspartic acid → glutamic acid), and 226 (lysine → isoleucine).

In a further aspect, the invention is directed to a nucleic acid encoding for a modified human CYP4B1 protein, wherein at least one part of the human CYP4B1 protein is replaced by a corresponding part of a CYP4B1 protein of a different species. By means of a nucleic acid it is possible to introduce a modified human CYP4B1 protein into a cell or organism. This can be achieved either directly or via an expression vector system, e.g. based on viral, non-viral transduction or on bacterial plasmid technology.

In a preferred embodiment, the nucleic acid is a DNA or RNA. Depending on the system used to introduce the nucleic acid into a cell for expressing the modified human CYP4B1 protein, DNA or RNA may be advantageous. For example, non-viral transduction and bacterial transduction systems are usually based on DNA, whereas some of the viral transduction systems, as e.g. retroviral transduction systems, are based on RNA constructs.

In a preferred embodiment, the nucleic acid is optimized for human codon usage. Human "codon usage" optimization is a method for increasing mRNA stability and translation efficiency. This is achieved for example, by excluding seldom codons and disadvantageous secondary structures as well as by establishing an even distribution of GC-base pairs. When the nucleic acid encoding for modified human CYP4B1 protein is optimized for human codon usage, cell survival rates decrease upon administration of 4-IPO from about 45 % (h-P427) to 37 % (codon-optimized (coop) H-P427) (**Figure 2**). The codon-optimized cDNA of the native human CYP4B1 protein is given in SEQ ID NO: 3.

In a preferred embodiment, the nucleic acid comprises a sequence selected from the group consisting of SEQ ID NO: 3, 62, 64, 66, 68, 70, 72, 74, 76, 78, 80, 82, 84, 86, 88, 90 and 92.

In a further aspect, the invention is directed to a plasmid comprising a nucleic acid encoding for a modified human CYP4B1 protein, wherein at least one part of the human CYP4B1 protein is replaced by a corresponding part of a CYP4B1 protein of a different species. Preferably, the plasmid comprises any of the above-mentioned nucleic acids.

In a further aspect, the invention is directed to an expression vector comprising a nucleic acid, preferably DNA or RNA encoding for a modified human CYP4B1 protein, wherein at least one part of the human CYP4B1 protein is replaced by the corresponding part of the CYP4B1 protein of a different species. The expression vector, also called expression construct, may be of viral, non-viral or bacterial origin. Expression vectors are used to introduce genes or gene constructs into target cells. Thus, the expression vector preferably comprises all properties needed to ensure expression of the introduced gene within the target cell or organism.

In a preferred embodiment, the nucleic acid, preferably DNA or RNA encoding for the modified human CYP4B1 protein is under control of a constitutively active promoter, preferably selected from the group consisting of a SFFV promoter, a heft-alpha promoter, a hPGK promoter and an ubiquitin promoter. When the modified human CYP4B1 protein is expressed from a constitutively active promoter, the protein will always be present in the cell carrying the expression vector. Thus, once the cell comes into contact with 4-IPO, the modified human CYP4B1 protein immediately processes the compound into its toxic metabolites. These metabolites accumulate within the cell and intercalate into its DNA, such that apoptotic processes are induced and lead to a fast elimination of the cell. Thus, by using a constitutively active promoter to drive expression of the modified human CYP4B1 protein, an immediate response of the modified cell to the administration of 4-IPO can be assured. This is advantageous where a fast and efficient elimination of all modified cells is needed.

In a preferred embodiment, the expression of the DNA or RNA encoding for the modified CYP4B1 protein is under the control of an activation-dependent promoter, preferably selected from the group consisting of a HLA-DR promoter, a CD69 promoter, a CD25/(IL2-receptor alpha-chain) promoter, a tetracycline-inducible promoter, an oestrogen-inducible promoter and a tamoxifen-inducible promoter. Using activation-depended or expression-inducible promoters to drive the modified human CYP4B1 protein expression will ensure that the protein is only produced if a certain internal or external trigger is present. When using the modified human CYP4B1 protein suicide gene system for donor-lymphocyte-infusions, it is particularly preferred to put the modified human CYP4B1 protein under a HLA-DR promoter or a CD96 promoter. In T-cells, both promoters are only active if the T-cells are activated, which means if the donor T-cell reacts to the recipient's cells, thereby inducing GvHD. Thus, the modified human CYP4B1 protein will only be expressed in those T-cells, which are activated against normal liver, skin or intestinal cells of the host and thus endanger the recipient's health. Upon administration of 4-IPO, these activated transgenic cells process the compound into its cytotoxic metabolites and subsequently undergo apoptosis. The transgenic donor T-cells, however, that do not react against the recipient's normal cells are not eliminated, since they do not actively express the modified human CYP4B1 protein, and are thus preserved and still available to kill leukemia cells.

In a preferred embodiment, the expression vector comprises a selection marker, preferably selected from the group consisting of either naturally or artificially truncated human CD34, low affinity NGF-receptor, huEGFRt and CD19 proteins. When using transduced cells for transplantation it is particularly important that as many transplanted cells as possible, preferably all, indeed comprise the transduced suicide gene construct. Since it is rarely ever possible to reach transduction rates of 100 %, it is necessary to specifically select and enrich for those cells which have been successfully transduced. A clinically applicable selection marker should fulfill the following criteria. It should be of human origin to prevent an immune response, it should show specific expression only in the genetically modified cells, it should not disturb the physiological function of the target cell and it should be compatible with fast selection processes for ex vivo cultivation. Based on these requirements, a selection using cell surface markers that can be purified on magnetic columns (such as clinical MACS system from Miltenyi Biotech, Germany) is preferred. The cytoplasmatically truncated CD34 version of the human stem cell antigen can serve as a human surface marker for selection of transduced T-cells, since physiologically this antigen is not expressed on T-lymphocytes. tCD34 is a naturally occurring splice variant of the CD34 antigen, in which the recognition site for signal transduction is deleted, such that the residual cytoplasmatic tail of tCD34 comprises only 16 amino acids. Modified human primary T-cells expressing tCD34 can be enriched to high purity (over 95 %) using commercially available immunomagnetic columns, which allow selection to Good Manufacturing Practice (GMP) standards and thus are approved for clinical application. The cytoplasmatically truncated low-affinity nerve growth factor receptor P75 can be used for purifying cells by CD271-specific magnetic beads. In the truncated huEGFRt, the extracellular N-terminal ligand binding domain and the intracellular signaling activity are deleted. Therefore, surface expression of this modified huEGFRt receptor can be used to separate huEGFRt expressing cells immunomagnetically by using biotinylated cetuximab (pharmaceutical grade anti-EGFR monoclonal antibody). In addition, the CD19 B-cell surface marker is a suitable selection marker, because the cytoplasmatic tail of CD19 is deleted in the truncated version, thus removing the intracellular signalling domains. CD19 is not expressed on T-cells and therefore can be used to specifically enrich genetically modified donor T-cells. By CD19 immunomagnetic selections, transduced cells can be enriched up to over 95 % purity.

In a preferred embodiment, the expression vector comprises an ubiquitous chromatin opening element (UCOE), an insulator sequence and/or a scaffold/matrix attachment region (S/MARs). UCOEs are methylation-free CpG islands associated with dual divergently transcribed promoters, which create a transcriptionally active, open chromatin environment around an integrated transgene preventing heterochromatin formation and transgene silencing. UCOEs are able to confer reproducible and stable transgene expression. The ubiquitous chromatin opening element (UCOE), derived from the human HNRPA2B1/CBX3 locus (A2UCOE) seems to be promising as a chromatin regulatory element which can provide reproducible and high stable transgene expression within a lentiviral gene therapy vector. The use of this fragment can significantly limit the size of transgene constructs that can be expressed, however, other groups have recently engineered a novel UCOE fragment of only 0.7 kb. Chromatin insulator sequences can shield genes against position effects of surrounding chromatin and have enhancer-blocking activity. These properties are desirable for a constant, reliable and safe transgene expression using randomly integrating vectors. Also insulator sequences can be introduced into the 3' LTR SIN U3 for protecting the integrated provirus from influences of the surrounding genome and thereby improving and stabilizing the transgene expression. The most commonly used insulator is the chicken hypersensitive site-4 (cHS4) element. The scaffold/matrix attachment regions (S/MARs) are genomic DNA sequences, which bind chromatin to the nuclear matrix. They are ubiquitous and highly conserved in eukaryotic chromosomes and thus provide only a low potential immunological risk. In addition, S/MAR sequences can prevent epigenetic silencing by protecting the transgenic sequence against the heterochromatin and ensure transcriptional active chromatin environment. The S/MAR elements mediate the complex organization of chromatin and thereby take effect on the regulation of gene expression.

In a preferred embodiment, the expression vector further comprises a nucleic acid encoding cytochrome-P450 reductase. Human P450 cytochrome enzymes perform monooxygenase reactions by interacting with a NADPH-dependent cytochrome-P450 reductase (POR). The cytochrome-P450 reductase provides the electrons needed by the cytochrome P450 enzyme to activate atmospheric oxygen. It was found that this transfer of electrons is the time limiting step in the mono-oxygenase reaction. Therefore, for increasing the reaction rate, it is advantageous to provide the cofactor, i.e. the NADPH-dependent reductase, together with the modified human CYP4B1 protein. The modified human CYP4B1 protein and the NADPH-dependent reductase may be expressed from the same expression vector e.g. separated by an IRES-site or as a fusion protein.

In a preferred embodiment the expression vector is a viral vector. Some of the most promising and best established systems to introduce foreign DNA into a target cell are based on viral systems. These systems take advantage of the natural ability of viruses to infect cells and to introduce their nucleic acid into the cell plasma or even into the cellular genome. For clinical transduction of T-cells, retroviral vectors are the gene transfer system of choice. Based on the evolutionary optimization, retroviral vectors efficiently integrate into the cellular genome of the target cells and thus facilitate stable and long-lasting expression of the transgene.

In a preferred embodiment, the viral vector is selected from the group consisting of an adenoviral vector, an adeno-associated viral vector and a retroviral vector, preferably a gammaretroviral, a lentiviral or a foamyviral vector. Foamyviral vectors are particularly preferred, because they are natively absent in humans. In general, foamy virus infections lead to chronic persistence of the wild type virus in different organ systems. It is therefore absolutely striking that despite the wide natural prevalence in primates/monkeys (more than 80 % of animals in captivity are positive) and their co-development in their hosts for long periods of time, there is absolutely no toxicity or pathology associated with foamy virus infection and persistence in their natural hosts. Accidentally infected humans, usually animal keepers that that have been bitten by infected animals, also never showed any foamyvirus associated health problems. This complete apathogenicity qualifies foamyviral vectors as ideal gene transfer vehicles for genetic therapies in humans and clearly distinguishes foamy viral vectors as gene delivery systems from HIV1-derived and other retrovirus-derived vectors. Foamyviral vectors are further preferred for gene therapy applications, because they can accommodate large transgenes (over 9 kb), efficiently transduce slowly dividing cells, and integrate as a provirus into the genome of target cells, thus enabling stable long term expression of the transgene (Rethwilm, 2007). In addition, foamyviral vectors achieve high transduction efficiency in pluripotent hematopoietic stem cell and have been successfully used in animal models to correct monogenetic diseases such as leukocyte adhesion deficiency in dogs and Fanconi anemia in mice (Cai et al., 2008).

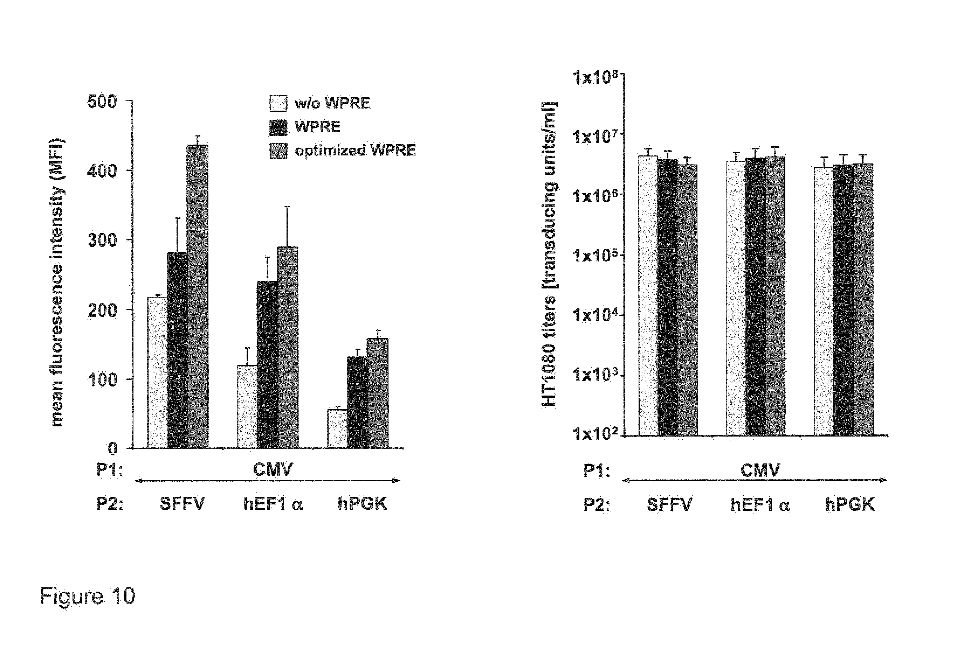

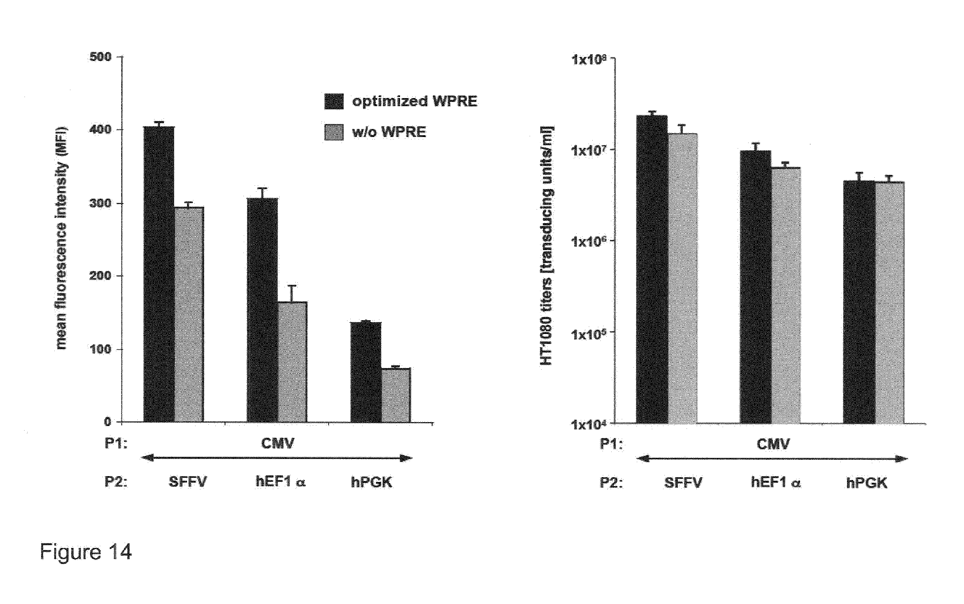

In a preferred embodiment, the viral vector comprises an optimized post-transcriptional regulatory element of woodchuck hepatitis virus (WRPO). The woodchuck hepatitis virus has a post-transcriptional regulatory element (WPRE = woodchuck hepatitis virus post-transcriptional regulatory element) which is responsible for an efficient expression of the viral transcripts. It has been shown that WPRE is able to sufficiently increase expression from retroviral and in particular lentiviral and foamyviral expression constructs, because it improves the transport of non-spliced mRNA from the nucleus thereby enabling an increased protein expression. A further improved variant of the WPRE (WPRO) differs from WPRE by deletion of the 3'-X-protein-promoter. This variant shows a similar increase in transgene expression but additionally provides an improved security mechanism since an unintentional expression from the X-protein promoter is not possible. Using WPRE the expression of the modified human CYP4B1 protein could be increased at least 1.5 times (**Figure 10** **and** **14**).

In a preferred embodiment, the virus particle is produced using a packaging system comprising a first expression plasmid encoding gag, a second expression plasmid encoding pol and a third expression plasmid encoding env. To increase the security of the gene transfer system, replication deficient viruses have been developed, in which the genes gag, pol, and env are provided in separate expression plasmids and lack a LTR or packaging signal. Thereby, none of the viral structural genes is included in the viral particle and the transduced target cell will not be able to produce a functional virus. Thus, the virus particle will only comprise the packaging vector with the transgene. In addition, it is preferred to use a self-inactivating vector which lacks enhancer and promoter sequences in the 3'-LTR-region. Following reverse transcription in the target cell, the deleted 3'-LTR will be copied to the 5'-LTR such that an inactive 5'-LTR is formed in the integrated provirus. These modifications increase the security of antiviral packaging systems, since they reduce the risk of pro-oncogene activation and unintended mobilization of the vector.

In a further aspect, the invention is directed to a cell comprising a modified CYP4B1 protein, wherein at least one part of the human CYP4B1 protein is replaced by the corresponding part of a CYP4B1 protein of a different species. In general, gene therapy is based on introducing genetic material (a transgene) into cells to correct cellular defects or to equip the cells with additional functions. Introducing foreign genes into a target cell, however, always bears the risk of undesired biological reactions, either from the modified cell itself or from an organism into which the modified cell is introduced. In particular, in clinical applications introducing modified cells into the human body can trigger strong immune responses, which can seriously threaten the patient's life. Therefore, it is particularly important to maintain a possibility to remove the modified cells from the body if necessary, preferably without killing any of the patient's endogenous cells. This can be achieved by providing a suicide gene system comprising a modified human CYP4B1 protein. The modified cells are equipped with the suicide gene system before introducing them into the patient's body. They may be specifically eliminated if needed by the administration of 4-IPO. The modified cells expressing the modified human CYP4B1 protein take up 4-IPO and transform it into cytotoxic metabolites within the cell. The metabolites intercalate into the DNA, disturb DNA to protein interactions, generate DNA strand breaks and thereby induce apoptotic cell death. Thus, in contrast to endogenous unmodified cells of the patient, which do not express an enzymatically active CYP4B1 protein, the modified cells will be specifically eliminated upon administration of 4-IPO. Since the processing of the 4-IPO occurs within the modified cells, the suicide gene system based on the modified human CYP4B1 protein does not cause any bystander effects. Thus, in a preferred embodiment, the cell is to be transplanted.

In a further preferred embodiment, the cell is to be transplanted by donor lymphocyte infusion. The risks of immune responses are particularly high when large amounts of cells are introduced into the human body as e.g. for transplantations of entire organs. Likewise, however, large amounts of allogeneic cells are also introduced into the patient's body during donor lymphocyte infusion following hematopoietic stem cell transplantation. The fresh immunocompetent donor T-cells are transplanted to mediate anti-leukemic graft-versus-leukemia (GvL) reactions. However, they can also induce life threatening graft-versus-host disease (GvHD). By modifying the donor T-cells prior to transplantation by introducing a suicide gene encoding for a modified human CYP4B1 protein, which is able to process 4-IPO, the cells can be deliberately eliminated once GvHD symptoms arise. Thus, the modified human CYP4B1 protein suicide gene system is particularly preferred for donor lymphocyte infusion. Accordingly, in a preferred embodiment, the cell is a human hematopoietic cell, preferably a T-lymphocyte.

In a further aspect, the invention is directed to a cell for use in donor lymphocyte infusion comprising a modified CYP4B1 protein with a serine to proline exchange in the meander region, wherein at least one part of the human CYP4B1 protein is replaced by the corresponding part of a CYP4B1 protein of a different species. For donor lymphocyte infusion, donor lymphocytes/T-cells are transduced with a gene construct encoding the modified human CYP4B1 protein. The resulting cells are used for transplantation into a recipient suffering from e.g. acute or chronic leukemia after allogeneic hematopoietic stem cell transplantation. Circulating in the recipient's blood system, the donor T-cells recognize and eliminate leukemic cells of the recipient (so-called graft-versus-leukemia effect). However, the donor T-cells also recognize the recipient's tissue cells and can cause GvHD, The risk for developing GvHD increases with the amount of T-cells transplanted. However, reducing the amount of donor T-cells also lowers the GvL effect. By providing a modified human CYP4B1 suicide gene system, it is possible to transplant as many donor T-cells as needed to induce a sufficient GvL effect, since the donor T-cells can be deliberately removed from the recipient's blood systems by administration of 4-IPO. Since only the donor T-cells possess an enzymatically active CYP4B1 protein, only these cells will metabolize 4-IPO and subsequently die from the cytotoxic effect of the metabolites. This allows for a specific elimination of the donor T-cells whereas the recipient and all recipient cells remain unaffected.

Accordingly, a further aspect of the invention is directed to a modified human CYP4B1 protein for use in treating transplantation induced graft-versus-host disease (GvHD), wherein at least one part of the human CYP4B1 protein is replaced by the corresponding part of a CYP4B1 protein of a different species.

A further aspect of the invention is directed to 4-ipomeanol (4-IPO) for use in killing a cell, wherein the cell comprises a modified human CYP4B1 protein with at least one part replaced by the corresponding part of a CYP4B1 protein of a different species. The furan analogue 4-ipomeanol (1-[3-furyl]-4-hydroxypentanone) 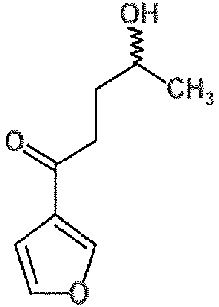 was found to be responsible for the epidemic death of cattle after consumption of moldy sweet potato (*ipomoea batatas*). The 4-IPO was processed by CYP4B1 expressed in lung cells into cytotoxic metabolites (furan epoxide) which caused the cattle's lung cells to undergo apoptosis and induced strong respiratory distress. In contrast to the CYP4B1 proteins of most species, native human CYP4B1 is not able to metabolize 4-IPO; therefore the compound is not toxic to native human cells.

However, in the course of the invention it has now been found that human CYP4B1 can be modified to provide an active human CYP4B1 by replacing at least one part of the human CYP4B1 protein by the corresponding part of the CYP4B1 protein of a different species. Thereby, 4-IPO can be used to specifically eliminate cells comprising the modified human CYP4B1 protein. Besides the natural 4-IPO, also artificially synthesized analogues of 4-IPO (Krauss et al., 2006) can be used according to the invention.

Accordingly, a method is provided for killing a cell in a patient, comprising administrating 4-IPO to the patient, wherein the cell comprises a modified human CYP4B1 protein with at least one part of the human CYP4B1 replaced by the corresponding part of a CYP4B1 protein of a different species.

In a preferred embodiment, the cell is a transplanted cell, a transplanted organ or a part thereof. During allogeneic transplantation, donor cells, which are derived from an organism other than the patient's are introduced into the patient's body. These cells differ from the recipient's own cells and therefore can cause rejection reactions, which can seriously endanger the patient's health or life. Therefore, it can become necessary to remove the transplanted cells from the patient's body. In this case, it is of particularly advantage to equip the donor cells with a suicide gene system prior to transplantation. Such a system is provided by the present invention. It is based on a modified human CYP4B1 protein which is enabled by genetic modification to process 4-IPO thereby producing cytotoxic metabolites which subsequently lead to apoptosis of the transplanted cell. Since only the modified human CYP4B1 protein is able to process 4-IPO, the recipient's cells will not be affected by the administration of 4-IPO. Thus, by use of the suicide gene system based on a modified human CYP4B1 protein a mechanism is provided to efficiently and specifically eliminate donor cells after transplantation from the recipient's body, if necessary.

In a preferred embodiment, the cell is transduced with a nucleic acid, preferably RNA or DNA encoding the modified CYP4B1 protein prior to transplantation.

In a preferred embodiment the cell induced graft-versus-host disease (GvHD).

In a further aspect, the invention is directed to 4-IPO for use in treating GvHD, wherein the GvHD is induced by a transplanted cell comprising a modified human CYP4B1 protein with a serine to proline exchange in the meander region and at least one part of the human CYP4B1 protein replaced by the corresponding part of a CYP4B1 protein of a different species. One of the most important applications for the suicide gene system based on a modified human CYP4B1 protein is to eliminate donor T-cells after donor lymphocyte infusion, in case the recipient develops GvHD. Once the patient shows symptoms of GvHD, 4-IPO is administrated. The compound is taken up by the cells in the recipient's body, however, only the donor T-cells express a modified human CYP4B1 protein, which is able to process 4-IPO. Thus, solely the donor T-cells accumulate the cytotoxic metabolites of 4-IPO and subsequently undergo apoptosis. This allows for a specific elimination of donor T-cells.

Accordingly, a method is provided for treating GvHD, comprising introducing a modified human CYP4B1 protein with a serine to proline exchange in the meander region into a donor T-cell wherein at least one part of the human CYP4B1 protein is replaced by the corresponding part of a CYP4B1 protein of a different species, transplanting the T-cell into a patient in need thereof, and administrating 4-IPO to the patient.

In a further aspect, the invention is directed to a suicide gene system comprising a viral vector expressing a modified human CYP4B1 protein with a serine to proline exchange in the meander region, wherein at least one part of the human CYP4B1 protein is replaced by the corresponding part of CYP4B1 protein of a different species.

In a further aspect, the invention is directed to a kit comprising a viral vector comprising a nucleic acid, preferably DNA or RNA encoding for a modified human CYP4B1 protein with a serine to proline exchange in the meander region, wherein at least one part of the human CYP4B1 protein is replaced by the corresponding part of a CYP4B1 protein of a different species. The kit provides a ready for use tool for equipping cells, e.g. before transplantation, with a suicide gene system based on a modified human CYP4B1 protein. The cells are modified by transducing them with a viral vector comprising a nucleic acid encoding for the modified human CYP4B1 protein. This protein is characterized in that at least one part of it is replaced by the corresponding part of a CYP4B1 protein of a different species, such that it is able to process 4-IPO. In case the cells equipped with the suicide gene system induce unintended reactions after transplantation, they can be specifically eliminated by administrating 4-IPO to the patient.

In a further aspect, the invention is directed to a method of equipping a cell with a suicide gene system comprising providing a nucleic acid molecule encoding for a modified human CYP4B1 protein with a serine to proline exchange in the meander region, and introducing the nucleic acid molecule into the cell, wherein at least one part of the human CYP4B1 protein is replaced by a corresponding part of a CYP4B1 protein of a different species. For modifying a cell with a suicide gene system a nucleic acid molecule is provided, which encodes for a modified human CYP4B1 protein that is able to process 4-IPO into cytotoxic metabolites. Preferably, the nucleic acid is codon optimized for usage in human cells to improve expression of the human modified CYP4B1 protein from the construct. The nucleic acid is then incorporated into the cell, preferably using a viral expression vector, more preferred using a retroviral, a lentiviral or a foamyviral vector. This vector is used to produce viral particles, e.g. by employing a retroviral, lentiviral or foamyviral packaging system, with later are employed to transduce cells. The successfully transduced cell can be killed deliberately and in a controlled fashion by applying 4-IPO, if necessary. The method is particularly preferred to provide cells for transplantation, which can be eliminated from the recipient's body in case of unintended biological reactions of the transplanted cells or of the recipient's immune system towards the transplanted cells.

In a further aspect, the invention is directed to a nucleic acid molecule encoding at least one tumor cell targeting chimeric antigen receptor and a modified human CYP4B1 protein, wherein at least one part of the human CYP4B1 protein is replaced by the corresponding part of a CYP4B1 protein of a different species. To improve the GvL reaction to a tumor cell, the T-cell is modified by introducing artificial T-cell receptor such as CD19 or CD20 targeting chimeric antigen receptors (CARs). These ectopically expressed CARs then direct the T-cells, on which they are expressed, against the lymphoma cells that express specific suitable target structures, e.g. CD19 or CD20 and initiate the destruction of the lymphoma/leukaemia cells. However, as CD19 and CD20 are also present on normal B-lymphocytes, the CARs lead to the destruction of any normal B-cell. In consequence, the patient will need infusions of immunoglobulins for the rest of his/her life. Including a suicide gene in the vector that introduces the CARs in the patient's T-cells would provide the option to eliminate the CAR expressing T-cell years after the successful leukemia treatment by administration of 4-IPO.

In a preferred embodiment, the tumor cell targeting chimeric antigen receptor is a CD19 or CD20 targeting chimeric antigen receptor.

In a preferred embodiment, the nucleic acid molecule is comprised in a plasmid or viral vector.

A further aspect, the invention is directed to a cell expressing at least one tumor cell targeting chimeric antigen receptor, wherein the cell comprises a modified human CYP4B1 protein with at least one part of the human CYP4B1 protein replaced by the corresponding part of a CYP4B1 protein of a different species.

In a preferred embodiment, the cell is a T-cell.

In a further aspect, the invention is directed to a nucleic acid molecule for use in killing tumor cells, wherein the nucleic acid encodes for at least one tumor cell targeting chimeric antigen receptor and a modified human CYP4B1 protein with a least one part of the human CYP4B1 protein replaced by the corresponding part a CYP4B1 protein of a different species. The donor T-cell can be used to target and eliminate tumor cells with the recipient's body. To increase their efficiency, the donor T-cells are modified such that they express a tumor cell targeting chimeric antigen receptor. However, the donor T-cells can also target and eliminate the recipient's healthy cells. In this case, the T-cells must be immediately eliminated to prevent the patient's health condition to further deteriorate. This can be achieved by equipping the T-cells prior to transplantation with a modified human CYP4B1 suicide gene system which is able to process 4-IPO. In case of unintended effects of the donor T-cells, 4-IPO is administrated to the patient and will be taken up and processed by the modified donor T-cells, which will subsequently undergo apoptosis in response to the accumulation of cytotoxic 4-IPO metabolites.

In a preferred embodiment the tumor cells are leukemic cells.

### Examples

### CYP4B1 as a human suicide gene

Biochemical characterization revealed that CYP4B1 proteins from different mammals vary in their amino acid sequence and in their activity of processing 4-IPO. The rabbit CYP4B1 is a very active enzyme in the activation of 4-IPO, while the human isoform has only ≤ 1 % of the activity to activate 4-IPO, despite the high similarity of 84 % on amino acid level between the rabbit and human protein (Czerwinski et al., 1991), as shown in **Figure 1**. It was found that the human CYP4B1 protein has a single amino acid change in the meander region of the enzyme, namely a serine at position 427, while the rabbit and all other tested CYP4B1 enzymes - including gorilla and chimpanzee CYP4B1s - have a proline at the corresponding position.

The function of the native human CYP4B1 protein is not known at all and thus the human CYP4B1 is considered to be an orphan P450 enzyme in humans. Although it was shown previously that the human CYP4B1 enzyme is involved in the reaction of aromatic amines into carcinogenic or mutagenic metabolites in the urinary bladder, it is not yet clear whether the CYP4B1 enzyme in humans can indeed process active metabolites - despite the amino acid serine in the meander region - and therefore might have any functional significance. It is worth mentioning that the proline in the meander region in almost all CYP4B1 proteins from different species, such as in fish, primates, rodents, etc., is highly conserved. In addition, multiple sequence alignments of meander region amino acids of the human xenobiotic-metabolizing CYP1-CYP4 P450 families revealed the proline residue to be conserved in these families, too. The conserved proline residue is part of a Pro-X-Arg/His motif in the meander region. The amino acid serine at position 427 in human CYP4B1 enzyme (Ser-x-Arg) destroys this motif and therefore influences the structural arrangement in relation to the ERR triad (highlighted in bold characters in **Figure 1**). It is assumed that the ERR triad plays an important role in stabilizing and anchoring the heme in the active center and is therefore responsible/essential for maintaining the enzyme activity of any cytochrome P450 enzyme. So far, humans are the only species that have the non-functional Ser residue. Thus, due to the absence of significant toxicities of 4-IPO in humans due to the inactivity of the human protein, an active CYP4B1 isoform might serve as a suicide gene in medical applications.

### Foamy viruses as an alternative apathogenic retroviral gene transfer system

For clinical suicide gene therapy, donor T-cells are equipped *in vitro* with the designated suicide gene, selected for suicide gene-positive cells and then infused into the patient. For this strategy, a gene transfer system is required that facilitates efficient integration into the target cell genome and allows robust and stable expression of both the suicide gene and a second gene for GMP-conform selection procedures.

Retroviral vectors are the gene transfer system of choice for clinical transduction of T-cells. Based on their evolutionary optimization, retroviral vectors efficiently integrate into the cellular genome of the target cells and thus facilitate stable and long-lasting expression of the transgene(s). Vectors based on simple gammaretroviruses such as the Moloney mouse leukemia virus (MoMLV) are still predominantly used for clinical T-cell gene therapy applications. However, as gammaretroviral vectors only infect actively dividing cells - the pre-integration complex cannot permeate the nuclear membrane - and have a short intracellular half-life of 4-6 hours, maximal activation and maintenance of proliferation of the primary T-cells is necessary for these vectors to integrate their transgene(s) in the target T-cells. The ability to integrate into the host genome is associated with the possibility of random non-specific integration into the cellular genome whereby oncogenes can be activated and tumor suppressor genes and regulatory or coding regions can be destroyed. Although these problems have been observed with gammaretroviral vectors in several human stem cell gene therapy trials, it is currently believed that mature T-cells are not susceptible of the transforming ability of gammaretroviruses.

Foamy viruses (FVes) are the largest retroviruses known today and are widespread among different mammals, including all non-human primate species, however are absent in humans. This complete apathogenicity qualifies FV vectors as ideal gene transfer vehicles for genetic therapies in humans and clearly distinguishes FV vectors as gene delivery system from HIV1-derived and also gammaretrovirus-derived vectors.

FV vectors are suitable for gene therapy applications because they can (1) accommodate large transgenes (> 9kb), (2) transduce slowly dividing cells efficiently, and (3) integrate as a provirus into the genome of target cells, thus enabling stable long term expression of the transgene(s). FV vectors do also need cell division for the pre-integration complex to enter the nucleus, however the complex is stable for at least 30 days and still infective. The intracellular half-life of the FV pre-integration complex is comparable to the one of lentiviruses and significantly higher than for gammaretroviruses, therefore FV are also - similar to LV vectors - able to transduce rarely dividing cells. FV vectors are natural self-inactivating vectors and characterized by the fact that they seem to have hardly any potential to activate neighboring genes. In addition, FV vectors can enter any cells known (although the receptor is not identified yet) and infectious vector particles can be concentrated 100-fold without loss of infectivity due to a stable envelope protein. FV vectors achieve high transduction efficiency in pluripotent hematopoietic stem cells and have been used in animal models to correct monogenetic diseases such as leukocyte adhesion deficiency (LAD) in dogs and Fanconi anemia in mice. FV vectors are also used in preclinical studies of β-thalassemia.

### Optimization of human suicide gene, CYP4B1

All experiments were done based on the human CYP4B1 protein of SEQ ID NO.: 1:

MVPSFLSLSFSSLGLWASGLILVLGFLKLIHLLLRRQTLAKAMDKFPGPPTHWLFGHAL
EIQETGSLDKVVSWAHQFPYAHPLWFGQFIGFLNIYEPDYAKAVYSRGDPKAPDVYDF
FLQWIGRGLLVLEGPKWLQHRKLLTPGFHYDVLKPYVAVFTESTRIMLDKWEEKAREG
KSFDIFCDVGHMALNTLMKCTFGRGDTGLGHRDSSYYLAVSDLTLLMQQRLVSFQYH
NDFIYWLTPHGRRFLRACQVAHDHTDQVIRERKAALQDEKVRKKIQNRRHLDFLDILLG
ARDEDDIKLSDADLRAEVDTFMFEGHDTTTSGISWFLYCMALYPEHQHRCREEVREIL
GDQDFFQWDDLGKMTYLTMCIKESFRLYPPVPQVYRQLSKPVTFVDGRSLPAGSLIS
MHIYALHRNSAVWPDPEVFDSLRFSTENASKRHPFAFMPFSAGPRNCIGQQFAMSEM
KVVTAMCLLRFEFSLDPSRLPIKMPQLVLRSKNGFHLHLKPLGPGSGK

However, analyses of a variant of hCYP4B1 having an insertion of a serine at position 207 did not reveal any functional differences.

Initially, the serine at position 427 in the human CYP4B1 was mutated back to profile (h-P427), which is present at this position in all CYP4B1 proteins. Additionally a proline to serine exchange was created in the native rabbit CYP4B1 enzyme at the corresponding position 422 (r-P422). To immediately exclude the possibility that the different activities between human and the rabbit protein isoforms are simply due to differences in mRNA stability, the human CYP4B1 cDNA (SEQ ID NO.: 2) was optimized for human-codon usage (SEQ ID NO.: 3) by *de novo* synthesis with silent mutagenesis of the cDNA, thus removing internal TATA-boxes, instability motives, RNA secondary structures and (cryptic) splice donor / acceptor sites and ensuring an average GC content of around 60%. All cDNAs were cloned into the same lentiviral (LV) HIV1-based expression vector and LV supernatants produced by plasmid transfection in HEK293T cells. Subsequently, the two rabbit and the four human cDNAs were stably expressed in the human liver cell line HepG2. Stably expressing transduced cells were challenged after five days with increasing concentrations of 4-IPO for 24, 48 and 72 hours and then the survival of cells analyzed by flow cytometry using propidium iodine for live/dead cell discrimination.

The results demonstrated (**Figure 2**) that the native human CYP4B1 S427 protein (SEQ ID NO.: 1) cannot process 4-IPO at all and that human codon-optimization (coop) did not lead to any major improvement in the activity of the enzyme. The point mutation S427P in the human protein, however, rendered this enzyme active for processing 4-IPO. In addition, the codon-optimization slightly increased the activity of the mutated enzyme in cells. The native rabbit CYP4B1 protein (with P422, SEQ ID NO.: 11), however, was still superior to the both human mutant proteins. Strikingly, the serine 422 mutation in the rabbit CYP4B1 caused a decrease in sensitivity to 4-IPO compared to the native protein, however, the enzyme was not inactive at all (**Figure 2**). After 24 hours of 4-IPO treatment the activity of the rabbit S422 mutant still eliminated 73% of transduced HepG2 cells whereas over 95% of the HepG2 cells expressing the wildtype rabbit P422 protein were dead. Strikingly, the mutated rabbit S422 enzyme still killed the HepG2 cells more effectively than the human P427 mutant. These results showed that the serine-to-proline exchange is not the only reason for the rabbit wildtype protein's strong activity to metabolize 4-IPO and the human CYP4B1's inactivity.

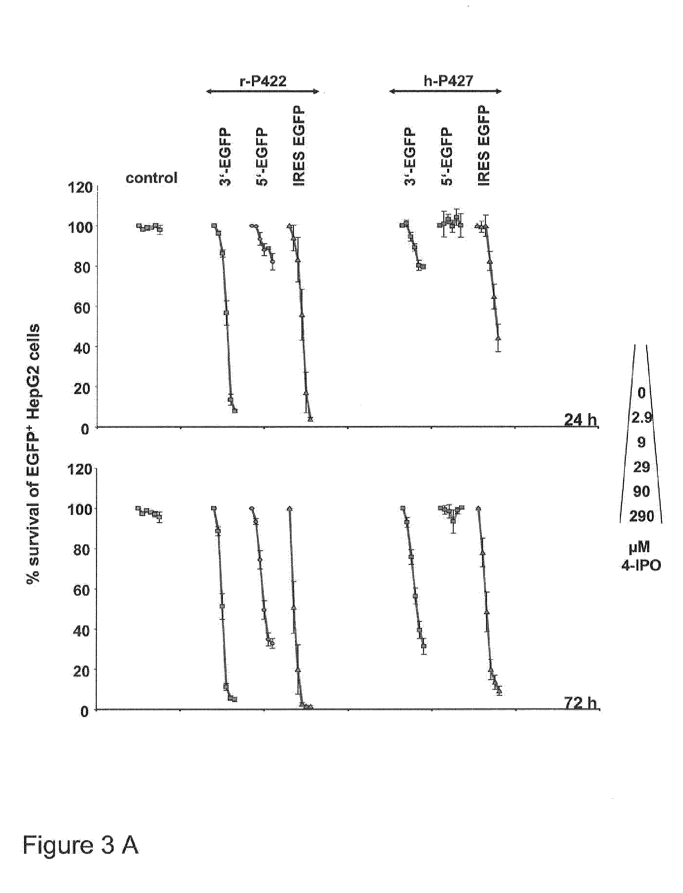

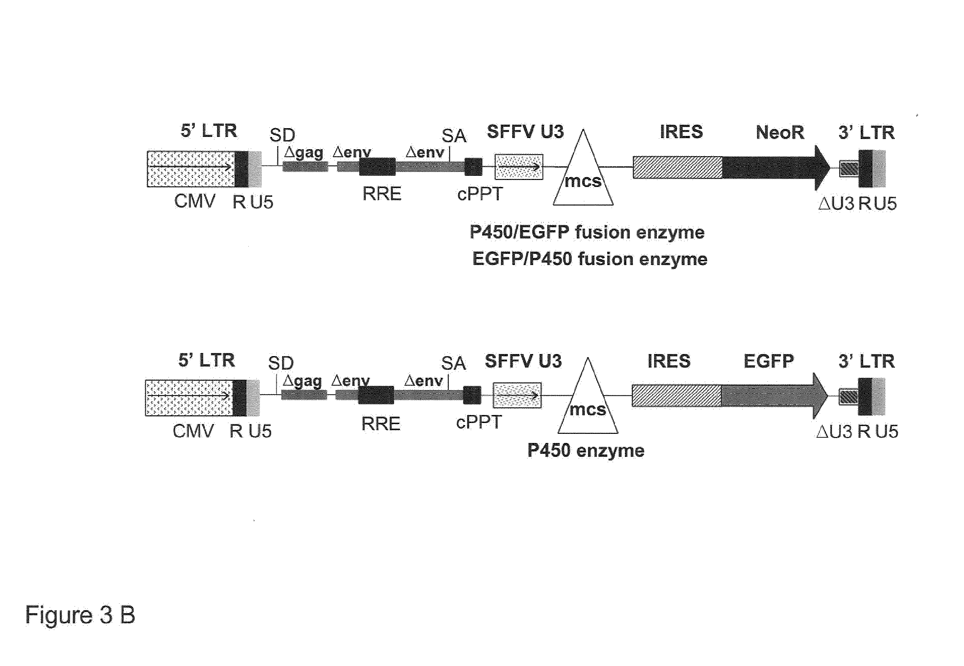

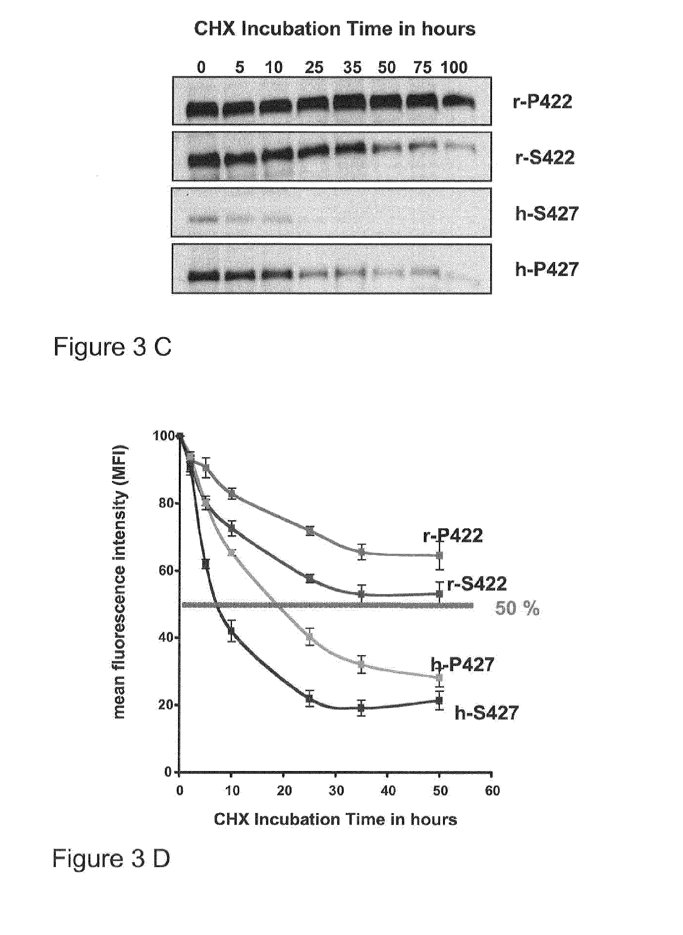

Next, it was analyzed whether the different activities of the human and the rabbit proteins in processing 4-IPO could be explained by differences in protein half-life. However, as no antibody was available that can detect both CYP4B1 proteins, EGFP fusion constructs were generated. With these constructs, EGFP antibodies can be utilized to visualize and compare the expression levels and the half-lives of the fusion proteins in cells by western blots. EGFP expression can also be used for flow cytometry assessment of the expression levels due to the fluorescent properties of the fusion proteins. 5'- and 3'-fusions of EGFP with the native rabbit P422 and the human P427 CYP4B1 proteins were cloned in LV vectors with an IRES-NEO (NEO =neomycin phosphotransferase cDNA, *npt II*) and stably expressed in HepG2 cells (**Figure 3** **B**).

For comparison, the corresponding IRES-EGFP constructs were also included, as these vectors had already been used in previous experiments and activity measurements thus already existed. Transduced cells were incubated for 24, 48 and 72 hours with increasing concentration of 4-IPO. Cultures were then harvested, cells stained with propidium iodine and subsequently analyzed for survival of transduced EGFP+ cells by flow cytometry (**Figure 3** **A**).

The analysis of FACS survival data (**Figure 3** **A**) demonstrated that the 5'-EGFP fusion strongly inhibits the activity of CYP4B1 proteins. The 3'-EGFP fusion also diminished the activity of the enzymes to metabolize 4-IPO compared to the IRES-EGFP constructs, however, this inhibition occurred to a significantly lower degree and the CYP4B1 proteins of both species were affected to a similar degree. After standard inhibition of *de novo* protein synthesis by cycloheximide, the half-life of the CYP4B1 proteins in stably transduced and G418-resistant HepG2 cells was assessed by determining the protein expression levels over time either in western blotting or in FACS analysis as EGFP fluorescence. The results of the western blots (**Figure 3** **C**) basically revealed the same results as the FACS analysis (**Figure 3** **D**): The wildtype rabbit CYP4B1 enzyme (r-P422) has the strongest protein stability and introduction of the proline-to-serine exchange decreases the half-life of the mutated rabbit enzyme (r-S422) to approx. 30 hours. For the human native protein (h-S427), the mean fluorescence indicates a short average half-life of only 6 hours. Here, the serine-to-proline exchange (h-P427) produced an improvement in protein half-life of the human mutated enzyme to 20 hours, which however is still inferior to the half-life of the r-S422 CYP4B1 protein.

These results clearly demonstrated that the differences in enzyme activity and protein half-life between the human and the rabbit CYP4B1 enzymes cannot solely be attributed to the proline-to-serine alteration in the meander region. Although the rabbit and the human CYP4B1 protein have a high similarity of 84%, 80 amino acids are still divergent. In order to identify smaller segments between the two proteins that influence the activity against 4-IPO, two unique restriction enzyme recognition sites, BstX I and Blp I, which exist in both cDNAs and are in frame (**Figure 4**), were used to exchange whole regions between the two proteins. To this end, the human and the rabbit *CYP4B1* cDNAs were divided in three areas/domains (5'-region, middle region and 3'-region), which were exchanged by cloning and the resulting hybrid proteins characterized functionally. The respective constructs were:

| Name | 5'-region | Middle region | 3'-region | amino acid 427(h)/422(r) | SEQ ID NO.: (protein) | SEQ ID NO.: (cDNA) |
|---|---|---|---|---|---|---|
| h-r-r P422 | human | rabbit | rabbit | proline | 71 | 72 |
| r-h-h P427 | rabbit | human | human | proline | 65 | 66 |
| r-h-h- S427 | rabbit | human | human | serine | | |
| r-h-r P422 | rabbit | human | rabbit | proline | 61 | 62 |
| h-r-h P427 | human | rabbit | human | proline | 67 | 68 |
| h-r-h S427 | human | rabbit | human | serine | | |
| h-h-r P422 | human | human | rabbit | proline | 69 | 70 |
| r-r-h P427 | rabbit | rabbit | human | proline | 63 | 64 |
| r-r-h S427 | rabbit | rabbit | human | serine | | |

The region exchanges between the human and rabbit enzymes revealed that each rabbit region (5'-, middle and 3'- region) causes an increase in the enzyme activity of the human P427 und S427 CYP4B1 proteins and that each human region decreased the activity of processing 4-IPO of the native rabbit P422 isoform (**Figure 6** **A**). The exchanges of the C-terminal end or the N-terminal leader region of the proteins between the two species demonstrated the lowest changes in CYP4B1 enzyme activity, while the largest differences in toxicity with 4-IPO were observed by exchanging the middle region. Therefore, the middle region of the human protein was further modified to achieve higher enzyme activity. To this end, the middle region of the rabbit CYP4B1 enzyme was divided into six small sections (**Figure 4**) and each section was cloned individually into the cDNAs for the human native S427 and P427 mutant CYP4B1 proteins (SEQ ID NOs.: 74, 76, 78, 80, 88, 90, 92 (cDNA); SEQ ID NOs.: 73, 75, 77, 79, 87, 89, 91 (protein)). The first three sections of rabbit protein produced an improvement in enzyme activity after 24, 48, and 72 h, if the human P427 mutant CYP4B1 protein was used (**Figure 6** **B**). In the human native S427 CYP4B1 protein, none of the six sections from the rabbit protein restored any activity of the human protein for 4-IPO (**Figure 6** **B**). In addition, individually changing any of the 18 amino acids differing between the rabbit and the human proteins in sections 1 to 3 in the human P427 CYP4B1 did not result in any increase in enzyme activity (**Figure 6** **C**). Therefore, four possible combinations of sections 1 to 3 of the rabbit protein were cloned into the human P427 isoform: 1 + 2 (SEQ ID NO.: 79, 80), 2 + 3 (SEQ ID NO.: 81, 82), 1 + 3 (SEQ ID NO.: 83, 84), 1+2+3 (SEQ ID NO.: 85, 83) and the resulting proteins were compared with respect to their 4-IPO processing activity (**Figure 6** **D**). Surprisingly, this approach revealed that the enzyme activity of the human P427 CYP4B1 protein with sections 1+2+3 of the middle region of the native rabbit protein for 4-IPO was almost as high as the activity of native rabbit protein. Actually, this mutant protein was even more active than the human P427 protein with the complete middle rabbit region (h-r-h P427) with all sections 1-6 (**Figure 6** **D**). Thus, the human P427 CYP4B1 protein containing sections 1+2+3 of the rabbit CYP4B1 protein (SEQ ID NO.: 85) is an excellent compromise for a construct with high activity of processing 4-IPO and with minimal amino acid changes compared to the native inactive CYP4B1 protein. Ultimately, this construct differs in only 19 amino acids from the native human CYP4B1 protein and therefore still has an identity of 96% to the human CYP4B1 protein (**Figure 5**).

Thus, in particular the modified human CYP4B1 P427 protein with the additional section 1 of the rabbit protein (SEQ ID NO.: 73), which differs in only four amino acid from the native human CYP4B1 protein, and the modified human CYP4B1 P427 protein with sections 1+2+3 of the rabbit protein (SEQ ID NO.: 85), which differs in only 19 amino acid from the native human CYP4B1 protein, had both increased activity for toxifying 4-IPO and led to efficient elimination of transduced cells.

### Effectiveness of CYP4B1 suicide gene in human primary T-lymphocytes

The success of suicide gene therapy as treatment option depends on the activity of the suicide gene in combination with characteristics of the pro-drug and is subject to certain conditions: (1) All donor T-cells that are infused into the patient must be transduced with the suicide gene, (2) the suicide gene expression should be effective, non-toxic and stable *in vivo* and not subject to immunological clearance of the transduced T-cells, (3) the pro-drug should not have any toxicities on the non-transduced cells of the donor, and (4) a rapid and selective elimination of the suicide gene-positive cells after administration of the pro-drug without major bystander effects for the surrounding tissues/cells must be guaranteed.

For the use of the optimized CYP4B1/4-IPO system for donor lymphocyte infusions after allogeneic transplantation, the mature T-lymphocytes from the healthy stem cell donor are the primary target cells. In contrast to the HSV-tk/ganciclovir system, the CYP4B1/4-IPO suicide gene system showed no bystander effect, which is very desirable for donor lymphocyte infusion applications, as genetically modified organ-infiltrating T-cells cells are specifically killed. For the donor lymphocyte infusion therapy, it is also advantageous that the CYP4B1 suicide gene system acts independently of cell proliferation, especially for treatment of chronic GvHD, as here the autoimmune T-lymphocytes have a relatively low proliferative activity. In contrast, the HSV-tk/ganciclovir-system is only effective against actively dividing cells, because ganciclovir inhibits DNA synthesis, whereas the rabbit CYP4B1/4-IPO system mediates a proliferation-independent elimination of cells. In addition, as evident from the above described experiments, serum concentrations up to 90 µM by 5-day infusion regimens, which have already been tested in phase I/II clinical trials for patients with lung and liver cancers (Rowinsky et al., 1993 and Kasturi et al., 1998), are highly efficient for killing human P427 mutant CYP4B1 transgenic human cells within 72 h.

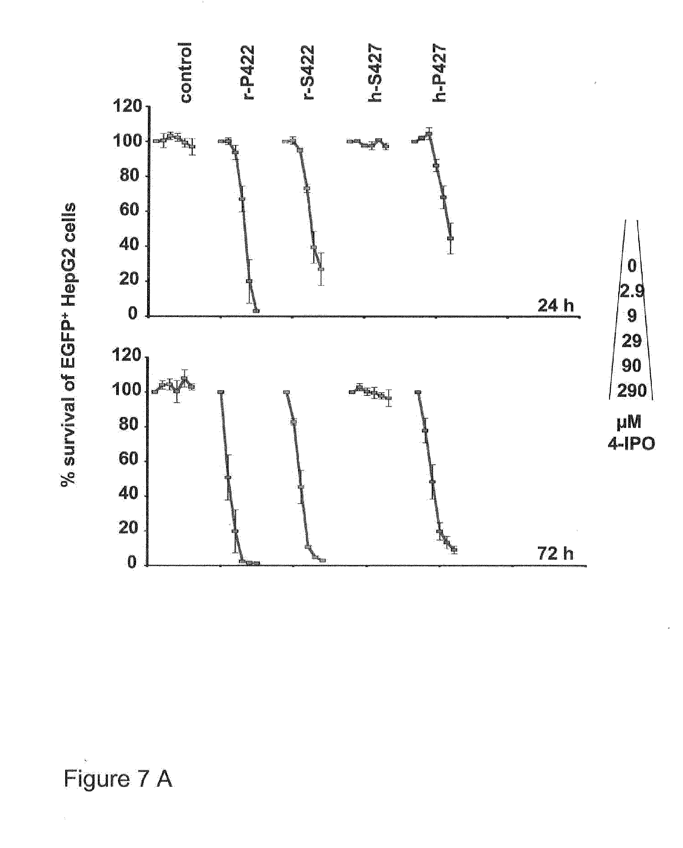

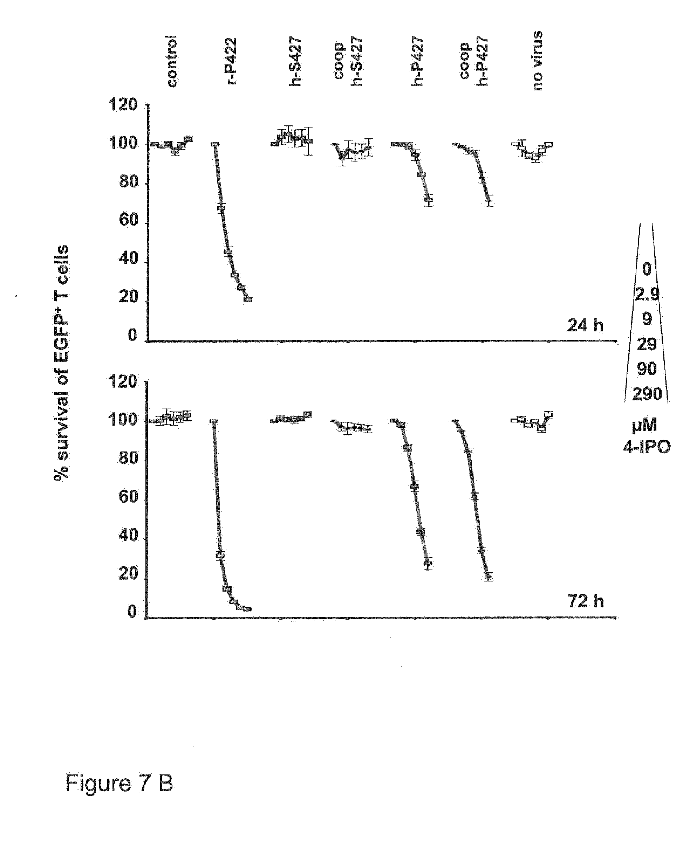

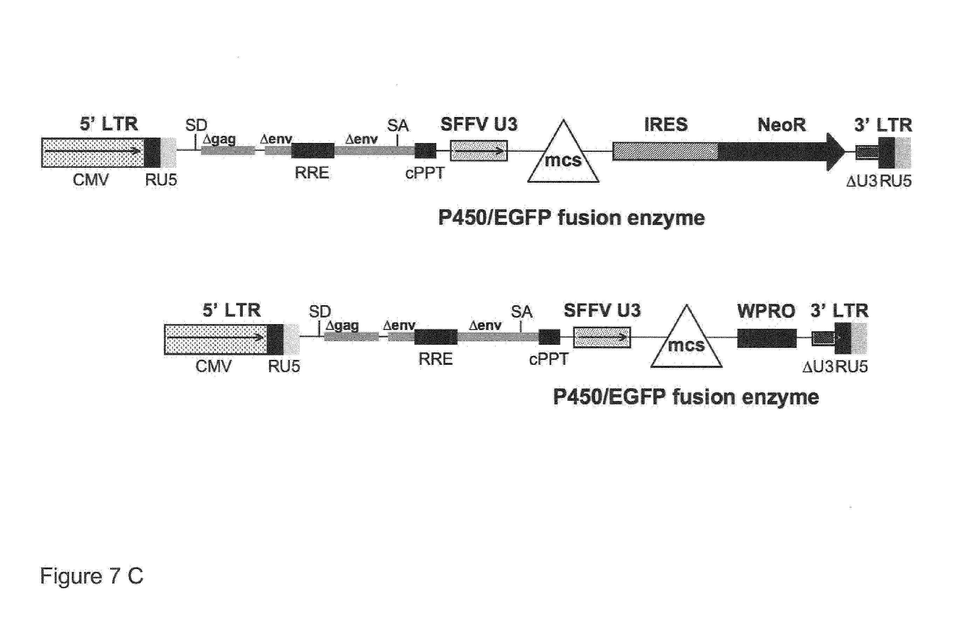

Compared to the HepG2 liver cells, primary T-cells might not provide an optimal environment for the function of P450 cytochromes (although the necessary reductase is ubiquitously expressed) and the infection of primary T-cells is considerably more complex. Therefore, in order to demonstrate that primary human T-cells can be eliminated with the modified human CYP4B1 suicide gene system, primary T-cells from healthy volunteers were stimulated by incubation on immobilized CD3 and CD28 antibodies for 3d and then transduced once on the recombinant fibronectin fragment CH-296 in the presence of IL-2 with the vectors shown in **Figure 7** **C**.

The survival curves for the HepG2 cells were generated with standard lentiviral vectors and showed the killing efficiency for the mutant P427 CYP4B1 enzyme fused to EGFP, (**Figure 7** **A**). For the T-cell experiments, the mutant P427 CYP4B1 (also after codon-optimization) fused to EGFP were expressed from an improved vector, where the IRES-NEO cassette was removed and an optimized shortened and safety-modified version of the Woodchuck hepatitis virus post-transcriptional regulatory element (WPRE => WPRO included. Overall, the transduction efficiency of primary human T-cells of 43-76% was, despite a good reference HT1080-titer (2-5 x 10⁶ TU/ml), significantly lower than in HepG2 cells and therefore the protein expression levels of the fusion proteins were clearly lower (MFI). Nevertheless, the initial results demonstrated that primary T-cells can be killed within three days with the combination of human CYP4B1 P427 enzyme and 24 h and 72 h exposure to increasing doses of 4-IPO (**Figure 7** **B**). Also here, the native rabbit enzyme is more active than the native P427 and the human codon-optimized P427 CYP4B1 enzymes (**Figure 7** **B**).

### Transduction of primary T-cells via foamy- and lentiviral vectors

The apathogenicity of foamy viruses in their natural animal hosts and in accidentally humans, their unique genomic integration pattern where two thirds of the proviral integrations occur outside of open reading frames, and their long half-life of more than 30 days in non-dividing cells makes foamyviral (FV) vectors an integrating gene transfer system next to none. FV vectors show a great flexibility in accepting foreign sequences and have a natural self-inactivating design after excluding the transactivator FV *tat* from the packaging system.

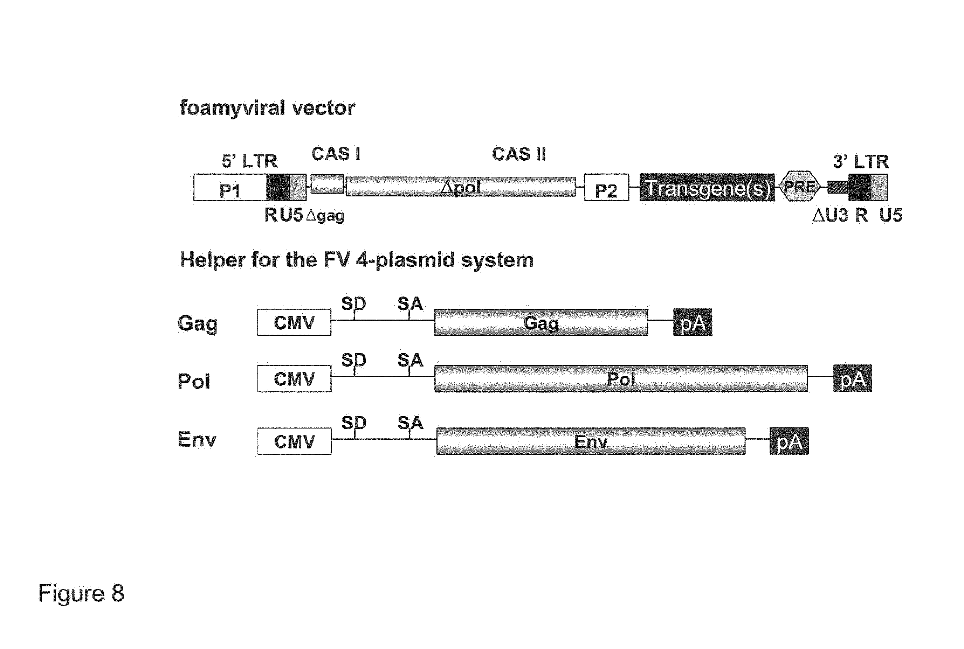

An optimal FV production system was developed, in which all three essential FV genes, *gag, pol,* and *env,* for generating infectious replication-incompetent recombinant vectors have been spread to three different expression plasmids with the heterologous CMV-IE promoters and also heterologous polyA sites (bGH pA) and also optimized for human codon usage. These codon-optimizations abrogate any natural retroviral gene regulation/splicing by modifying/enhancing/destroying splice active sites and by introducing artificial stop codons and mutating naturally used translational start sites (ATGs). In addition, the FV vector was modified according to the scheme shown in **Figure 8**, replacing the complete U3 region of the 5' LTR with the CMV-IE promoter (P1), deleting the *gag, pol, env, tat* and *bet* ORFs, and additional deletions of the FV enhancer and promoter in the U3 region of the 3' LTR. An internal heterologous promoter (P2) allows including non-viral promoter elements for expression of the transgenes and an optimized WPRE further improves transgene expression and further prevents read-through of the transgenes into the surrounding genome. With all these modifications, recombination events that would restore replication-competent infectious wildtype viruses can and will never occur (**Figure 8**).

In experiments with FV vectors, four internal constitutively active promoters were cloned into the standard FV vector puc2MD in the position of P2 and EGFP as a marker gene was included to assess gene transfer efficiencies. These were the viral SFFV (spleen focus-forming virus) U3 region and the three human promoters EF1α (elongation factor 1 alpha), human PGK (phosphoglycerate kinase) and human ubiquitin C. Similar constructs were also generated with the standard lentiviral vector puc2CL. The 5'-LTR hybrid promoter (P1) in both plasmids is the U3 region of the cytomegalovirus (CMV). FV and LV vectors were produced transiently by transfection of the vector and the three packaging plasmids into HEK293T cells according to a standard protocol. Then the viral supernatants were utilized to transduce primary T-cells on the recombinant FN fragment CH-296. The efficiency of the different promoter combinations was analyzed in primary T-cells by flow cytometry. Infectious titers were assessed on HT1080 cells.

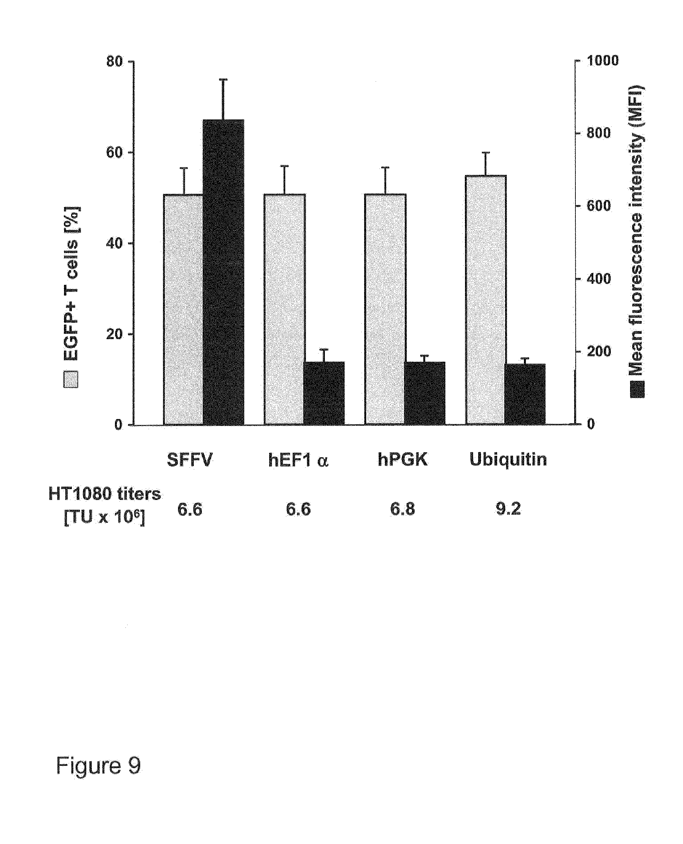

In FV vector with the human ubiquitin promoter achieved 55% transduction of the T-cells, however this vector also had the best virus titer on HT1080 cells. The other three internal promoters result in transduction efficiencies of approximately 50% of primary human T-cells with one round of vector exposure and with EGFP as transgene (**Figure 9**). With regard to transgene expression levels, the SFFV U3 promoter achieved by far in the most intense EGFP expression in primary T-cells, as the mean fluorescence intensity (MFI) values are approximately half a log higher than all three human promoters.

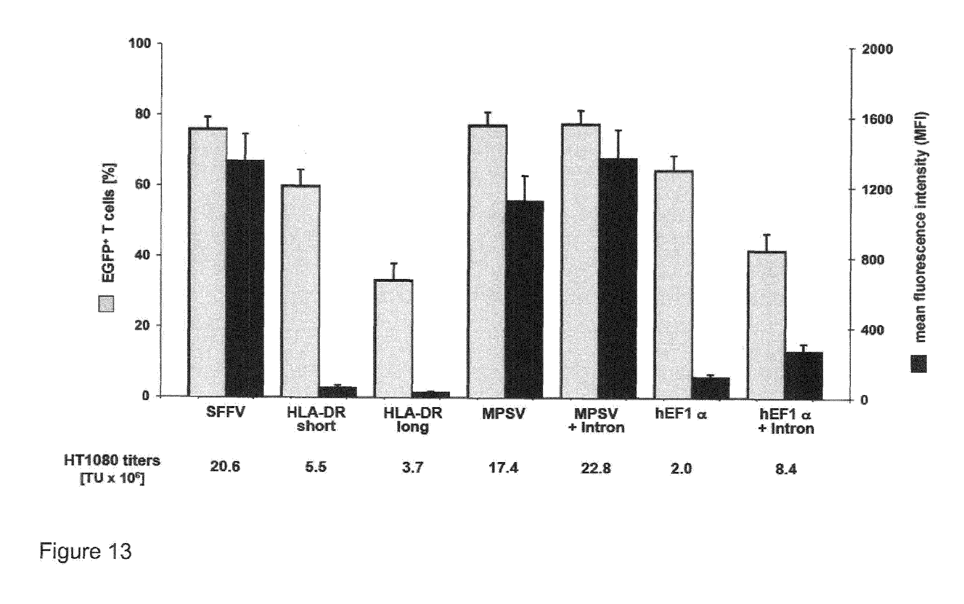

In lentiviral vector the MPSV and SFFV viral promoters achieved the highest viral titers and the best T-cell transduction efficiencies of around 80 % and the highest MFI values. The primary T cells were stimulated by CD3/CD28 antibodies, that means at the time of transduction activated T cells were present. Despite, the two activation-dependent HLA-DR promoter achieved the weakest EGFP expression. The shorter HLA-DR promoter reached a T-cell transduction efficiency of about 60 % and the longer promoter sequence, an efficiency of only 33 %. The other human internal promoter EF1α result in transduction efficiencies of 40 % - 60 % of primary human T-cells. In comparison to the viral promoters, the MFI values of the human promoters in primary T-cells are very low (**Figure 13**).

In some cells, post-transcriptional regulatory elements such as the woodchuck hepatitis virus (WPRE) possesses a cell cycle-and promoter-independent expression-enhancing effect and thus further improves the expression levels of the gene(s)-of-interest in the context of vector optimization. The commonly used WPRE of the Woodchuck hepatitis virus includes the open reading frame of the X-protein, which is responsible for efficient expression of viral transcripts and have been characterized as oncogene (Kingsman et al., 2005). Therefore, an optimized version of the WPRE (= WPRO) was cloned into FV vectors that differed only in their internal, constitutively active promoters (SFFV U3, human EF1α and human PGK). For these vectors, the associated transgene expression levels were examined. WPRO differs to WPRE by deletion of the 3'-located X-protein promoter. In lentivirus self-inactivating (LV SIN) vectors both post-transcriptional regulatory elements resulted in an effective increase of virus titer and transgene expression. FV vectors were produced as described above and used to transduce HT1080 cells in limiting dilutions.

For three tested promoters in both LV and FV vectors - SFFv, hPGK and hEF1alspha, EGFP expression was increased by both WPRE and WPRO at similar levels (**Figures 10** **and** **14**). The corresponding FV titers showed no significant differences between the FV vectors. Thus, the WPRO was included as a standard element in all FV vectors. The expressions enhancing effect has been attributed to the RNA export/stability and polyadenylation. The WPRE in combination with the SFFV U3 promoter has been shown to be an advantageous vector design for the transduction of T-cells.

### Internal promoter for transgene expression in FV and LV SIN vectors

The second internal promoter P2 is required to drive the gene(s) of interest after proviral integration, as the SIN deletion in the 3'LTR U3 region renders these vectors transcriptionally inactive. The flexibility of SIN FV and LV vectors to accept foreign DNA has led to the inclusion of a wide range of internal promoters and thereby allows optimizing and fine-tuning of transgene expression and protein level, respectively. As expression of the human CYP4B1 suicide gene did not cause any toxicity in the target cell, constitutively active promoters can be used in FV and LV vectors in position P2, such as human (PGK, EF1 alpha, Ubiquitin C) or even viral promoters (U3 regions of the SFFV and MPSV - myeloproliferative sarcoma virus).

However, activation-dependent promoters where the transcriptional activity is depending on the activation of the T-cells appear to be ideal for certain applications as e.g. the donor lymphocyte infusion application. Well known examples of these types of promoters in T-cells are (1) the HLA-DR promoter for HLA class II expression, which is constitutively active in B-cells and activation-induced in T-cells, (2) the CD69 promoter which drives this activation-induced lymphocyte adhesion molecule or the CD25/(IL2-receptor α-chain promoter necessary for expression of one of the activation induced IL2 receptor chain on T-cells. The use of such T-cell activation-dependent promoters would be very interesting for clinical usage, since the suicide gene would then only be expressed when the transduced donor T-cells are activated, as in the case of acute or chronic GvHD. This strategy would reduce the likelihood of any immunological effects due to the continuous high level expression of the suicide gene.

A high promoter activity increases the transactivation potential at the proviral integration sites and the transactivation potential is dependent on the chromatin environment of the target cell. For successful gene therapy, a promoter with both, low transactivation capacity and simultaneously robust transgene expression is preferred.

In a first set of constructs, at least 5 to 7 promoters were cloned in a standard FV vector and the promoter activities tested in primary T-cells using EGFP as marker gene. EGFP as transgene allows readily detecting the expression level and intensity by FACS analysis. For this comparison, it is mandatory to achieve comparable numbers of proviral integration per target cell genome. Therefore, the primary T-cells are transduced with equal numbers of infectious viral particles (for each variant separately) pseudotyped with the same envelopes. For constitutive promoters, the viral titers are assessed on HT1080 cells as a very sensitive reference cell line to calculate the number of infectious FV particles per ml (TU/ml). Primary T-cells are then transduced on CH-296 after pre-stimulation on CD3/CD28 with various dilutions of FV particles and then the promoter activity compared based on the MFI values of the EGFP+ T-cells at low gene transfer efficiencies where most likely only a single integration had occurred. For T-cell activation-induced promoters, HT1080 cells cannot be used for tittering as the promoters are not active in these cells. Here, the amount of viral particles is semi-standardize by western blots for FV gag (where a characteristic double band occurs) on the concentrated FV supernatant (particle preparation) or the human Jurkat T-cell ALL line is use as indicator cells. Jurkat T-cells can be activated by CD3 antibodies and show upregulation of activation induced antigens including the CD25alpha-chain of the IL2 receptor. Alternatively, the activation induced promoters are tested in a two promoter vector where the constitutive promoter is used for standardization of the proviral copy numbers. After transduction und FACS analysis of the different promoter variants, the two best internal promoters are each cloned into a FV vector harboring the CYP4B1/EGFP fusion expression cassette and then expression levels und activity of metabolizing 4-IPO correlated.

### Validation of human selection markers to purify transgenic T-cells for clinical use

Efficient donor lymphocyte infusion treatment requires transducing large numbers of primary T-cells with FV vectors. In order to control the cells *in vivo* in patients, all infused T-cells need to express the suicide gene. Practically, it is not possible to genetically modify all T-cells after *ex vivo* transduction with FV vectors. Therefore, enrichment/selection strategies are needed that facilitate efficient and fast selection of large numbers of transduced T-cells. A clinically applicable selection marker should ideally meet the following criteria:
- Human origin to prevent an immune response.
- Specific expression only in the genetically modified cells.
- Should not disturb the physiological functions of the target cells.
- GMP compatible selection conditions.
- Fast selection process for an *ex vivo* T-cell cultivation as minimal as possible.

Given this requirement profile, the marker protein EGFP and neomycin phosphotransferase and similar transgenes are not preferred for the use in humans because of their immunogenicity. Alternatively to selecting cells on the basis of resistance mechanisms, cells can be purified using magnetic columns according to their cell surface markers, for instance truncated CD34, low-affinity truncated nerve growth factor receptor (NGFR), human epidermal growth factor receptor (huEGFRt), and truncated CD19.

First, all the codon-optimized cDNAs of the above mentioned selection markers are cloned in a FV vector co-expressing EGFP. By co-expression of the selection marker and EGFP, the purity of the separated cell population and selection efficiency of the MACS system via FACS analysis is readily possible. In a first round of experiments, marker and GFP are tested under the control of a weak human promoter (e.g. EF1 alpha or PGK) in order to assess the effectiveness of selection, even at low level of gene expression. The assessment of the selection marker is carried out by the following criteria: Degree of purity after MACS purification, virus titer (TU / ml), transduction efficiency, transgene expression in T-cells and possible toxicity due to the transgene expression. If the expression of the selection marker is not efficient, stronger promoters are included.

### Linking of two cDNAs in order to express the suicide gene and selection marker cDNA in the same FV vector

In order to select the transduced T-cells with the suicide gene, two cDNAs are required in the FV vector: the human suicide gene and the human selection marker. Expression of two genes can be achieved by linking the two cDNAs either as fusion genes, by using an IRES site or a 2A site (of picornavirus or of the Foot and Mouth Disease Virus) (**Figure 14**).

Polyproteins that self-process co-translationally into separate components have been generated using self-cleaving 2A-like peptides of the Foot and Mouth Disease Virus (FMDV), from picornaviruses or from thosea asigna virus (T2A). The 2A like peptides consist of an approximately 19 amino acid long conserved motif (D(V/I)EXNPGP), which has been tested successfully in many eukaryotic systems both *in vivo* and *in vitro.* In a direct comparison with the IRES element, the 2A site showed a 4 times higher transgene expression. The use of 2A sites is limited because it adds a stretch of 19 amino acids of FMDV origin to the first protein and therefore may interfere the activity, stability and immunogenicity of the protein. The most common approach in multiple gene transfer systems, however, has relied on using internal ribosome entry sites (IRESs). The IRES element allows the expression of several genes from the same transcript. To express two transgenes in a single gene transfer vector and to overcome the limitations of the approaches described above, a 2-promoter-system and a bidirectional promoter are tested. The advantage of bidirectional promoters are that no connecting element for the two transgenes (suicide gene and marker) is required and one single promoter still ensures the expression. There are naturally occurring bidirectional promoters such as the DAXX, TIGD1, Rab30, Top3b, HNRNPH2/GLA, UCOS, or UBA52 promoters or fusions of two minimal promoters with a bidirectional enhancer element such as the human PGK and the minimal CMV promoter (mCMV/hPGK).

Initial testing of the vector design comprises with EGFP and E2 CRIMSON. For deciding on the optimal concept, it is important to investigate the transgene expression levels of both genes (MFI value by FACS) at both positions and with both promoters. For clinical application, a strong expression of the suicide gene is needed. The selection marker can also be expressed more weakly leading to a lower amount of selected T-cells, but at least all cells highly express the modified human CYP4B1 suicide gene. For the donor lymphocyte infusion application, it is important that all T-cells are equipped with a suicide gene and express this sufficiently so that in the case of GvHD all cells can be eliminated and therefore safety more cells can be applied. Therefore, the human codon-optimized cDNAs (suicide gene and selection marker) are cloned into the respective vectors and are tested for their efficiency in the 4-IPO toxicity assays.

### Improving and stabilizing transgene expression

Silencing of transgene expression by epigenetic modification (DNA methylation, histone modification) of vector sequences is a major concern in gene therapy. In the context of gene therapy studies, epigenetic silencing of transgene expression occurs with consequent reduction of therapeutic gene expression or even the full repression.

Genetic elements that enable the integrated plasmid to generate a transcriptionally active gene locus independently of the integration site reduce the effect of heterochromatin and allow a stable and efficient expression of the transgene in a euchromatic environment. For these purpose protective cis-regulatory elements such as insulator sequences, scaffold/matrix attachment regions and ubiquitous chromatin opening elements are commonly used. They operate by shielding the integrated genes against position effects. These elements comprise the Ubiquitous Chromatin Opening Element (UCOE), Insulator sequences and S/MAR. All the expression-enhancing elements are tested in a GFP expressing FV vector. The marker GFP is expressed via a strong viral (SFFV U3) and a weaker human (PGK) promoter avoiding that the promoter strength distorts the result. In addition, in case of excessive expression the sensitivity of the FACS machine is limited, whereby low differences can not be separated from each other. The effect of the elements is analyzed especially in primary T-cells. The readout is carried primarily by the strength of the transgene expression (GFP), which is determined by the MFI value as assessed by FACS. Another important aspect is the number of produced virus particles, because an additional vector element enlarges the transgene cassette and the vector size, thus potentially leading to a decreasing virus titer. Determining the number of virus (TU/ml) is carried out by titration of the virus supernatant to the HT1080 reference cell line. The elements that have enabled an improved GFP expression are cloned in the FV suicide gene vector (expression of CYP4B1) and analyzed in the toxicity assay with addition of 4-IPO.

### Stimulation of primary T-lymphocytes

Beside the vector optimization, also the transduction efficiency in primary T-lymphocyte is essential for an optimal transgene expression. However, the transduction efficiency may depend on the proliferation status of the T-cell. The *in vitro* cultivation/stimulation of T-cells should be kept as short as possible and without affecting their phenotype, since a long *ex vivo* cultivation and strong stimulation can have a negative impact on the functionality of the cells:
- Reduction of *in vivo* alloreactivity against leukemic blasts.
- Changes in migration and homing behavior of T-cells and thus associated to a change in the *in vivo* function.
- Reduction of immune competence which is essential for the GVL effect.
- Changes in the TCR Vβ repertoire.
- Shift of the CD4+/CD8+ ratio.

The polyclonal activation of T-cells was achieved by strong stimulation with CD3/CD28 and IL-2. In addition, the long intracellular half-life of FV vectors also allows testing a gentle stimulation, since no rapid proliferation of T-cells is required.

The gene transfer rates through gentle stimulation are lower than CD3/CD28 activated T-cells. Therefore, a gentle T-cell stimulation coupled with efficient transduction rate and a reasonable rate of proliferation is developed. For this purpose, the transduction efficiency on T-cells pre-stimulated with CD3 and CD28 antibodies and interleukin IL-2, IL-7, IL-15, and IL-21 (alone or in combination) is tested.

These experiments are performed with existing FV vectors, which possess a strong viral SFFV U3 or a weaker human PGK promoter. The optimization of the T-cell stimulation is independent of the FV vector development, since the transduction efficiency is primarily dependent on the T-cell proliferation and the viral envelope. Pseudotyping of FV vectors with other *env* proteins such as VSV-G or GALV is not possible, therefore no optimization regarding the envelope can be fulfilled. Thus, the optimization of the T-cell stimulation is performed parallel to the vector development. EGFP used as transgene achieves a simple and quick assessment of the transduction efficiency by FACS. Furthermore, the T-cell expansion is assessed via cell counting (using a defined initial cell number after Ficoll) and the toxicity by propidium iodide staining (using FACS).

### References:

Barrett, A.J. and V. Malkovska, Graft-versus-leukaemia: understanding and using the alloimmune response to treat haematological malignancies. Br J Haematol, 1996. 93(4): p. 754-61.
Bonini, C., et al., HSV-TK gene transfer into donor lymphocytes for control of allogeneic graft-versus-leukemia [see comments]. Science, 1997. 276(5319): p. 1719-24.
Bordignon, C., et al., Transfer of the HSV-tk gene into donor peripheral blood lymphocytes for in vivo modulation of donor anti-tumor immunity after allogeneic bone marrow transplantation. Hum Gene Ther, 1995. 6(6): p. 813-9.
Boyd, M.R. and H.M. Reznik-Schuller, Metabolic basis for the pulmonary Clara cell as a target for pulmonary carcinogenesis. Toxicol Pathol, 1984. 12(1): p. 56-61.
Cai, S., et al., In vivo selection of hematopoietic stem cells transduced at a low multiplicity-of-infection with a foamyviral MGMT(P140K) vector. Exp Hematol, 2008. 36(3): p. 283-92.
Collins, R.H., Jr., et al., Donor leukocyte infusions in 140 patients with relapsed malignancy after allogeneic bone marrow transplantation. J Clin Oncol, 1997. 15(2): p. 433-44.
Czerwinski, M., et al., Metabolic activation of 4-ipomeanol by complementary DNA-expressed human cytochromes P-450: evidence for species-specific metabolism. Cancer Res, 1991. 51(17); p. 4636-8.
Kasturi, V.K., et al., Phase I study of a five-day dose schedule of 4-Ipomeanol in patients with non-small cell lung cancer. Clin Cancer Res, 1998. 4(9): p. 2095-102.
Kingsman, S.M., et al., Potential oncogene activity of the woodchuck hepatitis post-transcriptional regulatory element (WPRE). Gene Ther, 2005. 12(1); p. 3-4.
Krauss J., et al., Short and Effective Synthesis of a Thiophene Analogue of (±)-4-Ipomeanol and Its Biological Evaluation. Turk J Chem, 2006. 30: p. 451 - 454.
Rethwilm, A., Foamy virus vectors: an awaited alternative to gammaretro- and lentiviral vectors. Curr Gene Ther, 2007. 7(4): p. 261-71.
Rowinsky, E.K., et al., Phase I and pharmacological study of the pulmonary cytotoxin 4-ipomeanol on a single dose schedule in lung cancer patients: hepatotoxicity is dose limiting in humans. Cancer Res, 1993. 53(8): p. 1794-801.
Tiberghien, P., et al., Administration of herpes simplex-thymidine kinase-expressing donor T cells with a T-celf-depleted allogeneic marrow graft. Blood, 2001. 97(1): p. 63-72.

## Claims

1. A modified human CYP4B1 protein with a serine to proline exchange in the meander region, wherein the 5'-region, the middle region, the 3'-region of the human CYP4B1 protein and/or a part of any of the regions is replaced by a corresponding part of a CYP4B1 protein of a different species.

2. The modified human CYP4B1 protein of claim 1, wherein the part of the middle region that is replaced by a corresponding part of a CYP4B1 protein of a different species consists of amino acids 124-135, amino acids 156-190 and/or amino acids 199-227, preferably of amino acids 324-227 of the human GYP4B1 protein.

3. The modified human CYP4B1 protein of claim 2, wherein amino acids 124-135 are replaced by SEQ ID NO.: 16, amino acids 156-190 are replaced by SEQ ID NO.: 17, and/or amino acids 199-227 are replaced by SEQ ID NO.: 18, preferably amino acids 124-227 are replaced by SEQ ID NO.: 19.

4. A modified human CYP4B1 protein, wherein at least one part of the human CYP4B1 protein is replaced by a corresponding part of a CYP4B1 protein of a different species.

5. The modified human CYP4B1 protein of claim 4, wherein the part of the human CYP4B1 protein, which is replaced, comprises a serine to proline exchange in the meander region, preferably at amino acid position 427 of the human CYP4B1 protein.

6. The modified human CYP4B1 protein of claim 4 or 5, wherein the part of the CYP4B1 protein, which is replaced, comprises the 5'-region, the middle region, the 3'-region of the CYP4B1 protein and/or a part of any of the regions.

7. The modified human CYP4B1 protein of any of claims 1 to 6, wherein the different species is a non-human mammal, preferably selected from the group consisting of rabbit, dog, rat, mouse, chimpanzee and gorilla.

8. A modified human CPY4B1 protein with a serine to proline exchange at position 427, wherein amino acids 124-227 consist of SEQ ID NO.: 19.

9. A nucleic acid encoding for a modified human CYP4B1 protein, wherein at least one part of the human CYP4B1 protein is replaced by a corresponding part of a CYP4B1 protein of a different species.

10. The nucleic acid of claim 9, wherein the nucleic acid is optimized for human codon usage.

11. An expression vector comprising a nucleic acid, preferably DNA or RNA, encoding for a modified human CYP4B1 protein, wherein at least one part of the human CYP4B1 protein is replaced by the corresponding part of a CYP4B1 protein of a different species.

12. A cell comprising a modified CYP4B1 protein, wherein at least one part of the human CYP4B1 protein is replaced by the corresponding part of a CYP4B1 protein of a different species.

13. The cell of claim 12, wherein the cell is to be transplanted, preferably by donor lymphocyte infusion.

14. A modified human CYP4B1 protein for use in treating transplantation induced graft-versus-host-disease (GVHD), wherein at least one part of the human CYP4B1 protein is replaced by the corresponding part of a CYP4B1 protein of a different species.

15. 4-ipomeanol (4-IPO) for use in killing a cell, wherein the cell comprises a modified human CYP4B1 protein with at least one part of the human CYP4B1 protein replaced by a corresponding part of a CYP4B1 protein of a different species.


SEQUENCE LISTING

<110>  Hanenberg, Helmut

<120>  Cytochrome P450 suicide gene system

<130>  H30012

<160>  92

<170>  PatentIn version 3.3

<210>  1
<211>  511
<212>  PRT
<213>  Homo sapiens

<400>  1

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Ser Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  2
<211>  1536
<212>  DNA
<213>  Homo sapiens

<400>  2
atggtgccca gcttcctctc cctgagcttc tcctccttgg gcctgtgggc ttctggcctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgactc tctgcgcttt tccactgaga atgcatccaa acgccatccc 1320

tttgccttta tgcccttctc tgctgggccc aggaactgca ttgggcagca gtttgccatg 1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 3
<211> 1536
<212> DNA
<213> Homo sapiens

<400> 3
atggtgccca gctttctgag cctgagcttc agctctctgg gcctgtgggc atctggcctg 60

atcctggtgc tgggcttcct gaagctgatc cacctgctgc tgcggcggca gaccctggcc 120

aaggccatgg acaagttccc cggacccccc acccactggc tgttcggcca cgccctggaa 180

atccaggaaa ccggcagcct ggacaaggtg gtgtcctggg cccaccagtt cccttacgcc 240

caccccctgt ggttcggcca gttcatcggc tttctgaaca tctacgagcc cgactacgcc 300

aaggccgtct acagcagagg cgaccccaag gcccccgacg tgtacgactt cttcctgcag 360

tggatcggca gaggcctcct ggtcctggaa ggccccaagt ggctgcagca ccggaagctg 420

ctgacccccg gcttccacta cgacgtgctg aagccctacg tggctgtgtt cacagagtct 480

acccggatca tgctggacaa gtgggaggaa aaggcccggg agggcaagag cttcgacatc 540

ttctgcgatg tgggccatat ggctctgaac accctgatga agtgcacctt tggcagagga 600

gataccggcc tgggccaccg ggacagcagc tactacctgg ccgtgagcga cctgaccctg 660

ctgatgcagc agcggctggt gtccttccag taccacaacg acttcatcta ctggctgaca 720

ccccatggca ggcggtttct gcgggcctgc caggtggccc acgaccacac cgaccaggtg 780

atccgggagc ggaaggccgc tctgcaggat gagaaagtgc ggaagaagat ccagaaccgg 840

cggcacctgg actttctgga catcctgctg ggcgccaggg acgaggacga catcaagctg 900

tccgacgccg acctgcgggc cgaggtggac accttcatgt tcgagggcca cgacaccacc 960

acctccggca tcagctggtt tctgtactgc atggccctgt accccgagca ccagcacagg 1020

tgccgggagg aagtgcggga gatcctgggc gaccaggact tcttccagtg ggacgacctg 1080

ggcaagatga cctacctgac catgtgcatc aaagagagct tccggctgta cccccccgtg 1140

ccccaggtgt accggcagct gtccaagccc gtgaccttcg tggacggcag aagcctgcct 1200

gccggctccc tgatcagcat gcacatctac gccctgcacc ggaacagcgc cgtgtggccc 1260

gaccccgagg tgttcgactc cctgcggttc agcaccgaga acgccagcaa gcggcacccc 1320

ttcgccttca tgcccttcag cgccggaccc cggaactgca tcggccagca gttcgccatg 1380

agcgagatga aggtggtgac cgccatgtgc ctgctgcggt tcgagttcag cctggacccc 1440

tctcggctcc ccatcaagat gccccagctg gtgctgcgga gcaagaacgg cttccacctg 1500

cacctgaagc ccctgggccc tggcagcggc aagtga 1536

<210> 4
<211> 121
<212> PRT
<213> Homo sapiens

<400> 4

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp
        115                 120

<210> 5
<211> 266
<212> PRT
<213> Homo sapiens

<400> 5

Ile Gly Arg Gly Leu Leu Val Leu Glu Gly Pro Lys Trp Leu Gln His
1               5                   10                  15

Arg Lys Leu Leu Thr Pro Gly Phe His Tyr Asp Val Leu Lys Pro Tyr
            20                  25                  30

Val Ala Val Phe Thr Glu Ser Thr Arg Ile Met Leu Asp Lys Trp Glu
        35                  40                  45

Glu Lys Ala Arg Glu Gly Lys Ser Phe Asp Ile Phe Cys Asp Val Gly
    50                  55                  60

His Met Ala Leu Asn Thr Leu Met Lys Cys Thr Phe Gly Arg Gly Asp
65                  70                  75                  80

Thr Gly Leu Gly His Arg Asp Ser Ser Tyr Tyr Leu Ala Val Ser Asp
                85                  90                  95

Leu Thr Leu Leu Met Gln Gln Arg Leu Val Ser Phe Gln Tyr His Asn
            100                 105                 110

Asp Phe Ile Tyr Trp Leu Thr Pro His Gly Arg Arg Phe Leu Arg Ala
        115                 120                 125

Cys Gln Val Ala His Asp His Thr Asp Gln Val Ile Arg Glu Arg Lys
    130                 135                 140

Ala Ala Leu Gln Asp Glu Lys Val Arg Lys Lys Ile Gln Asn Arg Arg
145                 150                 155                 160

His Leu Asp Phe Leu Asp Ile Leu Leu Gly Ala Arg Asp Glu Asp Asp
                165                 170                 175

Ile Lys Leu Ser Asp Ala Asp Leu Arg Ala Glu Val Asp Thr Phe Met
            180                 185                 190

Phe Glu Gly His Asp Thr Thr Thr Ser Gly Ile Ser Trp Phe Leu Tyr
        195                 200                 205

Cys Met Ala Leu Tyr Pro Glu His Gln His Arg Cys Arg Glu Glu Val
    210                 215                 220

Arg Glu Ile Leu Gly Asp Gln Asp Phe Phe Gln Trp Asp Asp Leu Gly
225                 230                 235                 240

Lys Met Thr Tyr Leu Thr Met Cys Ile Lys Glu Ser Phe Arg Leu Tyr
                245                 250                 255

Pro Pro Val Pro Gln Val Tyr Arg Gln Leu
            260                 265

<210>  6
<211>  124
<212>  PRT
<213>  Homo sapiens

<400>  6

Ser Lys Pro Val Thr Phe Val Asp Gly Arg Ser Leu Pro Ala Gly Ser
1               5                   10                  15

Leu Ile Ser Met His Ile Tyr Ala Leu His Arg Asn Ser Ala Val Trp
            20                  25                  30

Pro Asp Pro Glu Val Phe Asp Pro Leu Arg Phe Ser Thr Glu Asn Ala
        35                  40                  45

Ser Lys Arg His Pro Phe Ala Phe Met Pro Phe Ser Ala Gly Pro Arg
    50                  55                  60

Asn Cys Ile Gly Gln Gln Phe Ala Met Ser Glu Met Lys Val Val Thr
65                  70                  75                  80

Ala Met Cys Leu Leu Arg Phe Glu Phe Ser Leu Asp Pro Ser Arg Leu
                85                  90                  95

Pro Ile Lys Met Pro Gln Leu Val Leu Arg Ser Lys Asn Gly Phe His
            100                 105                 110

Leu His Leu Lys Pro Leu Gly Pro Gly Ser Gly Lys
        115                 120

<210>  7
<211>  12
<212>  PRT
<213>  Homo sapiens

<400>  7

Arg Gly Leu Leu Val Leu Glu Gly Pro Lys Trp Leu
1               5                   10

<210>  8
<211>  35
<212>  PRT
<213>  Homo sapiens

<400>  8

Val Phe Thr Glu Ser Thr Arg Ile Met Leu Asp Lys Trp Glu Glu Lys
1               5                   10                  15

Ala Arg Glu Gly Lys Ser Phe Asp Ile Phe Cys Asp Val Gly His Met
            20                  25                  30

Ala Leu Asn
        35

<210>  9
<211>  29
<212>  PRT
<213>  Homo sapiens

<400>  9

Arg Gly Asp Thr Gly Leu Gly His Arg Asp Ser Ser Tyr Tyr Leu Ala
1               5                   10                  15

Val Ser Asp Leu Thr Leu Leu Met Gln Gln Arg Leu Val
            20                  25

<210>  10
<211>  115
<212>  PRT
<213>  Homo sapiens

<400>  10

Ile Gly Arg Gly Leu Leu Val Leu Glu Gly Pro Lys Trp Leu Gln His
1               5                   10                  15

Arg Lys Leu Leu Thr Pro Gly Phe His Tyr Asp Val Leu Lys Pro Tyr
            20                  25                  30

Val Ala Val Phe Thr Glu Ser Thr Arg Ile Met Leu Asp Lys Trp Glu
        35                  40                  45

Glu Lys Ala Arg Glu Gly Lys Ser Phe Asp Ile Phe Cys Asp Val Gly
    50                  55                  60

His Met Ala Leu Asn Thr Leu Met Lys Cys Thr Phe Gly Arg Gly Asp
65                  70                  75                  80

Thr Gly Leu Gly His Arg Asp Ser Ser Tyr Tyr Leu Ala Val Ser Asp
                85                  90                  95

Leu Thr Leu Leu Met Gln Gln Arg Leu Val Ser Phe Gln Tyr His Asn
            100                 105                 110

Asp Phe Ile
        115

<210>  11
<211>  503
<212>  PRT
<213>  Oryctolagus cuniculus

<400>  11

Met Leu Gly Phe Leu Ser Arg Leu Gly Leu Trp Ala Ser Gly Leu Ile
1               5                   10                  15

Leu Ile Leu Gly Phe Leu Lys Leu Leu Arg Leu Leu Leu Arg Arg Gln
            20                  25                  30

Arg Leu Ala Arg Ala Met Asp Ser Phe Pro Gly Pro Pro Thr His Trp
        35                  40                  45

Leu Phe Gly His Ala Leu Glu Ile Gln Lys Thr Gly Ser Leu Asp Lys
    50                  55                  60

Val Val Thr Trp Thr Gln Gln Phe Pro Tyr Ala His Pro Leu Trp Val
65                  70                  75                  80

Gly Gln Phe Ile Gly Phe Leu Asn Ile Tyr Glu Pro Asp Tyr Ala Lys
                85                  90                  95

Ala Val Tyr Ser Arg Gly Asp Pro Lys Ala Pro Asp Val Tyr Asp Phe
            100                 105                 110

Phe Leu Gln Trp Ile Gly Lys Gly Leu Leu Val Leu Asp Gly Pro Lys
        115                 120                 125

Trp Phe Gln His Arg Lys Leu Leu Thr Pro Gly Phe His Tyr Asp Val
    130                 135                 140

Leu Lys Pro Tyr Val Ala Ile Phe Ala Asp Ser Thr Arg Ile Met Leu
145                 150                 155                 160

Glu Lys Trp Glu Lys Lys Ala Cys Glu Gly Lys Ser Phe Asp Ile Phe
                165                 170                 175

Ser Asp Val Gly His Met Ala Leu Asp Thr Leu Met Lys Cys Thr Phe
            180                 185                 190

Gly Lys Gly Asp Ser Gly Leu Asn His Arg Asp Ser Ser Tyr Tyr Val
        195                 200                 205

Ala Val Ser Glu Leu Thr Leu Leu Met Gln Gln Arg Ile Asp Ser Phe
    210                 215                 220

Gln Tyr His Asn Asp Phe Ile Tyr Trp Leu Thr Pro His Gly Arg Arg
225                 230                 235                 240

Phe Leu Arg Ala Cys Arg Ala Ala His Asp His Thr Asp Arg Val Ile
                245                 250                 255

Arg Gln Arg Lys Ala Ala Leu Gln Asp Glu Lys Glu Arg Glu Lys Ile
            260                 265                 270

Gln Asn Arg Arg His Leu Asp Phe Leu Asp Ile Leu Leu Asp Val Arg
        275                 280                 285

Gly Glu Ser Gly Val Gln Leu Ser Asp Thr Asp Leu Arg Ala Glu Val
    290                 295                 300

Asp Thr Phe Met Phe Glu Gly His Asp Thr Thr Thr Ser Gly Ile Ser
305                 310                 315                 320

Trp Phe Leu Tyr Cys Met Ala Leu Tyr Pro Glu His Gln Gln Arg Cys
                325                 330                 335

Arg Glu Glu Val Arg Glu Ile Leu Gly Asp Gln Asp Ser Phe Gln Trp
            340                 345                 350

Glu Asp Leu Ala Lys Met Thr Tyr Leu Thr Met Cys Met Lys Glu Cys
        355                 360                 365

Phe Arg Leu Tyr Pro Pro Val Pro Gln Val Tyr Arg Gln Leu Ser Lys
    370                 375                 380

Pro Val Ser Phe Val Asp Gly Arg Ser Leu Pro Ala Gly Ser Leu Ile
385                 390                 395                 400

Ser Leu His Ile Tyr Ala Leu His Arg Asn Ser Asp Val Trp Pro Asp
                405                 410                 415

Pro Glu Val Phe Asp Pro Leu Arg Phe Ser Pro Glu Asn Ser Ser Gly
            420                 425                 430

Arg His Pro Tyr Ala Phe Ile Pro Phe Ser Ala Gly Pro Arg Asn Cys
        435                 440                 445

Ile Gly Gln Gln Phe Ala Met Asn Glu Met Lys Val Val Thr Ala Leu
    450                 455                 460

Cys Leu Leu Arg Phe Glu Phe Ser Val Asp Pro Leu Arg Leu Pro Ile
465                 470                 475                 480

Lys Leu Pro Gln Leu Val Leu Arg Ser Lys Asn Gly Ile His Leu Tyr
                485                 490                 495

Leu Lys Pro Leu Gly Pro Lys
            500

<210>  12
<211>  1512
<212>  DNA
<213>  Oryctolagus cuniculus

<400>  12
atgctcggct tcctctcccg cctgggcctg tgggcttccg gactgatctt gatcctaggc      60

ttcctcaagc tcctccgcct gctgcttcga aggcagaggt tggcccgggc catggacagc     120

ttcccagggc cacccactca ctggctgttt gggcacgccc tcgagatcca gaagacgggg     180

agcctggaca aggtggtgac ctggacccag cagttcccct acgcccaccc tctctgggtt     240

ggacagttca ttggcttcct gaacatctac gagcccgact acgccaaagc tgtgtacagc 300

cgtggggacc ctaaagcccc ggatgtgtat gacttcttcc tccagtggat tggcaaaggc 360

ctgctggttc tggatggccc caagtggttc cagcaccgca agctgctcac gcctggcttc 420

cattacgacg tgctgaagcc ctacgtggcc atctttgccg actccacacg catcatgctg 480

gaaaaatggg agaaaaaggc ctgtgagggt aagagcttcg acatcttctc tgacgtgggc 540

cacatggcgc tcgacacgct catgaagtgt acgtttggca aaggagacag tggcctgaat 600

cacagggaca gcagctacta cgtggcagtc agcgagctca cgctgctgat gcagcaacgc 660

atcgactcct tccagtacca caacgacttc atctactggc tcactccgca cggccgccgc 720

ttcctgcggg cctgcagggc ggcccacgac cacaccgaca gggtcatcag acagcggaag 780

gcagccctgc aggatgagaa ggagcgggag aagatccaga accggagaca tctggacttc 840

ctggacattc tcttggatgt ccgcggtgaa agtggagtcc agctgtcgga cacagacctc 900

cgcgctgaag tggacacgtt catgttcgaa ggtcatgaca ccaccaccag cggcatctcc 960

tggttcctct actgcatggc cttgtaccct gagcaccagc agcgctgtag ggaggaggtc 1020

cgtgagatcc tgggagacca ggactccttc cagtgggagg acttggccaa gatgacctac 1080

ctgaccatgt gcatgaagga gtgcttccgc ctctacccgc ccgtgcccca ggtgtaccgc 1140

cagctcagca agcccgtcag ctttgtggac ggccgctccc tgcctgcagg cagcctgatc 1200

tccctgcata tctacgccct ccataggaac agcgacgtgt ggcctgaccc tgaggtcttt 1260

gaccccctgc gcttttcccc ggagaactcg tctggacgcc acccctatgc cttcattccc 1320

ttctctgccg ggcccaggaa ctgcatcggg cagcagttcg ccatgaacga gatgaaggtg 1380

gtcacagccc tgtgcctgct ccgctttgag ttctccgtgg accccctgcg gctgcccatc 1440

aagctgcccc agctggtcct gcgctccaag aatggcatcc acctctactt gaagcctctg 1500

ggccccaagt ag 1512

<210> 13
<211> 116
<212> PRT
<213> Oryctolagus cuniculus

<400> 13

Met Leu Gly Phe Leu Ser Arg Leu Gly Leu Trp Ala Ser Gly Leu Ile
1               5                   10                  15

Leu Ile Leu Gly Phe Leu Lys Leu Leu Arg Leu Leu Leu Arg Arg Gln
            20                  25                  30

Arg Leu Ala Arg Ala Met Asp Ser Phe Pro Gly Pro Pro Thr His Trp
        35                  40                  45

Leu Phe Gly His Ala Leu Glu Ile Gln Lys Thr Gly Ser Leu Asp Lys
    50                  55                  60

Val Val Thr Trp Thr Gln Gln Phe Pro Tyr Ala His Pro Leu Trp Val
65                  70                  75                  80

Gly Gln Phe Ile Gly Phe Leu Asn Ile Tyr Glu Pro Asp Tyr Ala Lys
                85                  90                  95

Ala Val Tyr Ser Arg Gly Asp Pro Lys Ala Pro Asp Val Tyr Asp Phe
            100                 105                 110

Phe Leu Gln Trp
        115

<210>  14
<211>  266
<212>  PRT
<213>  Oryctolagus cuniculus

<400>  14

Ile Gly Lys Gly Leu Leu Val Leu Asp Gly Pro Lys Trp Phe Gln His
1               5                   10                  15

Arg Lys Leu Leu Thr Pro Gly Phe His Tyr Asp Val Leu Lys Pro Tyr
            20                  25                  30

Val Ala Ile Phe Ala Asp Ser Thr Arg Ile Met Leu Glu Lys Trp Glu
        35                  40                  45

Lys Lys Ala Cys Glu Gly Lys Ser Phe Asp Ile Phe Ser Asp Val Gly
    50                  55                  60

His Met Ala Leu Asp Thr Leu Met Lys Cys Thr Phe Gly Lys Gly Asp
65                  70                  75                  80

Ser Gly Leu Asn His Arg Asp Ser Ser Tyr Tyr Val Ala Val Ser Glu
                85                  90                  95

Leu Thr Leu Leu Met Gln Gln Arg Ile Asp Ser Phe Gln Tyr His Asn
            100                 105                 110

Asp Phe Ile Tyr Trp Leu Thr Pro His Gly Arg Arg Phe Leu Arg Ala
        115                 120                 125

Cys Arg Ala Ala His Asp His Thr Asp Arg Val Ile Arg Gln Arg Lys
    130                 135                 140

Ala Ala Leu Gln Asp Glu Lys Glu Arg Glu Lys Ile Gln Asn Arg Arg
145                 150                 155                 160

His Leu Asp Phe Leu Asp Ile Leu Leu Asp Val Arg Gly Glu Ser Gly
                165                 170                 175

Val Gln Leu Ser Asp Thr Asp Leu Arg Ala Glu Val Asp Thr Phe Met
            180                 185                 190

Phe Glu Gly His Asp Thr Thr Thr Ser Gly Ile Ser Trp Phe Leu Tyr
        195                 200                 205

Cys Met Ala Leu Tyr Pro Glu His Gln Gln Arg Cys Arg Glu Glu Val
    210                 215                 220

Arg Glu Ile Leu Gly Asp Gln Asp Ser Phe Gln Trp Glu Asp Leu Ala
225                 230                 235                 240

Lys Met Thr Tyr Leu Thr Met Cys Met Lys Glu Cys Phe Arg Leu Tyr
                245                 250                 255

Pro Pro Val Pro Gln Val Tyr Arg Gln Leu
            260                 265

<210>  15
<211>  121
<212>  PRT
<213>  Oryctolagus cuniculus

<400>  15

Ser Lys Pro Val Ser Phe Val Asp Gly Arg Ser Leu Pro Ala Gly Ser
1               5                   10                  15

Leu Ile Ser Leu His Ile Tyr Ala Leu His Arg Asn Ser Asp Val Trp
            20                  25                  30

Pro Asp Pro Glu Val Phe Asp Pro Leu Arg Phe Ser Pro Glu Asn Ser
        35                  40                  45

Ser Gly Arg His Pro Tyr Ala Phe Ile Pro Phe Ser Ala Gly Pro Arg
    50                  55                  60

Asn Cys Ile Gly Gln Gln Phe Ala Met Asn Glu Met Lys Val Val Thr
65                  70                  75                  80

Ala Leu Cys Leu Leu Arg Phe Glu Phe Ser Val Asp Pro Leu Arg Leu
                85                  90                  95

Pro Ile Lys Leu Pro Gln Leu Val Leu Arg Ser Lys Asn Gly Ile His
            100                 105                 110

Leu Tyr Leu Lys Pro Leu Gly Pro Lys
        115                 120

<210>  16
<211>  12
<212>  PRT
<213>  Oryctolagus cuniculus

<400>  16

Lys Gly Leu Leu Val Leu Asp Gly Pro Lys Trp Phe
1               5                   10

<210>  17
<211>  35
<212>  PRT
<213>  Oryctolagus cuniculus

<400>  17

Ile Phe Ala Asp Ser Thr Arg Ile Met Leu Glu Lys Trp Glu Lys Lys
1               5                   10                  15

Ala Cys Glu Gly Lys Ser Phe Asp Ile Phe Ser Asp Val Gly His Met
            20                  25                  30

Ala Leu Asp
        35

<210>  18
<211>  29
<212>  PRT
<213>  Oryctolagus cuniculus

<400>  18

Lys Gly Asp Ser Gly Leu Asn His Arg Asp Ser Ser Tyr Tyr Val Ala
1               5                   10                  15

Val Ser Glu Leu Thr Leu Leu Met Gln Gln Arg Ile Asp
            20                  25

<210>  19
<211>  115
<212>  PRT
<213>  Oryctolagus cuniculus

<400>  19

Ile Gly Lys Gly Leu Leu Val Leu Asp Gly Pro Lys Trp Phe Gln His
1               5                   10                  15

Arg Lys Leu Leu Thr Pro Gly Phe His Tyr Asp Val Leu Lys Pro Tyr
            20                  25                  30

Val Ala Ile Phe Ala Asp Ser Thr Arg Ile Met Leu Glu Lys Trp Glu

35                  40                  45

Lys Lys Ala Cys Glu Gly Lys Ser Phe Asp Ile Phe Ser Asp Val Gly
    50                  55                  60

His Met Ala Leu Asp Thr Leu Met Lys Cys Thr Phe Gly Lys Gly Asp
65                  70                  75                  80

Ser Gly Leu Asn His Arg Asp Ser Ser Tyr Tyr Val Ala Val Ser Glu
                85                  90                  95

Leu Thr Leu Leu Met Gln Gln Arg Ile Asp Ser Phe Gln Tyr His Asn
            100                 105                 110

Asp Phe Ile
        115

<210>  20
<211>  512
<212>  PRT
<213>  Homo sapiens

<400>  20

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Arg Gly Asp Thr Gly Leu Gly His Ser Arg
        195                 200                 205

Asp Ser Ser Tyr Tyr Leu Ala Val Ser Asp Leu Thr Leu Leu Met Gln
    210                 215                 220

Gln Arg Leu Val Ser Phe Gln Tyr His Asn Asp Phe Ile Tyr Trp Leu
225                 230                 235                 240

Thr Pro His Gly Arg Arg Phe Leu Arg Ala Cys Gln Val Ala His Asp
                245                 250                 255

His Thr Asp Gln Val Ile Arg Glu Arg Lys Ala Ala Leu Gln Asp Glu
            260                 265                 270

Lys Val Arg Lys Lys Ile Gln Asn Arg Arg His Leu Asp Phe Leu Asp
        275                 280                 285

Ile Leu Leu Gly Ala Arg Asp Glu Asp Asp Ile Lys Leu Ser Asp Ala
    290                 295                 300

Asp Leu Arg Ala Glu Val Asp Thr Phe Met Phe Glu Gly His Asp Thr
305                 310                 315                 320

Thr Thr Ser Gly Ile Ser Trp Phe Leu Tyr Cys Met Ala Leu Tyr Pro
                325                 330                 335

Glu His Gln His Arg Cys Arg Glu Glu Val Arg Glu Ile Leu Gly Asp
            340                 345                 350

Gln Asp Phe Phe Gln Trp Asp Asp Leu Gly Lys Met Thr Tyr Leu Thr
        355                 360                 365

Met Cys Ile Lys Glu Ser Phe Arg Leu Tyr Pro Pro Val Pro Gln Val
    370                 375                 380

Tyr Arg Gln Leu Ser Lys Pro Val Thr Phe Val Asp Gly Arg Ser Leu
385                 390                 395                 400

Pro Ala Gly Ser Leu Ile Ser Met His Ile Tyr Ala Leu His Arg Asn
                405                 410                 415

Ser Ala Val Trp Pro Asp Pro Glu Val Phe Asp Ser Leu Arg Phe Ser
            420                 425                 430

Thr Glu Asn Ala Ser Lys Arg His Pro Phe Ala Phe Met Pro Phe Ser
        435                 440                 445

Ala Gly Pro Arg Asn Cys Ile Gly Gln Gln Phe Ala Met Ser Glu Met
    450                 455                 460

Lys Val Val Thr Ala Met Cys Leu Leu Arg Phe Glu Phe Ser Leu Asp
465                 470                 475                 480

Pro Ser Arg Leu Pro Ile Lys Met Pro Gln Leu Val Leu Arg Ser Lys
                485                 490                 495

Asn Gly Phe His Leu His Leu Lys Pro Leu Gly Pro Gly Ser Gly Lys
            500                 505                 510

<210>  21
<211>  1539
<212>  DNA
<213>  Homo sapiens

<400>  21
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag gagcgacagc agctactacc ttgcagtcag cgatctcact     660

ctgttgatgc agcagcgcct tgtgtccttc cagtaccata atgacttcat ctactggctc     720

accccacatg gccgccgctt cctgcgggcc tgccaggtgg cccatgacca tacagaccag     780

gtcatcaggg agcggaaggc agccctgcag gatgagaagg tgcggaagaa gatccagaac     840

cggaggcacc tggacttcct ggacattctc ctgggtgccc gggatgaaga tgacatcaaa     900

ctgtcagatg cagacctccg ggctgaagtg gacacattca tgtttgaagg ccatgacacc     960

accaccagtg gtatctcctg gtttctctac tgcatggccc tgtaccctga gcaccagcat 1020

cgttgtagag aggaggtccg cgagatccta ggggaccagg acttcttcca gtgggatgat 1080

ctgggcaaaa tgacttatct gaccatgtgc atcaaggaga gcttccgcct ctacccacct 1140

gtgccccagg tgtaccgcca gctcagcaag cctgtcacct ttgtggatgg ccggtctcta 1200

cctgcaggaa gcctgatctc tatgcatatc tatgccctcc ataggaacag tgctgtatgg 1260

cccgaccctg aggtctttga ctctctgcgc ttttccactg agaatgcatc caaacgccat 1320

ccctttgcct ttatgccctt ctctgctggg cccaggaact gcattgggca gcagtttgcc 1380

atgagtgaga tgaaggtggt cacagccatg tgcttgctcc gctttgagtt ctctctggac 1440

ccctcacggc tgcccatcaa gatgccccag cttgtcctgc gctccaagaa tggctttcac 1500

ctccacctga agccactggg ccctgggtct gggaagtag 1539

<210> 22
<211> 363
<212> DNA
<213> Homo sapiens

<400> 22
atggtgccca gcttcctctc cctgagcttc tcctccttgg gcctgtgggc ttctgggctg 60

atcttggtct taggctttct caagctcatc cacctgctgc tgcggaggca gacgttggct 120

aaggctatgg acaaattccc agggcctccc acccactggc tttttggaca tgccctcgag 180

atccaggaga cggggagcct ggacaaagtg gtgtcctggg cccaccagtt cccgtatgcc 240

cacccactct ggttcggaca gttcattggc ttcctgaaca tctatgagcc tgactatgcc 300

aaagctgtgt acagccgtgg ggaccctaag gcccctgatg tgtatgactt cttcctccag 360

tgg 363

<210> 23
<211> 798
<212> DNA
<213> Homo sapiens

<400> 23
attgggagag gcctgctggt tcttgagggg cccaagtggt tgcagcaccg caagctgctc 60

acacctggct ttcattatga tgtgctgaag ccctatgtgg ccgtgttcac tgagtctaca 120

cgtatcatgc tggacaagtg ggaagagaaa gctcgggagg gtaagtcctt tgacatcttc 180

tgcgatgtgg gtcacatggc gctgaacaca ctcatgaagt gcacctttgg aagaggagac 240

accggcctgg gccacaggga cagcagctac taccttgcag tcagcgatct cactctgttg 300

atgcagcagc gccttgtgtc cttccagtac cataatgact tcatctactg gctcacccca 360

catggccgcc gcttcctgcg ggcctgccag gtggcccatg accatacaga ccaggtcatc 420

agggagcgga aggcagccct gcaggatgag aaggtgcgga agaagatcca gaaccggagg 480

cacctggact tcctggacat tctcctgggt gcccgggatg aagatgacat caaactgtca 540

gatgcagacc tccgggctga agtggacaca ttcatgtttg aaggccatga caccaccacc 600

agtggtatct cctggtttct ctactgcatg gccctgtacc ctgagcacca gcatcgttgt 660

agagaggagg tccgcgagat cctaggggac caggacttct tccagtggga tgatctgggc 720

aaaatgactt atctgaccat gtgcatcaag gagagcttcc gcctctaccc acctgtgccc 780

caggtgtacc gccagctc 798

<210> 24
<211> 375
<212> DNA
<213> Homo sapiens

<400> 24
agcaagcctg tcacctttgt ggatggccgg tctctacctg caggaagcct gatctctatg 60

catatctatg ccctccatag gaacagtgct gtatggcccg accctgaggt ctttgaccct 120

ctgcgctttt ccactgagaa tgcatccaaa cgccatccct ttgcctttat gcccttctct 180

gctgggccca ggaactgcat tgggcagcag tttgccatga gtgagatgaa ggtggtcaca 240

gccatgtgct tgctccgctt tgagttctct ctggacccct cacggctgcc catcaagatg 300

ccccagcttg tcctgcgctc caagaatggc tttcacctcc acctgaagcc actgggccct 360

gggtctggga agtag 375

<210> 25
<211> 348
<212> DNA
<213> Oryctolagus cuniculus

<400> 25
atgctcggct tcctctcccg cctgggcctg tgggcttccg gactgatctt gatcctaggc 60

ttcctcaagc tcctccgcct gctgcttcga aggcagaggt tggcccgggc catggacagc 120

ttcccagggc cacccactca ctggctgttt gggcacgccc tcgagatcca gaagacgggg 180

agcctggaca aggtggtgac ctggacccag cagttcccct acgcccaccc tctctgggtt 240

ggacagttca ttggcttcct gaacatctac gagcccgact acgccaaagc tgtgtacagc 300

cgtggggacc ctaaagcccc ggatgtgtat gacttcttcc tccagtgg 348

<210> 26
<211> 798
<212> DNA
<213> Oryctolagus cuniculus

<400> 26
attggcaaag gcctgctggt tctggatggg cccaagtggt tccagcaccg caagctgctc 60

acgcctggct tccattacga cgtgctgaag ccctacgtgg ccatctttgc cgactccaca 120

cgcatcatgc tggaaaaatg ggagaaaaag gcctgtgagg gtaagagctt cgacatcttc 180

tctgacgtgg gccacatggc gctcgacacg ctcatgaagt gtacgtttgg caaaggagac 240

agtggcctga atcacaggga cagcagctac tacgtggcag tcagcgagct cacgctgctg 300

atgcagcaac gcatcgactc cttccagtac cacaacgact tcatctactg gctcactccg 360

cacggccgcc gcttcctgcg ggcctgcagg gcggcccacg accacaccga cagggtcatc 420

agacagcgga aggcagccct gcaggatgag aaggagcggg agaagatcca gaaccggaga 480

catctggact tcctggacat tctcttggat gtccgcggtg aaagtggagt ccagctgtcg 540

gacacagacc tccgcgctga agtggacacg ttcatgttcg aaggtcatga caccaccacc 600

agcggcatct cctggttcct ctactgcatg gccttgtacc ctgagcacca gcagcgctgt 660

agggaggagg tccgtgagat cctgggagac caggactcct tccagtggga ggacttggcc 720

aagatgacct acctgaccat gtgcatgaag gagtgcttcc gcctctaccc gcccgtgccc 780

caggtgtacc gccagctc 798

<210> 27
<211> 366
<212> DNA
<213> Oryctolagus cuniculus

<400> 27
agcaagcccg tcagctttgt ggacggccgc tccctgcctg caggcagcct gatctccctg 60

catatctacg ccctccatag gaacagcgac gtgtggcctg accctgaggt ctttgacccc 120

ctgcgctttt ccccggagaa ctcgtctgga cgccacccct atgccttcat tcccttctct 180

gccgggccca ggaactgcat cgggcagcag ttcgccatga acgagatgaa ggtggtcaca 240

gccctgtgcc tgctccgctt tgagttctcc gtggaccccc tgcggctgcc catcaagctg 300

ccccagctgg tcctgcgctc caagaatggc atccacctct acttgaagcc tctgggcccc 360

aagtag 366

<210> 28
<211> 12
<212> PRT
<213> Homo sapiens

<400> 28

Tyr Gly Leu Leu Leu Leu Asn Gly Gln Thr Trp Phe
1               5                   10

<210> 29
<211> 35
<212> PRT
<213> Homo sapiens

<400> 29

Leu Met Ala Asp Ser Val Arg Val Met Leu Asp Lys Trp Glu Glu Leu
1               5                   10                  15

Leu Gly Gln Asp Ser Pro Leu Glu Val Phe Gln His Val Ser Leu Met
            20                  25                  30

Thr Leu Asp
        35

<210> 30
<211> 30
<212> PRT
<213> Homo sapiens

<400> 30

His Gln Gly Ser Ile Gln Val Asp Arg Asn Ser Gln Ser Tyr Ile Gln
1               5                   10                  15

Ala Ile Ser Asp Leu Asn Asn Leu Val Phe Ser Arg Val Arg
            20                  25                  30

<210> 31
<211> 12
<212> PRT
<213> Homo sapiens

<400> 31

Tyr Gly Leu Leu Leu Leu Asn Gly Gln Thr Trp Phe
1               5                   10

<210> 32
<211> 35
<212> PRT
<213> Homo sapiens

<400> 32

Leu Met Ala Asp Ser Val Arg Val Met Leu Asp Lys Trp Glu Glu Leu
1               5                   10                  15

Leu Gly Gln Asp Ser Pro Leu Glu Val Phe Gln His Val Ser Leu Met
            20                  25                  30

Thr Leu Asp
        35

<210> 33
<211> 30
<212> PRT
<213> Homo sapiens

<400> 33

His Gln Gly Ser Ile Gln Val Asp Arg Asn Ser Gln Ser Tyr Ile Gln
1               5                   10                  15

Ala Ile Ser Asp Leu Asn Ser Leu Val Phe Cys Cys Met Arg
            20                  25                  30

<210>  34
<211>  12
<212>  PRT
<213>  Homo sapiens

<400>  34

Asp Gly Leu Leu Leu Ser Ala Gly Asp Lys Trp Ser
1               5                   10

<210>  35
<211>  36
<212>  PRT
<213>  Homo sapiens

<400>  35

Ile Phe Asn Glu Ser Val Asn Ile Met His Ala Lys Trp Gln Leu Leu
1               5                   10                  15

Ala Ser Glu Gly Ser Ala Cys Leu Asp Met Phe Glu His Ile Ser Leu
            20                  25                  30

Met Thr Leu Asp
        35

<210>  36
<211>  28
<212>  PRT
<213>  Homo sapiens

<400>  36

Phe Asp Ser His Cys Gln Glu Lys Pro Ser Glu Tyr Ile Ala Ala Ile
1               5                   10                  15

Leu Glu Leu Ser Ala Leu Val Ser Lys Arg His His
            20                  25

<210>  37
<211>  12
<212>  PRT
<213>  Homo sapiens

<400>  37

Asp Gly Leu Leu Leu Ser Ala Gly Glu Lys Trp Ser
1               5                   10

<210>  38
<211>  36
<212>  PRT
<213>  Homo sapiens

<400>  38

Ile Phe Asn Glu Ser Val Asn Ile Met His Ala Lys Trp Gln Leu Leu
1               5                   10                  15

Ala Ser Glu Gly Ser Ala Arg Leu Asp Met Phe Glu His Ile Ser Leu
            20                  25                  30

Met Thr Leu Asp
        35

<210>  39
<211>  28
<212>  PRT
<213>  Homo sapiens

<400>  39

Phe Asp Ser His Cys Gln Glu Lys Pro Ser Glu Tyr Ile Ala Ala Ile
1               5                   10                  15

Leu Glu Leu Ser Ala Leu Val Thr Lys Arg His Gln
            20                  25

<210>  40
<211>  12
<212>  PRT
<213>  Homo sapiens

<400>  40

Asp Gly Leu Leu Leu Ser Val Gly Asp Lys Trp Arg
1               5                   10

<210>  41
<211>  36
<212>  PRT
<213>  Homo sapiens

<400>  41

Ile Phe Ser Lys Ser Ala Asn Ile Met His Ala Lys Trp Gln Arg Leu
1               5                   10                  15

Ala Met Glu Gly Ser Thr Cys Leu Asp Val Phe Glu His Ile Ser Leu
            20                  25                  30

Met Thr Leu Asp
        35

<210>  42
<211>  28
<212>  PRT
<213>  Homo sapiens

<400>  42

Phe Asp Ser Asn Cys Gln Glu Lys Pro Ser Glu Tyr Ile Thr Ala Ile
1               5                   10                  15

Met Glu Leu Ser Ala Leu Val Val Lys Arg Asn Asn
            20                  25

<210>  43
<211>  12
<212>  PRT
<213>  Homo sapiens

<400>  43

Asp Gly Leu Leu Leu Ser Gly Gly Asp Lys Trp Ser
1               5                   10

<210>  44
<211>  36
<212>  PRT
<213>  Homo sapiens

<400>  44

Ile Phe Asn Lys Ser Val Asn Ile Met His Asp Lys Trp Gln Arg Leu
1               5                   10                  15

Ala Ser Glu Gly Ser Ala Arg Leu Asp Met Phe Glu His Ile Ser Leu
            20                  25                  30

Met Thr Leu Asp
        35

<210>  45
<211>  28
<212>  PRT
<213>  Homo sapiens

<400>  45

Phe Glu Ser Asn Cys Gln Glu Lys Pro Ser Glu Tyr Ile Ala Ala Ile
1               5                   10                  15

Leu Glu Leu Ser Ala Phe Val Glu Lys Arg Asn Gln
            20                  25

<210>  46
<211>  12
<212>  PRT
<213>  Homo sapiens

<400>  46

Glu Gly Ile Leu Leu Ser Gly Gly Asp Lys Trp Ser
1               5                   10

<210>  47
<211>  36
<212>  PRT
<213>  Homo sapiens

<400>  47

Ile Phe Asn Lys Ser Ala Asn Ile Met Leu Asp Lys Trp Gln His Leu
1               5                   10                  15

Ala Ser Glu Gly Ser Ser Cys Leu Asp Met Phe Glu His Ile Ser Leu
            20                  25                  30

Met Thr Leu Asp
        35

<210>  48
<211>  28
<212>  PRT
<213>  Homo sapiens

<400>  48

Phe Asp Ser His Cys Gln Glu Arg Pro Ser Glu Tyr Ile Ala Thr Ile
1               5                   10                  15

Leu Glu Leu Ser Ala Leu Val Glu Lys Arg Ser Gln
            20                  25

<210>  49
<211>  12
<212>  PRT
<213>  Homo sapiens

<400>  49

Asp Gly Leu Leu Leu Ser Lys Gly Asp Lys Trp Ser
1               5                   10

<210>  50
<211>  36
<212>  PRT
<213>  Homo sapiens

<400>  50

Ile Phe Asn Gln Ser Ala Asp Ile Met His Ala Lys Trp Arg His Leu
1               5                   10                  15

Ala Glu Gly Ser Ala Val Ser Leu Asp Met Phe Glu His Ile Ser Leu
            20                  25                  30

Met Thr Leu Asp
        35

<210>  51

<211>  28
<212>  PRT
<213>  Homo sapiens

<400>  51

Tyr Asn Ser Asn Cys Gln Glu Lys Met Ser Asp Tyr Ile Ser Ala Ile
1               5                   10                  15

Ile Glu Leu Ser Ala Leu Ser Val Arg Arg Gln Tyr
            20                  25

<210>  52
<211>  12
<212>  PRT
<213>  Homo sapiens

<400>  52

Leu Gly Leu Leu Thr Ser Thr Gly Asn Lys Trp Arg
1               5                   10

<210>  53
<211>  34
<212>  PRT
<213>  Homo sapiens

<400>  53

Ile Met Asn Glu Gln Ala Asn Ile Leu Val Lys Lys Leu Glu Lys His
1               5                   10                  15

Ile Asn Gln Glu Ala Phe Asn Cys Phe Phe Tyr Ile Thr Leu Cys Ala
            20                  25                  30

Leu Asp

<210>  54
<211>  29
<212>  PRT
<213>  Homo sapiens

<400>  54

Lys Asn Ile Gly Ala Gln Ser Asn Asp Asp Ser Glu Tyr Val Arg Ala
1               5                   10                  15

Val Tyr Arg Met Ser Glu Met Ile Phe Arg Arg Ile Lys
            20                  25

<210>  55
<211>  12
<212>  PRT
<213>  Homo sapiens

<400>  55

Lys Gly Leu Ala Ala Leu Asp Gly Pro Lys Trp Phe
1               5                   10

<210>  56
<211>  36
<212>  PRT
<213>  Homo sapiens

<400>  56

Val Met Ala His Ser Val Lys Met Met Leu Asp Lys Trp Glu Lys Ile
1               5                   10                  15

Cys Ser Thr Gln Asp Thr Ser Val Glu Val Tyr Glu His Ile Asn Ser
            20                  25                  30

Met Ser Leu Asp
        35

<210>  57
<211>  30
<212>  PRT
<213>  Homo sapiens

<400>  57

Lys Glu Thr Asn Cys Gln Thr Asn Ser Thr His Asp Pro Tyr Ala Lys
1               5                   10                  15

Ala Ile Phe Glu Leu Ser Lys Ile Ile Phe His Arg Leu Tyr
            20                  25                  30

<210>  58
<211>  12
<212>  PRT
<213>  Homo sapiens

<400>  58

Arg Gly Leu Val Thr Leu Asp Gly Ser Lys Trp Lys
1               5                   10

<210>  59
<211>  35
<212>  PRT
<213>  Homo sapiens

<400>  59

Met Met Ser Glu Ser Val Arg Met Met Leu Asn Lys Trp Glu Glu His
1               5                   10                  15

Ile Ala Gln Asn Ser Arg Leu Glu Leu Phe Gln His Val Ser Leu Met
            20                  25                  30

Thr Leu Asp
        35

<210>  60
<211>  30
<212>  PRT
<213>  Homo sapiens

<400>  60

His Gln Gly Ser Ile Gln Leu Asp Ser Thr Leu Asp Ser Tyr Leu Lys
1               5                   10                  15

Ala Val Phe Asn Leu Ser Lys Ile Ser Asn Gln Arg Met Asn
            20                  25                  30

<210>  61
<211>  503
<212>  PRT
<213>  hybrid

<400>  61

Met Leu Gly Phe Leu Ser Arg Leu Gly Leu Trp Ala Ser Gly Leu Ile
1               5                   10                  15

Leu Ile Leu Gly Phe Leu Lys Leu Leu Arg Leu Leu Leu Arg Arg Gln
            20                  25                  30

Arg Leu Ala Arg Ala Met Asp Ser Phe Pro Gly Pro Pro Thr His Trp
        35                  40                  45

Leu Phe Gly His Ala Leu Glu Ile Gln Lys Thr Gly Ser Leu Asp Lys
    50                  55                  60

Val Val Thr Trp Thr Gln Gln Phe Pro Tyr Ala His Pro Leu Trp Val
65                  70                  75                  80

Gly Gln Phe Ile Gly Phe Leu Asn Ile Tyr Glu Pro Asp Tyr Ala Lys
                85                  90                  95

Ala Val Tyr Ser Arg Gly Asp Pro Lys Ala Pro Asp Val Tyr Asp Phe
            100                 105                 110

Phe Leu Gln Trp Ile Gly Arg Gly Leu Leu Val Leu Glu Gly Pro Lys
        115                 120                 125

Trp Leu Gln His Arg Lys Leu Leu Thr Pro Gly Phe His Tyr Asp Val
    130                 135                 140

Leu Lys Pro Tyr Val Ala Val Phe Thr Glu Ser Thr Arg Ile Met Leu
145                 150                 155                 160

Asp Lys Trp Glu Glu Lys Ala Arg Glu Gly Lys Ser Phe Asp Ile Phe
                165                 170                 175

Cys Asp Val Gly His Met Ala Leu Asn Thr Leu Met Lys Cys Thr Phe
            180                 185                 190

Gly Arg Gly Asp Thr Gly Leu Gly His Arg Asp Ser Ser Tyr Tyr Leu
        195                 200                 205

Ala Val Ser Asp Leu Thr Leu Leu Met Gln Gln Arg Leu Val Ser Phe
    210                 215                 220

Gln Tyr His Asn Asp Phe Ile Tyr Trp Leu Thr Pro His Gly Arg Arg
225                 230                 235                 240

Phe Leu Arg Ala Cys Gln Val Ala His Asp His Thr Asp Gln Val Ile
                245                 250                 255

Arg Glu Arg Lys Ala Ala Leu Gln Asp Glu Lys Val Arg Lys Lys Ile
            260                 265                 270

Gln Asn Arg Arg His Leu Asp Phe Leu Asp Ile Leu Leu Gly Ala Arg
        275                 280                 285

Asp Glu Asp Asp Ile Lys Leu Ser Asp Ala Asp Leu Arg Ala Glu Val
    290                 295                 300

Asp Thr Phe Met Phe Glu Gly His Asp Thr Thr Thr Ser Gly Ile Ser
305                 310                 315                 320

Trp Phe Leu Tyr Cys Met Ala Leu Tyr Pro Glu His Gln His Arg Cys
                325                 330                 335

Arg Glu Glu Val Arg Glu Ile Leu Gly Asp Gln Asp Phe Phe Gln Trp
            340                 345                 350

Asp Asp Leu Gly Lys Met Thr Tyr Leu Thr Met Cys Ile Lys Glu Ser
        355                 360                 365

Phe Arg Leu Tyr Pro Pro Val Pro Gln Val Tyr Arg Gln Leu Ser Lys
    370                 375                 380

Pro Val Ser Phe Val Asp Gly Arg Ser Leu Pro Ala Gly Ser Leu Ile
385                 390                 395                 400

Ser Leu His Ile Tyr Ala Leu His Arg Asn Ser Asp Val Trp Pro Asp
                405                 410                 415

Pro Glu Val Phe Asp Pro Leu Arg Phe Ser Pro Glu Asn Ser Ser Gly
            420                 425                 430

Arg His Pro Tyr Ala Phe Ile Pro Phe Ser Ala Gly Pro Arg Asn Cys
        435                 440                 445

Ile Gly Gln Gln Phe Ala Met Asn Glu Met Lys Val Val Thr Ala Leu
    450                 455                 460

Cys Leu Leu Arg Phe Glu Phe Ser Val Asp Pro Leu Arg Leu Pro Ile
465                 470                 475                 480

Lys Leu Pro Gln Leu Val Leu Arg Ser Lys Asn Gly Ile His Leu Tyr
                485                 490                 495

Leu Lys Pro Leu Gly Pro Lys
            500

<210> 62
<211> 1512
<212> DNA
<213> hybrid

<400> 62
atgctcggct tcctctcccg cctgggcctg tgggcttccg gactgatctt gatcctaggc 60

ttcctcaagc tcctccgcct gctgcttcga aggcagaggt tggcccgggc catggacagc 120

ttcccagggc cacccactca ctggctgttt gggcacgccc tcgagatcca gaagacgggg 180

agcctggaca aggtggtgac ctggacccag cagttcccct acgcccaccc tctctgggtt 240

ggacagttca ttggcttcct gaacatctac gagcccgact acgccaaagc tgtgtacagc 300

cgtggggacc ctaaagcccc ggatgtgtat gacttcttcc tccagtggat tgggagaggc 360

ctgctggttc ttgaggggcc caagtggttg cagcaccgca agctgctcac acctggcttt 420

cattatgatg tgctgaagcc ctatgtggcc gtgttcactg agtctacacg tatcatgctg 480

gacaagtggg aagagaaagc tcgggagggt aagtcctttg acatcttctg cgatgtgggt 540

cacatggcgc tgaacacact catgaagtgc acctttggaa gaggagacac cggcctgggc 600

cacagggaca gcagctacta ccttgcagtc agcgatctca ctctgttgat gcagcagcgc 660

cttgtgtcct tccagtacca taatgacttc atctactggc tcaccccaca tggccgccgc 720

ttcctgcggg cctgccaggt ggcccatgac catacagacc aggtcatcag ggagcggaag 780

gcagccctgc aggatgagaa ggtgcggaag aagatccaga accggaggca cctggacttc 840

ctggacattc tcctgggtgc ccgggatgaa gatgacatca aactgtcaga tgcagacctc 900

cgggctgaag tggacacatt catgtttgaa ggccatgaca ccaccaccag tggtatctcc 960

tggtttctct actgcatggc cctgtaccct gagcaccagc atcgttgtag agaggaggtc 1020

cgcgagatcc taggggacca ggacttcttc cagtgggatg atctgggcaa aatgacttat 1080

ctgaccatgt gcatcaagga gagcttccgc ctctacccac ctgtgcccca ggtgtaccgc 1140

cagctcagca agcccgtcag ctttgtggac ggccgctccc tgcctgcagg cagcctgatc 1200

tccctgcata tctacgccct ccataggaac agcgacgtgt ggcctgaccc tgaggtcttt 1260

gaccccctgc gcttttcccc ggagaactcg tctggacgcc acccctatgc cttcattccc 1320

ttctctgccg ggcccaggaa ctgcatcggg cagcagttcg ccatgaacga gatgaaggtg 1380

gtcacagccc tgtgcctgct ccgctttgag ttctccgtgg accccctgcg gctgcccatc 1440

aagctgcccc agctggtcct gcgctccaag aatggcatcc acctctactt gaagcctctg 1500

ggccccaagt ag 1512

<210> 63
<211> 506
<212> PRT
<213> hybrid

<400> 63

Met Leu Gly Phe Leu Ser Arg Leu Gly Leu Trp Ala Ser Gly Leu Ile
1               5                   10                  15

Leu Ile Leu Gly Phe Leu Lys Leu Leu Arg Leu Leu Leu Arg Arg Gln
            20                  25                  30

Arg Leu Ala Arg Ala Met Asp Ser Phe Pro Gly Pro Pro Thr His Trp
        35                  40                  45

Leu Phe Gly His Ala Leu Glu Ile Gln Lys Thr Gly Ser Leu Asp Lys
    50                  55                  60

Val Val Thr Trp Thr Gln Gln Phe Pro Tyr Ala His Pro Leu Trp Val
65                  70                  75                  80

Gly Gln Phe Ile Gly Phe Leu Asn Ile Tyr Glu Pro Asp Tyr Ala Lys
                85                  90                  95

Ala Val Tyr Ser Arg Gly Asp Pro Lys Ala Pro Asp Val Tyr Asp Phe
            100                 105                 110

Phe Leu Gln Trp Ile Gly Lys Gly Leu Leu Val Leu Asp Gly Pro Lys
        115                 120                 125

Trp Phe Gln His Arg Lys Leu Leu Thr Pro Gly Phe His Tyr Asp Val
    130                 135                 140

Leu Lys Pro Tyr Val Ala Ile Phe Ala Asp Ser Thr Arg Ile Met Leu
145                 150                 155                 160

Glu Lys Trp Glu Lys Lys Ala Cys Glu Gly Lys Ser Phe Asp Ile Phe
                165                 170                 175

Ser Asp Val Gly His Met Ala Leu Asp Thr Leu Met Lys Cys Thr Phe
            180                 185                 190

Gly Lys Gly Asp Ser Gly Leu Asn His Arg Asp Ser Ser Tyr Tyr Val
        195                 200                 205

Ala Val Ser Glu Leu Thr Leu Leu Met Gln Gln Arg Ile Asp Ser Phe
    210                 215                 220

Gln Tyr His Asn Asp Phe Ile Tyr Trp Leu Thr Pro His Gly Arg Arg
225                 230                 235                 240

Phe Leu Arg Ala Cys Arg Ala Ala His Asp His Thr Asp Arg Val Ile
                245                 250                 255

Arg Gln Arg Lys Ala Ala Leu Gln Asp Glu Lys Glu Arg Glu Lys Ile
            260                 265                 270

Gln Asn Arg Arg His Leu Asp Phe Leu Asp Ile Leu Leu Asp Val Arg
        275                 280                 285

Gly Glu Ser Gly Val Gln Leu Ser Asp Thr Asp Leu Arg Ala Glu Val
    290                 295                 300

Asp Thr Phe Met Phe Glu Gly His Asp Thr Thr Thr Ser Gly Ile Ser
305                 310                 315                 320

Trp Phe Leu Tyr Cys Met Ala Leu Tyr Pro Glu His Gln Gln Arg Cys
                325                 330                 335

Arg Glu Glu Val Arg Glu Ile Leu Gly Asp Gln Asp Ser Phe Gln Trp
            340                 345                 350

Glu Asp Leu Ala Lys Met Thr Tyr Leu Thr Met Cys Met Lys Glu Cys
        355                 360                 365

Phe Arg Leu Tyr Pro Pro Val Pro Gln Val Tyr Arg Gln Leu Ser Lys
    370                 375                 380

Pro Val Thr Phe Val Asp Gly Arg Ser Leu Pro Ala Gly Ser Leu Ile
385                 390                 395                 400

Ser Met His Ile Tyr Ala Leu His Arg Asn Ser Ala Val Trp Pro Asp
                405                 410                 415

Pro Glu Val Phe Asp Pro Leu Arg Phe Ser Thr Glu Asn Ala Ser Lys

420                 425                 430

Arg His Pro Phe Ala Phe Met Pro Phe Ser Ala Gly Pro Arg Asn Cys
        435                 440                 445

Ile Gly Gln Gln Phe Ala Met Ser Glu Met Lys Val Val Thr Ala Met
    450                 455                 460

Cys Leu Leu Arg Phe Glu Phe Ser Leu Asp Pro Ser Arg Leu Pro Ile
465                 470                 475                 480

Lys Met Pro Gln Leu Val Leu Arg Ser Lys Asn Gly Phe His Leu His
                485                 490                 495

Leu Lys Pro Leu Gly Pro Gly Ser Gly Lys
            500                 505

<210>  64
<211>  1521
<212>  DNA
<213>  hybrid

<400>  64
atgctcggct tcctctcccg cctgggcctg tgggcttccg gactgatctt gatcctaggc      60

ttcctcaagc tcctccgcct gctgcttcga aggcagaggt tggcccgggc catggacagc     120

ttcccagggc cacccactca ctggctgttt gggcacgccc tcgagatcca gaagacgggg     180

agcctggaca aggtggtgac ctggacccag cagttcccct acgcccaccc tctctgggtt     240

ggacagttca ttggcttcct gaacatctac gagcccgact acgccaaagc tgtgtacagc     300

cgtggggacc ctaaagcccc ggatgtgtat gacttcttcc tccagtggat tggcaaaggc     360

ctgctggttc tggatggccc caagtggttc cagcaccgca agctgctcac gcctggcttc     420

cattacgacg tgctgaagcc ctacgtggcc atctttgccg actccacacg catcatgctg     480

gaaaaatggg agaaaaaggc ctgtgagggt aagagcttcg acatcttctc tgacgtgggc     540

cacatggcgc tcgacacgct catgaagtgt acgtttggca aaggagacag tggcctgaat     600

cacagggaca gcagctacta cgtggcagtc agcgagctca cgctgctgat gcagcaacgc     660

atcgactcct tccagtacca caacgacttc atctactggc tcactccgca cggccgccgc     720

ttcctgcggg cctgcagggc ggcccacgac cacaccgaca gggtcatcag acagcggaag     780

gcagccctgc aggatgagaa ggagcgggag aagatccaga accggagaca tctggacttc     840

ctggacattc tcttggatgt ccgcggtgaa agtggagtcc agctgtcgga cacagacctc     900

cgcgctgaag tggacacgtt catgttcgaa ggtcatgaca ccaccaccag cggcatctcc     960

tggttcctct actgcatggc cttgtaccct gagcaccagc agcgctgtag ggaggaggtc    1020

cgtgagatcc tgggagacca ggactccttc cagtgggagg acttggccaa gatgacctac    1080

ctgaccatgt gcatgaagga gtgcttccgc ctctacccgc ccgtgcccca ggtgtaccgc 1140

cagctcagca agcctgtcac ctttgtggat ggccggtctc tacctgcagg aagcctgatc 1200

tctatgcata tctatgccct ccataggaac agtgctgtat ggcccgaccc tgaggtcttt 1260

gaccctctgc gcttttccac tgagaatgca tccaaacgcc atccctttgc ctttatgccc 1320

ttctctgctg ggcccaggaa ctgcattggg cagcagtttg ccatgagtga gatgaaggtg 1380

gtcacagcca tgtgcttgct ccgctttgag ttctctctgg acccctcacg gctgcccatc 1440

aagatgcccc agcttgtcct gcgctccaag aatggctttc acctccacct gaagccactg 1500

ggccctgggt ctgggaagta g 1521

<210> 65
<211> 506
<212> PRT
<213> hybrid

<400> 65

Met Leu Gly Phe Leu Ser Arg Leu Gly Leu Trp Ala Ser Gly Leu Ile
1               5                   10                  15

Leu Ile Leu Gly Phe Leu Lys Leu Leu Arg Leu Leu Leu Arg Arg Gln
            20                  25                  30

Arg Leu Ala Arg Ala Met Asp Ser Phe Pro Gly Pro Pro Thr His Trp
        35                  40                  45

Leu Phe Gly His Ala Leu Glu Ile Gln Lys Thr Gly Ser Leu Asp Lys
    50                  55                  60

Val Val Thr Trp Thr Gln Gln Phe Pro Tyr Ala His Pro Leu Trp Val
65                  70                  75                  80

Gly Gln Phe Ile Gly Phe Leu Asn Ile Tyr Glu Pro Asp Tyr Ala Lys
                85                  90                  95

Ala Val Tyr Ser Arg Gly Asp Pro Lys Ala Pro Asp Val Tyr Asp Phe
            100                 105                 110

Phe Leu Gln Trp Ile Gly Arg Gly Leu Leu Val Leu Glu Gly Pro Lys
        115                 120                 125

Trp Leu Gln His Arg Lys Leu Leu Thr Pro Gly Phe His Tyr Asp Val
    130                 135                 140

Leu Lys Pro Tyr Val Ala Val Phe Thr Glu Ser Thr Arg Ile Met Leu
145                 150                 155                 160

Asp Lys Trp Glu Glu Lys Ala Arg Glu Gly Lys Ser Phe Asp Ile Phe

                165                 170                 175

Cys Asp Val Gly His Met Ala Leu Asn Thr Leu Met Lys Cys Thr Phe
            180                 185                 190

Gly Arg Gly Asp Thr Gly Leu Gly His Arg Asp Ser Ser Tyr Tyr Leu
        195                 200                 205

Ala Val Ser Asp Leu Thr Leu Leu Met Gln Gln Arg Leu Val Ser Phe
    210                 215                 220

Gln Tyr His Asn Asp Phe Ile Tyr Trp Leu Thr Pro His Gly Arg Arg
225                 230                 235                 240

Phe Leu Arg Ala Cys Gln Val Ala His Asp His Thr Asp Gln Val Ile
                245                 250                 255

Arg Glu Arg Lys Ala Ala Leu Gln Asp Glu Lys Val Arg Lys Lys Ile
            260                 265                 270

Gln Asn Arg Arg His Leu Asp Phe Leu Asp Ile Leu Leu Gly Ala Arg
        275                 280                 285

Asp Glu Asp Asp Ile Lys Leu Ser Asp Ala Asp Leu Arg Ala Glu Val
    290                 295                 300

Asp Thr Phe Met Phe Glu Gly His Asp Thr Thr Thr Ser Gly Ile Ser
305                 310                 315                 320

Trp Phe Leu Tyr Cys Met Ala Leu Tyr Pro Glu His Gln His Arg Cys
                325                 330                 335

Arg Glu Glu Val Arg Glu Ile Leu Gly Asp Gln Asp Phe Phe Gln Trp
            340                 345                 350

Asp Asp Leu Gly Lys Met Thr Tyr Leu Thr Met Cys Ile Lys Glu Ser
        355                 360                 365

Phe Arg Leu Tyr Pro Pro Val Pro Gln Val Tyr Arg Gln Leu Ser Lys
    370                 375                 380

Pro Val Thr Phe Val Asp Gly Arg Ser Leu Pro Ala Gly Ser Leu Ile
385                 390                 395                 400

Ser Met His Ile Tyr Ala Leu His Arg Asn Ser Ala Val Trp Pro Asp
                405                 410                 415

Pro Glu Val Phe Asp Pro Leu Arg Phe Ser Thr Glu Asn Ala Ser Lys
            420                 425                 430

Arg His Pro Phe Ala Phe Met Pro Phe Ser Ala Gly Pro Arg Asn Cys
        435                 440                 445

Ile Gly Gln Gln Phe Ala Met Ser Glu Met Lys Val Val Thr Ala Met
    450                 455                 460

Cys Leu Leu Arg Phe Glu Phe Ser Leu Asp Pro Ser Arg Leu Pro Ile
465                 470                 475                 480

Lys Met Pro Gln Leu Val Leu Arg Ser Lys Asn Gly Phe His Leu His
                485                 490                 495

Leu Lys Pro Leu Gly Pro Gly Ser Gly Lys
            500                 505

<210>  66
<211>  1521
<212>  DNA
<213>  hybrid

<400>  66
atgctcggct tcctctcccg cctgggcctg tgggcttccg gactgatctt gatcctaggc      60

ttcctcaagc tcctccgcct gctgcttcga aggcagaggt tggcccgggc catggacagc     120

ttcccagggc cacccactca ctggctgttt gggcacgccc tcgagatcca gaagacgggg     180

agcctggaca aggtggtgac ctggacccag cagttcccct acgcccaccc tctctgggtt     240

ggacagttca ttggcttcct gaacatctac gagcccgact acgccaaagc tgtgtacagc     300

cgtggggacc ctaaagcccc ggatgtgtat gacttcttcc tccagtggat tgggagaggc     360

ctgctggttc ttgaggggcc caagtggttg cagcaccgca agctgctcac acctggcttt     420

cattatgatg tgctgaagcc ctatgtggcc gtgttcactg agtctacacg tatcatgctg     480

gacaagtggg aagagaaagc tcgggagggt aagtcctttg acatcttctg cgatgtgggt     540

cacatggcgc tgaacacact catgaagtgc acctttggaa gaggagacac cggcctgggc     600

cacagggaca gcagctacta ccttgcagtc agcgatctca ctctgttgat gcagcagcgc     660

cttgtgtcct tccagtacca taatgacttc atctactggc tcaccccaca tggccgccgc     720

ttcctgcggg cctgccaggt ggcccatgac catacagacc aggtcatcag ggagcggaag     780

gcagccctgc aggatgagaa ggtgcggaag aagatccaga accggaggca cctggacttc     840

ctggacattc tcctgggtgc ccgggatgaa gatgacatca aactgtcaga tgcagacctc     900

cgggctgaag tggacacatt catgtttgaa ggccatgaca ccaccaccag tggtatctcc     960

tggtttctct actgcatggc cctgtaccct gagcaccagc atcgttgtag agaggaggtc    1020

cgcgagatcc taggggacca ggacttcttc cagtgggatg atctgggcaa aatgacttat    1080

ctgaccatgt gcatcaagga gagcttccgc ctctacccac ctgtgcccca ggtgtaccgc    1140

cagctcagca agcctgtcac ctttgtggat ggccggtctc tacctgcagg aagcctgatc 1200

tctatgcata tctatgccct ccataggaac agtgctgtat ggcccgaccc tgaggtcttt 1260

gaccctctgc gcttttccac tgagaatgca tccaaacgcc atccctttgc ctttatgccc 1320

ttctctgctg ggcccaggaa ctgcattggg cagcagtttg ccatgagtga gatgaaggtg 1380

gtcacagcca tgtgcttgct ccgctttgag ttctctctgg acccctcacg gctgcccatc 1440

aagatgcccc agcttgtcct gcgctccaag aatggctttc acctccacct gaagccactg 1500

ggccctgggt ctgggaagta g 1521

<210> 67
<211> 511
<212> PRT
<213> hybrid

<400> 67

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
        180                 185                 190

Met Lys Cys Thr Phe Gly Lys Gly Asp Ser Gly Leu Asn His Arg Asp
    195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Arg Ala Ala His Asp His
                245                 250                 255

Thr Asp Arg Val Ile Arg Gln Arg Lys Ala Ala Leu Gln Asp Glu Lys
            260                 265                 270

Glu Arg Glu Lys Ile Gln Asn Arg Arg His Leu Asp Phe Leu Asp Ile
        275                 280                 285

Leu Leu Asp Val Arg Gly Glu Ser Gly Val Gln Leu Ser Asp Thr Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln Gln Arg Cys Arg Glu Glu Val Arg Glu Ile Leu Gly Asp Gln
            340                 345                 350

Asp Ser Phe Gln Trp Glu Asp Leu Ala Lys Met Thr Tyr Leu Thr Met
        355                 360                 365

Cys Met Lys Glu Cys Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210> 68
<211> 1536
<212> DNA
<213> hybrid

<400> 68
atggtgccca gcttcctctc cctgagcttc tcctccttgg gcctgtgggc ttctgggctg 60

atcttggtct taggctttct caagctcatc cacctgctgc tgcggaggca gacgttggct 120

aaggctatgg acaaattccc agggcctccc acccactggc tttttggaca tgccctcgag 180

atccaggaga cggggagcct ggacaaagtg gtgtcctggg cccaccagtt cccgtatgcc 240

cacccactct ggttcggaca gttcattggc ttcctgaaca tctatgagcc tgactatgcc 300

aaagctgtgt acagccgtgg ggaccctaag gcccctgatg tgtatgactt cttcctccag 360

tggattggca aaggcctgct ggttctggat gggcccaagt ggttccagca ccgcaagctg 420

ctcacgcctg gcttccatta cgacgtgctg aagccctacg tggccatctt tgccgactcc 480

acacgcatca tgctggaaaa atgggagaaa aaggcctgtg agggtaagag cttcgacatc 540

ttctctgacg tgggccacat ggcgctcgac acgctcatga agtgtacgtt tggcaaagga 600

gacagtggcc tgaatcacag ggacagcagc tactacgtgg cagtcagcga gctcacgctg 660

ctgatgcagc aacgcatcga ctccttccag taccacaacg acttcatcta ctggctcact 720

ccgcacggcc gccgcttcct gcgggcctgc agggcggccc acgaccacac cgacagggtc 780

atcagacagc ggaaggcagc cctgcaggat gagaaggagc gggagaagat ccagaaccgg 840

agacatctgg acttcctgga cattctcttg gatgtccgcg gtgaaagtgg agtccagctg 900

tcggacacag acctccgcgc tgaagtggac acgttcatgt tcgaaggtca tgacaccacc 960

accagcggca tctcctggtt cctctactgc atggccttgt accctgagca ccagcagcgc 1020

tgtagggagg aggtccgtga gatcctggga gaccaggact ccttccagtg ggaggacttg 1080

gccaagatga cctacctgac catgtgcatg aaggagtgct tccgcctcta cccgcccgtg 1140

ccccaggtgt accgccagct cagcaagcct gtcacctttg tggatggccg gtctctacct 1200

gcaggaagcc tgatctctat gcatatctat gccctccata ggaacagtgc tgtatggccc 1260

gaccctgagg tctttgaccc tctgcgcttt tccactgaga atgcatccaa acgccatccc 1320

tttgccttta tgcccttctc tgctgggccc aggaactgca ttgggcagca gtttgccatg 1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 69
<211> 508
<212> PRT
<213> hybrid

<400> 69

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
        180                 185                 190

Met Lys Cys Thr Phe Gly Arg Gly Asp Thr Gly Leu Gly His Arg Asp
    195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Ser Phe Val Asp Gly Arg Ser Leu Pro
385                 390                 395                 400

Ala Gly Ser Leu Ile Ser Leu His Ile Tyr Ala Leu His Arg Asn Ser
                405                 410                 415

Asp Val Trp Pro Asp Pro Glu Val Phe Asp Pro Leu Arg Phe Ser Pro
            420                 425                 430

Glu Asn Ser Ser Gly Arg His Pro Tyr Ala Phe Ile Pro Phe Ser Ala
        435                 440                 445

Gly Pro Arg Asn Cys Ile Gly Gln Gln Phe Ala Met Asn Glu Met Lys
    450                 455                 460

Val Val Thr Ala Leu Cys Leu Leu Arg Phe Glu Phe Ser Val Asp Pro
465                 470                 475                 480

Leu Arg Leu Pro Ile Lys Leu Pro Gln Leu Val Leu Arg Ser Lys Asn
                485                 490                 495

Gly Ile His Leu Tyr Leu Lys Pro Leu Gly Pro Lys
            500                 505

<210>  70
<211>  1527
<212>  DNA
<213>  hybrid

<400>  70
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagccc gtcagctttg tggacggccg ctccctgcct    1200

gcaggcagcc tgatctccct gcatatctac gccctccata ggaacagcga cgtgtggcct    1260

gaccctgagg tctttgaccc cctgcgcttt tccccggaga actcgtctgg acgccacccc    1320

tatgccttca ttcccttctc tgccgggccc aggaactgca tcgggcagca gttcgccatg    1380

aacgagatga aggtggtcac agccctgtgc ctgctccgct ttgagttctc cgtggacccc    1440

ctgcggctgc ccatcaagct gccccagctg gtcctgcgct ccaagaatgg catccacctc    1500

tacttgaagc ctctgggccc caagtag                                        1527

<210>  71
<211>  508
<212>  PRT
<213>  hybrid

<400>  71

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
            180                 185                 190

Met Lys Cys Thr Phe Gly Lys Gly Asp Ser Gly Leu Asn His Arg Asp
        195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Arg Ala Ala His Asp His
                245                 250                 255

Thr Asp Arg Val Ile Arg Gln Arg Lys Ala Ala Leu Gln Asp Glu Lys
            260                 265                 270

Glu Arg Glu Lys Ile Gln Asn Arg Arg His Leu Asp Phe Leu Asp Ile
        275                 280                 285

Leu Leu Asp Val Arg Gly Glu Ser Gly Val Gln Leu Ser Asp Thr Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln Gln Arg Cys Arg Glu Glu Val Arg Glu Ile Leu Gly Asp Gln
            340                 345                 350

Asp Ser Phe Gln Trp Glu Asp Leu Ala Lys Met Thr Tyr Leu Thr Met
        355                 360                 365

Cys Met Lys Glu Cys Phe Arg Leu Tyr Pro Pro Val Pro Gln Val Tyr
    370                 375                 380

Arg Gln Leu Ser Lys Pro Val Ser Phe Val Asp Gly Arg Ser Leu Pro
385                 390                 395                 400

Ala Gly Ser Leu Ile Ser Leu His Ile Tyr Ala Leu His Arg Asn Ser
                405                 410                 415

Asp Val Trp Pro Asp Pro Glu Val Phe Asp Pro Leu Arg Phe Ser Pro
            420                 425                 430

Glu Asn Ser Ser Gly Arg His Pro Tyr Ala Phe Ile Pro Phe Ser Ala

435                 440                 445

Gly Pro Arg Asn Cys Ile Gly Gln Gln Phe Ala Met Asn Glu Met Lys
    450                 455                 460

Val Val Thr Ala Leu Cys Leu Leu Arg Phe Glu Phe Ser Val Asp Pro
465                 470                 475                 480

Leu Arg Leu Pro Ile Lys Leu Pro Gln Leu Val Leu Arg Ser Lys Asn
                485                 490                 495

Gly Ile His Leu Tyr Leu Lys Pro Leu Gly Pro Lys
            500                 505

<210>  72
<211>  1527
<212>  DNA
<213>  hybrid

<400>  72
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggca aaggcctgct ggttctggat gggcccaagt ggttccagca ccgcaagctg     420

ctcacgcctg gcttccatta cgacgtgctg aagccctacg tggccatctt tgccgactcc     480

acacgcatca tgctggaaaa atgggagaaa aaggcctgtg agggtaagag cttcgacatc     540

ttctctgacg tgggccacat ggcgctcgac acgctcatga agtgtacgtt tggcaaagga     600

gacagtggcc tgaatcacag ggacagcagc tactacgtgg cagtcagcga gctcacgctg     660

ctgatgcagc aacgcatcga ctccttccag taccacaacg acttcatcta ctggctcact     720

ccgcacggcc gccgcttcct gcgggcctgc agggcggccc acgaccacac cgacagggtc     780

atcagacagc ggaaggcagc cctgcaggat gagaaggagc gggagaagat ccagaaccgg     840

agacatctgg acttcctgga cattctcttg gatgtccgcg gtgaaagtgg agtccagctg     900

tcggacacag acctccgcgc tgaagtggac acgttcatgt tcgaaggtca tgacaccacc     960

accagcggca tctcctggtt cctctactgc atggccttgt accctgagca ccagcagcgc    1020

tgtagggagg aggtccgtga gatcctggga gaccaggact ccttccagtg ggaggacttg    1080

gccaagatga cctacctgac catgtgcatg aaggagtgct tccgcctcta cccgcccgtg    1140

ccccaggtgt accgccagct cagcaagccc gtcagctttg tggacggccg ctccctgcct    1200

gcaggcagcc tgatctccct gcatatctac gccctccata ggaacagcga cgtgtggcct    1260

gaccctgagg tctttgaccc cctgcgcttt tccccggaga actcgtctgg acgccacccc    1320

tatgccttca ttcccttctc tgccgggccc aggaactgca tcgggcagca gttcgccatg    1380

aacgagatga aggtggtcac agccctgtgc ctgctccgct ttgagttctc cgtggacccc    1440

ctgcggctgc ccatcaagct gccccagctg gtcctgcgct ccaagaatgg catccacctc    1500

tacttgaagc ctctgggccc caagtag                                        1527

<210>  73
<211>  511
<212>  PRT
<213>  hybrid

<400>  73

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu

180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  74
<211>  1536
<212>  DNA
<213>  hybrid

<400>  74
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga aaggcctgct ggttcttgat gggcccaagt ggttccagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc 1320

tttgccttta tgcccttctc tgctgggccc aggaactgca ttgggcagca gtttgccatg 1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 75
<211> 511
<212> PRT
<213> hybrid

<400> 75

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  76
<211>  1536
<212>  DNA
<213>  hybrid

<400>  76
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccatctt cgccgactct     480

acacgtatca tgctggaaaa gtgggaaaaa aaagcttgtg agggtaagtc ctttgacatc     540

ttctctgatg tgggtcacat ggcgctggac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc 1320

tttgccttta tgcccttctc tgctgggccc aggaactgca ttgggcagca gtttgccatg 1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 77
<211> 511
<212> PRT
<213> hybrid

<400> 77

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Lys Gly Asp Ser Gly Leu Asn His Arg Asp
        195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  78
<211>  1536
<212>  DNA
<213>  hybrid

<400>  78
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaaaagga     600

gacagtggcc tgaatcacag ggacagcagc tactacgtgg cagtcagcga gctcactctg     660

ttgatgcagc agcgcatcga ctccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg 1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 79
<211> 511
<212> PRT
<213> hybrid

<400> 79

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys

450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  80
<211>  1536
<212>  DNA
<213>  hybrid

<400>  80
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga aaggcctgct ggttcttgat gggcccaagt ggttccagca ccgcaagctg     420

ctcacacctg gctttcatta tgatgtgctg aagccctatg tggccatctt cgccgactct     480

acacgtatca tgctggaaaa gtgggaaaaa aaagcttgtg agggtaagtc ctttgacatc     540

ttctctgatg tgggtcacat ggcgctggac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc    1440

tcacggctgc ccatcaagat gccccagctt gtcctgcgct ccaagaatgg ctttcacctc    1500

cacctgaagc cactgggccc tgggtctggg aagtag                              1536

<210>  81
<211>  511
<212>  PRT
<213>  hybrid

<400>  81

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
            180                 185                 190

Met Lys Cys Thr Phe Gly Lys Gly Asp Ser Gly Leu Asn His Arg Asp

195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  82
<211>  1536
<212>  DNA
<213>  hybrid

<400>  82
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccatctt cgccgactct     480

acacgtatca tgctggaaaa gtgggaaaaa aaagcttgtg agggtaagtc ctttgacatc     540

ttctctgatg tgggtcacat ggcgctggac acactcatga agtgcacctt tggaaaagga     600

gacagtggcc tgaatcacag ggacagcagc tactacgtgg cagtcagcga gctcactctg     660

ttgatgcagc agcgcatcga ctccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 83
<211> 511
<212> PRT
<213> hybrid

<400> 83

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Lys Gly Asp Ser Gly Leu Asn His Arg Asp
        195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  84
<211>  1536
<212>  DNA
<213>  hybrid

<400>  84
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga aaggcctgct ggttcttgat gggcccaagt ggttccagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaaaagga     600

gacagtggcc tgaatcacag ggacagcagc tactacgtgg cagtcagcga gctcactctg     660

ttgatgcagc agcgcatcga ctccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc 1440

tcacggctgc ccatcaagat gccccagctt gtcctgcgct ccaagaatgg ctttcacctc 1500

cacctgaagc cactgggccc tgggtctggg aagtag 1536

<210> 85
<211> 511
<212> PRT
<213> hybrid

<400> 85

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Lys Gly Leu Leu Val
        115                 120                 125

Leu Asp Gly Pro Lys Trp Phe Gln His Arg Lys Leu Leu Thr Pro Gly
    130                 135                 140

Phe His Tyr Asp Val Leu Lys Pro Tyr Val Ala Ile Phe Ala Asp Ser
145                 150                 155                 160

Thr Arg Ile Met Leu Glu Lys Trp Glu Lys Lys Ala Cys Glu Gly Lys
                165                 170                 175

Ser Phe Asp Ile Phe Ser Asp Val Gly His Met Ala Leu Asp Thr Leu
            180                 185                 190

Met Lys Cys Thr Phe Gly Lys Gly Asp Ser Gly Leu Asn His Arg Asp
        195                 200                 205

Ser Ser Tyr Tyr Val Ala Val Ser Glu Leu Thr Leu Leu Met Gln Gln
    210                 215                 220

Arg Ile Asp Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  86
<211>  1536
<212>  DNA
<213>  hybrid

<400>  86
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga aaggcctgct ggttcttgat gggcccaagt ggttccagca ccgcaagctg     420

ctcacacctg gctttcatta tgatgtgctg aagccctatg tggccatctt cgccgactct     480

acacgtatca tgctggaaaa gtgggaaaaa aaagcttgtg agggtaagtc ctttgacatc     540

ttctctgatg tgggtcacat ggcgctggac acactcatga agtgcacctt tggaaaagga     600

gacagtggcc tgaatcacag ggacagcagc tactacgtgg cagtcagcga gctcactctg     660

ttgatgcagc agcgcatcga ctccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc    1440

tcacggctgc ccatcaagat gccccagctt gtcctgcgct ccaagaatgg ctttcacctc 1500

cacctgaagc cactgggccc tgggtctggg aagtag 1536

<210> 87
<211> 511
<212> PRT
<213> hybrid

<400> 87

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Arg Ala Ala His Asp His
                245                 250                 255

Thr Asp Arg Val Ile Arg Gln Arg Lys Ala Ala Leu Gln Asp Glu Lys
            260                 265                 270

Glu Arg Glu Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro

465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  88
<211>  1536
<212>  DNA
<213>  hybrid

<400>  88
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc agggcggccc atgaccatac agacagggtc     780

atcaggcagc ggaaggcagc cctgcaggat gagaaggagc gggagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc    1440

tcacggctgc ccatcaagat gccccagctt gtcctgcgct ccaagaatgg ctttcacctc 1500

cacctgaagc cactgggccc tgggtctggg aagtag 1536

<210> 89
<211> 511
<212> PRT
<213> hybrid

<400> 89

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln

210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Asp Val Arg Gly Glu Ser Gly Val Gln Leu Ser Asp Thr Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln His Arg Cys Arg Glu Glu Val Arg Glu Ile Leu Gly Asp Gln
            340                 345                 350

Asp Phe Phe Gln Trp Asp Asp Leu Gly Lys Met Thr Tyr Leu Thr Met
        355                 360                 365

Cys Ile Lys Glu Ser Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  90
<211>  1536
<212>  DNA
<213>  hybrid

<400>  90
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg gatgtccggg gtgaaagtgg agtccagctg     900

tcagatacag acctccgggc tgaagtggac acattcatgt ttgaaggcca tgacaccacc     960

accagtggta tctcctggtt tctctactgc atggccctgt accctgagca ccagcatcgt    1020

tgtagagagg aggtccgcga gatcctaggg gaccaggact tcttccagtg ggatgatctg    1080

ggcaaaatga cttatctgac catgtgcatc aaggagagct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc    1440

tcacggctgc ccatcaagat gccccagctt gtcctgcgct ccaagaatgg ctttcacctc    1500

cacctgaagc cactgggccc tgggtctggg aagtag                                    1536

<210>  91
<211>  511
<212>  PRT
<213>  hybrid

<400>  91

Met Val Pro Ser Phe Leu Ser Leu Ser Phe Ser Ser Leu Gly Leu Trp
1               5                   10                  15

Ala Ser Gly Leu Ile Leu Val Leu Gly Phe Leu Lys Leu Ile His Leu
            20                  25                  30

Leu Leu Arg Arg Gln Thr Leu Ala Lys Ala Met Asp Lys Phe Pro Gly
        35                  40                  45

Pro Pro Thr His Trp Leu Phe Gly His Ala Leu Glu Ile Gln Glu Thr
    50                  55                  60

Gly Ser Leu Asp Lys Val Val Ser Trp Ala His Gln Phe Pro Tyr Ala
65                  70                  75                  80

His Pro Leu Trp Phe Gly Gln Phe Ile Gly Phe Leu Asn Ile Tyr Glu
                85                  90                  95

Pro Asp Tyr Ala Lys Ala Val Tyr Ser Arg Gly Asp Pro Lys Ala Pro
            100                 105                 110

Asp Val Tyr Asp Phe Phe Leu Gln Trp Ile Gly Arg Gly Leu Leu Val
        115                 120                 125

Leu Glu Gly Pro Lys Trp Leu Gln His Arg Lys Leu Leu Thr Pro Gly
    130                 135                 140

Phe His Tyr Asp Val Leu Lys Pro Tyr Val Ala Val Phe Thr Glu Ser
145                 150                 155                 160

Thr Arg Ile Met Leu Asp Lys Trp Glu Glu Lys Ala Arg Glu Gly Lys
                165                 170                 175

Ser Phe Asp Ile Phe Cys Asp Val Gly His Met Ala Leu Asn Thr Leu
            180                 185                 190

Met Lys Cys Thr Phe Gly Arg Gly Asp Thr Gly Leu Gly His Arg Asp
        195                 200                 205

Ser Ser Tyr Tyr Leu Ala Val Ser Asp Leu Thr Leu Leu Met Gln Gln
    210                 215                 220

Arg Leu Val Ser Phe Gln Tyr His Asn Asp Phe Ile Tyr Trp Leu Thr
225                 230                 235                 240

Pro His Gly Arg Arg Phe Leu Arg Ala Cys Gln Val Ala His Asp His
                245                 250                 255

Thr Asp Gln Val Ile Arg Glu Arg Lys Ala Ala Leu Gln Asp Glu Lys
            260                 265                 270

Val Arg Lys Lys Ile Gln Asn Arg Arg His Leu Asp Phe Leu Asp Ile
        275                 280                 285

Leu Leu Gly Ala Arg Asp Glu Asp Asp Ile Lys Leu Ser Asp Ala Asp
    290                 295                 300

Leu Arg Ala Glu Val Asp Thr Phe Met Phe Glu Gly His Asp Thr Thr
305                 310                 315                 320

Thr Ser Gly Ile Ser Trp Phe Leu Tyr Cys Met Ala Leu Tyr Pro Glu
                325                 330                 335

His Gln Gln Arg Cys Arg Glu Glu Val Arg Glu Ile Leu Gly Asp Gln
            340                 345                 350

Asp Ser Phe Gln Trp Glu Asp Leu Ala Lys Met Thr Tyr Leu Thr Met
        355                 360                 365

Cys Met Lys Glu Cys Phe Arg Leu Tyr Pro Pro Val Pro Gln Val Tyr
    370                 375                 380

Arg Gln Leu Ser Lys Pro Val Thr Phe Val Asp Gly Arg Ser Leu Pro
385                 390                 395                 400

Ala Gly Ser Leu Ile Ser Met His Ile Tyr Ala Leu His Arg Asn Ser
                405                 410                 415

Ala Val Trp Pro Asp Pro Glu Val Phe Asp Pro Leu Arg Phe Ser Thr
            420                 425                 430

Glu Asn Ala Ser Lys Arg His Pro Phe Ala Phe Met Pro Phe Ser Ala
        435                 440                 445

Gly Pro Arg Asn Cys Ile Gly Gln Gln Phe Ala Met Ser Glu Met Lys
    450                 455                 460

Val Val Thr Ala Met Cys Leu Leu Arg Phe Glu Phe Ser Leu Asp Pro
465                 470                 475                 480

Ser Arg Leu Pro Ile Lys Met Pro Gln Leu Val Leu Arg Ser Lys Asn
                485                 490                 495

Gly Phe His Leu His Leu Lys Pro Leu Gly Pro Gly Ser Gly Lys
            500                 505                 510

<210>  92
<211>  1536
<212>  DNA
<213>  hybrid

<400>  92
atggtgccca gcttcctctc cctgagcttc tcctccttgg gcctgtgggc ttctgggctg      60

atcttggtct taggctttct caagctcatc cacctgctgc tgcggaggca gacgttggct     120

aaggctatgg acaaattccc agggcctccc acccactggc tttttggaca tgccctcgag     180

atccaggaga cggggagcct ggacaaagtg gtgtcctggg cccaccagtt cccgtatgcc     240

cacccactct ggttcggaca gttcattggc ttcctgaaca tctatgagcc tgactatgcc     300

aaagctgtgt acagccgtgg ggaccctaag gcccctgatg tgtatgactt cttcctccag     360

tggattggga gaggcctgct ggttcttgag gggcccaagt ggttgcagca ccgcaagctg     420

ctcacacctg gctttcatta tgatgtgctg aagccctatg tggccgtgtt cactgagtct     480

acacgtatca tgctggacaa gtgggaagag aaagctcggg agggtaagtc ctttgacatc     540

ttctgcgatg tgggtcacat ggcgctgaac acactcatga agtgcacctt tggaagagga     600

gacaccggcc tgggccacag ggacagcagc tactaccttg cagtcagcga tctcactctg     660

ttgatgcagc agcgccttgt gtccttccag taccataatg acttcatcta ctggctcacc     720

ccacatggcc gccgcttcct gcgggcctgc caggtggccc atgaccatac agaccaggtc     780

atcagggagc ggaaggcagc cctgcaggat gagaaggtgc ggaagaagat ccagaaccgg     840

aggcacctgg acttcctgga cattctcctg ggtgcccggg atgaagatga catcaaactg     900

tcagatgcag acctccgggc tgaagtggac acattcatgt ttgaaggcca tgacaccacc     960

accagtggta tctcctggtt tctctactgc atggccctgt accctgagca ccagcagcgt    1020

tgtagagagg aggtccgcga gatcctaggg gaccaggact ccttccagtg ggaggatctg    1080

gccaaaatga cttatctgac catgtgcatg aaggagtgct tccgcctcta cccacctgtg    1140

ccccaggtgt accgccagct cagcaagcct gtcacctttg tggatggccg gtctctacct    1200

gcaggaagcc tgatctctat gcatatctat gccctccata ggaacagtgc tgtatggccc    1260

gaccctgagg tctttgaccc tctgcgcttt tccactgaga atgcatccaa acgccatccc    1320

tttgccttta tgcccttctc tgctgggccc aggaactgca ttgggcagca gtttgccatg    1380

agtgagatga aggtggtcac agccatgtgc ttgctccgct ttgagttctc tctggacccc    1440

tcacggctgc ccatcaagat gccccagctt gtcctgcgct ccaagaatgg ctttcacctc    1500

cacctgaagc cactgggccc tgggtctggg aagtag 1536